# EUROPEAN PATENT APPLICATION

(11) **EP 4 786 494 A1**
(43) Date of publication of application: **05.08.2026**
(21) Application number: 24870979.2
(22) Date of filing: 27.09.2024
(51) Int. Cl.: C07K 16/28, A61K 39/395, A61P 35/00

(54) **ANTI-FGFR2 ANTIBODY AND MULTISPECIFIC BINDING MOLECULE COMPRISING SAME**

(30) Priority: 28.09.2023 WO PCT/CN2023/122802
(71) Applicant: Nanjing Leads Biolabs Co., Ltd., Jiangbei New Area Nanjing Jiangsu 210031 (CN)
(72) Inventor: DANG, Yujia, Nanjing, Jiangsu 210019 (CN); HUANG, Xiao, Nanjing, Jiangsu 210019 (CN); NI, Chengze, Nanjing, Jiangsu 210019 (CN); ZHANG, Peng, Nanjing, Jiangsu 210019 (CN); LING, Hong, Nanjing, Jiangsu 210019 (CN)
(74) Representative: Bianchetti & Minoja with Trevisan & Cuonzo IPS SRL
(86) International application number: PCT/CN2024/121805
(87) International publication number: WO 2025/067442

(57) **Abstract**

The present disclosure relates to an isolated antibody or an antigen-binding fragment specifically binding to FGFR2 protein and a multispecific binding molecule comprising the isolated antibody or the antigen-binding fragment specifically binding to FGFR2 protein. The present disclosure also relates to a nucleic acid encoding the isolated antibody or the antigen-binding fragment specifically binding to FGFR2 protein or the multispecific binding molecule, a host cell comprising the nucleic acid encoding the isolated antibody or the antigen-binding fragment specifically binding to FGFR2 protein or the multispecific binding molecule, and a method for preparing the isolated antibody or the antigen-binding fragment specifically binding to FGFR2 protein or the multispecific binding molecule. In addition, the present disclosure relates to diagnostic, prophylactic, and/or therapeutic use of the isolated antibody or the antigen-binding fragment specifically binding to FGFR2 protein or the multispecific binding molecule.

## Description

### TECHNICAL FIELD

The present disclosure generally relates to binding molecules and use thereof. More specifically, the present disclosure relates to an isolated antibody or an antigen-binding fragment specifically binding to FGFR2, and a multispecific binding molecule (e.g., comprising the antibody or the antigen-binding fragment thereof). The present disclosure further relates to diagnostic, prophylactic, and/or therapeutic use of the isolated antibody or the antigen-binding fragment specifically binding to FGFR2 and the multispecific binding molecule.

### BACKGROUND

Fibroblast growth factor receptor 2 (FGFR2) is a tyrosine kinase receptor having variants in various forms. The alternative splicing in FGFR2 extracellular domains produces variants containing all three Ig-like domains (alpha isoform) or only two Ig-like domains (beta isoform) [Toshiyuki Ishiwata, Role of fibroblast growth factor receptor-2 splicing in normal and cancer cells. Frontiers In Bioscience, Landmark, 23,626-639, January 1, 2018]. According to the sources of exons, FGFR2 can be divided into two types, FGFR2IIIb and FGFR2IIIc. When the second half at the C-terminus of the third extracellular domain is encoded by exon 8, the FGFR2IIIb variant is produced, and when the second half at the C-terminus is encoded by exon 9, the FGFR2IIIc variant is produced [Toshiyuki Ishiwata, Role of fibroblast growth factor receptor-2 splicing in normal and cancer cells. Frontiers In Bioscience, Landmark, 23,626-639, January 1, 2018]. FGFR2IIIb is mainly expressed in epithelial cells and binds to ligand FGF7, as well as FGF10, FGF22, etc. The binding of FGFR2 to its ligand causes receptor dimerization and subsequent phosphorylation of kinase domains, which results in the docking of adaptor proteins and the activation of four key downstream pathways: RAS-RAF-MAPK, PI3K-AKT, signal transducer and activator of transcription (STAT), and phospholipase Cγ (PLCγ) [Nicholas Turner and Richard Grose, Fibroblast growth factor signalling: from development to cancer. DOI:10.1038/nrc2780], thereby affecting cell proliferation, survival, migration, and invasion. FGFR2IIIb is overexpressed in a variety of cancers, including gastric cancer, breast cancer, lung cancer, esophageal cancer, pancreatic cancer, colorectal cancer, and the like.

Gastric cancer is the fifth most diagnosed malignancy worldwide, with more than 1 million new cases each year and 784,000 deaths worldwide in 2018 [Elizabeth CSmyth, et al., Gastric cancer. Lancet 2020; 396:635-48]. Gastric cancer can be divided into early gastric cancer and advanced gastric cancer. Since gastric cancer is often in the advanced stage at the time of diagnosis, the mortality rate of gastric cancer is high, making gastric cancer the third most common cause of cancer-related death. Although there are many targeted therapies for gastric cancer, the current standard of care for gastric cancer is still surgery, leaving a great unmet clinical demand.

Bemarituzumab, which targets FGFR2IIIb, is a humanized defucosylated monoclonal antibody against FGFR2b. It can inhibit the growth of FGFR2b gene-amplified gastric cancer tumor cells in animal pharmacodynamic studies via 2 proven mechanisms of action: blocking the FGFR2b signaling by competitively binding to and inhibiting FGF, and inducing antibody-dependent cell-mediated cytotoxicity (ADCC) against FGFR2b-overexpressing tumor cells [Hong Xiang et al., Preclinical characterization of bemarituzumab, an anti-FGFR2b antibody for the treatment of cancer. MABS, 2021, VOL. 13, NO.1, e1981202(16pages). DOI:10.1080/19420862.2021.1981202]. Bemarituzumab is currently in its phase III clinical trial for the treatment of advanced gastric cancer and gastroesophageal junction carcinoma in combination with mFOLFOX6. RLY-4008 is a highly selective FGFR2 inhibitor that can inhibit the growth of a variety of FGFR2-mutant tumors *in vivo*, and is currently in its phase I trial for unresectable or metastatic bile duct cancer or other solid tumors [Jessica Casaletto et al.,: RLY-4008, a Novel Precision Therapy for FGFR2-Driven Cancers Designed to Potently and Selectively Inhibit FGFR2 and FGFR2 Resistance Mutations. AACR, 2021, Poster1455]. In addition, some pan-FGFR inhibitors have been approved. Erdafitinib, the first FDA-approved oral FGFR inhibitor, can target FGFR subtypes 1/2/3 and is used for treating adult patients with locally advanced or metastatic bladder cancer with disease progression to platinum-based chemotherapy with FGFR3 or FGFR2 mutations. Currently, the indications of its ongoing clinical trials include non-small cell lung cancer, gastric cancer, esophageal cancer, bile duct cancer, multiple myeloma, and the like.

Vascular endothelial growth factor A (hereinafter referred to as VEGFA) is a homodimeric glycoprotein binding to heparin with a molecular weight of 40-45 kDa. It is a member of the growth factor family, which includes VEGFB, VEGFC, VEGFD, and placental growth factor (PlGF). The human VEGFA gene is located on chromosome 6p21.1 and consists of 8 exons and 7 introns. Vascular endothelial growth factor acts by interacting with two highly related tyrosine kinase receptors, VEGFR1 (also known as Flt1) and VEGFR2 (also known as Flk1 or KDR), which are expressed mainly in endothelial cells and a wide range of non-endothelial cells, as well as cells of different tumor types [Daniela Frezzetti et al., VEGF as a potential target in lung cancer. EXPERT OPINION ON THERAPEUTIC TARGETS, 2017, VOL. 21, NO. 10, 959-966]. VEGFR1 has a higher affinity for VEGFA, but the binding of VEGFA to VEGFR2 induces homodimerization or heterodimerization, which may result in the phosphorylation of tyrosine kinase domains and the activation of several signaling proteins, including mitogen-activated protein kinase and PI-3-kinase (PI-3-K), to regulate different cellular functions, including cell proliferation, survival, and migration, and thus is a major pathway for angiogenesis [Chloe J, et al., Molecular Pharmacology of VEGF-A Isoforms: Binding and Signalling at VEGFR2. Int.J. Mol.Sci. 2018, 19, 1264; Doi:10.3390/ijms19041264].

Bevacizumab is an FDA-approved anti-angiogenic drug. It is a recombinant humanized monoclonal antibody against VEGFA that can bind to VEGFA and block its biological activity, thereby inhibiting the process of angiogenesis. Studies have shown that the inhibition of vascular endothelial growth factor ligands by bevacizumab can prevent the growth of tumors. Bevacizumab has been approved for the following conditions: gastric cancer, metastatic colorectal cancer, non-squamous cell lung cancer, metastatic breast cancer, metastatic renal cell carcinoma, etc.

Therefore, there is a need to develop antibodies against FGFR2b and multispecific binding molecules constructed on the basis of same.

### SUMMARY

In one aspect, the present disclosure relates to an isolated antibody or an antigen-binding fragment thereof specifically binding to FGFR2 protein.

Another aspect of the present disclosure relates to a multispecific binding molecule, e.g., a bispecific binding molecule or a fusion protein, comprising an antibody or an antigen-binding fragment thereof specifically binding to FGFR2 protein. In one embodiment, the multispecific binding molecule (e.g., the bispecific binding molecule or the fusion protein) specifically binds to FGFR2 protein and VEGF, e.g., VEGFA, such as VEGF165 molecule.

Other aspects of the present disclosure also relate to a nucleic acid encoding the isolated antibody or the antigen-binding fragment specifically binding to FGFR2 protein or the multispecific binding molecule, a host cell comprising the isolated antibody or the antigen-binding fragment specifically binding to FGFR2 protein or the multispecific binding molecule or the nucleic acid, and a method for preparing the isolated antibody or the antigen-binding fragment specifically binding to FGFR2 protein or the multispecific binding molecule. In addition, the present disclosure relates to diagnostic, prophylactic, and/or therapeutic use of the isolated antibody or the antigen-binding fragment specifically binding to FGFR2 protein or the multispecific binding molecule.

The multispecific binding molecule against FGFR2b and VEGFA obtained by the present disclosure, such as a humanized antibody fusion protein comprising an anti-FGFR2 antibody and a VEGFR1 domain, in which the antibody moiety is capable of blocking FGF factor-mediated FGFR2b signaling and inducing antibody-dependent cell-mediated cytotoxicity, while the VEGFR1 moiety is capable of capturing VEGFA factor through the "trap" function to block the generation of downstream signals of VEGFR2, thereby inhibiting intratumoral angiogenesis.

### BRIEF DESCRIPTION OF THE DRAWINGS

The preferred embodiments of the present disclosure described in detail below will be better understood when read in combination with the following drawings. For the purpose of illustrating the present disclosure, currently preferred embodiments are shown in the drawings. However, it will be appreciated that the present disclosure is not limited to the precise arrangement and means of the embodiments shown in the drawings.
FIGs. 1 and 2 show the binding of murine chimeric anti-FGFR2 antibodies (i.e., antibody E3A1, antibody D12D8, antibody F6B6, antibody A1B2, antibody A5H12, antibody F3F8, antibody G11B8, and antibody H3F7-a) to gastric cancer tumor cells KATO-III expressing human FGFR2 at the cellular level.
FIGs. 3-8 show the binding assay of murine chimeric anti-FGFR2 antibodies (i.e., antibody F3F8, antibody D12D8, antibody G11B8, antibody A1B2, antibody H3F7-a, antibody E3A1, antibody A5H12, and antibody F6B6) to human FGFR2's homologous proteins FGFR1, FGFR3, and FGFR4.
FIG. 9 shows the binding assay of murine chimeric anti-FGFR2 antibodies (i.e., antibody G11B8, antibody A1B2, antibody H3F7-a, and antibody A5H12) to human FGFR2-IIIc (FGFR2c).
FIGs. 10 and 11 show the binding assay of the humanized anti-FGFR2 antibodies (i.e., humanized antibodies huG1B8-1 to huG11B8-12) to gastric cancer tumor cells KATO-III expressing human FGFR2 at the cellular level.
FIGs. 12-14 show that the humanized anti-FGFR2 antibody (i.e., humanized antibody huG11B8-5) can block the binding of human FGFR2 to its ligand factors FGF7, FGF10, and FGF22, respectively.
FIG. 15 shows the assay results for the blocking effect of the antibody fusion protein against human FGFR2-VEGFR1 (i.e., antibody huG11B8-VEGFR1) on the binding of VEGF165 to VEGFR2 in the presence of engineered 293T cells with high expression of human VEGFR2.
FIG. 16 shows antibody-mediated receptor internalization and trogocytosis.
FIG. 17 shows the killing effects of NK92MI-CD16aV against FGFR2-expressing Ba/F3 cells induced by FGFR2 antibodies and fusion proteins thereof.
FIGs. 18 and 20 show the tumor volume changes in tumor-bearing mice after huG11B8-VEGFR1 administration.
FIGs. 19 and 21 show the body weight changes in tumor-bearing mice after huG11B8-VEGFR1 administration.
FIG. 22 shows an exemplary fusion protein structure.

### DETAILED DESCRIPTION

Before the present disclosure is described in detail, it should be noted that the present disclosure is not limited to the particular methods or experimental conditions described in the specification, as the methods and conditions may vary. In addition, the terms used herein are for the purpose of describing particular embodiments only and are not intended to be limiting.

### I. Definitions

In order to illustrate this description, the following definitions will be used, and wherever appropriate, terms used in the singular form may also include the plural form, and vice versa. Unless otherwise defined, any technical and scientific term used herein has the same meaning as commonly understood by those of ordinary skill in the art to which the present disclosure belongs.

The term "about" used in combination with a numerical value is intended to encompass the numerical values in a range from a lower limit less than the specified numerical value by 5% to an upper limit greater than the specified numerical value by 5%.

As used herein, the term "and/or" refers to any one of the options or two or more or all of the options.

As used herein, the term "comprise" or "include" is intended to refer to the inclusion of the elements, integers, or steps, without the exclusion of any other elements, integers, or steps. The term "comprise" or "include" used herein, unless indicated otherwise, also encompasses the situation where the entirety consists of the described elements, integers, or steps. For example, when referring to an antibody variable region "comprising" a specific sequence, it is also intended to encompass an antibody variable region consisting of the specific sequence.

When referring to "first" and "second" herein, it is only to distinguish two domains or two chains, and does not indicate the locations of the two domains in any way.

The effector cells include effector T cells (T lymphocytes), such as CD4+ T cells, CD8+ T cells, Th1, Th2, and regulatory T cells (Tregs). The effector cells may also include natural killer cells, macrophages, granulocytes, plasma cells, or B cells (lymphocytes).

The term "FGFR2" refers to fibroblast growth factor receptor 2 (e.g., human FGFR2 or cynomolgus FGFR2, e.g., the human FGFR2 protein under Accession No. NP_001138385.1 or the cynomolgus FGFR2 protein under Accession No. XP_028682763.1). In one embodiment, the FGFR2 of the present disclosure is FGFR2IIIb, e.g., human FGFR2IIIb. In one embodiment, the human FGFR2 protein of the present disclosure comprises or consists of the amino acid sequence set forth in SEQ ID NO: 94 (or an amino acid sequence without a His tag), or an amino acid sequence having at least 85%, 90%, 91%, 92%, 93%, 94%, 95%, 96%, 97%, 98%, or 99% identity thereto. In one embodiment, the cynomolgus FGFR2 protein of the present disclosure comprises or consists of the amino acid sequence set forth in SEQ ID NO: 95 (or an amino acid sequence without a His tag), or an amino acid sequence having at least 85%, 90%, 91%, 92%, 93%, 94%, 95%, 96%, 97%, 98%, or 99% identity thereto.

The term "anti-FGFR2 antibody", "anti-FGFR2", "FGFR2 antibody", or "antibody binding to FGFR2" used herein refers to such an antibody that is capable of binding to FGFR2 protein or a fragment thereof with sufficient affinity. In one embodiment, the anti-FGFR2 antibody binds to a non-FGFR2 protein to an extent of about 10%, about 20%, about 30%, about 40%, about 50%, about 60%, about 70%, about 80%, about 90%, or more, weaker than the antibody binding to FGFR2. In some embodiments, the binding is measured by, e.g., radioimmunoassay (RIA), biolayer interferometry (BLI), bioluminescence interferometry, MSD assay or surface plasmon resonance (SPR), or flow cytometry.

In one embodiment, the antigen-binding region binding to FGFR2 in the anti-FGFR2 antibody or the antigen-binding fragment thereof or the binding molecule of the present disclosure has high affinity and binding activity for cells expressing human FGFR2 or cynomolgus FGFR2 or an extracellular domain thereof. In one embodiment, the antigen-binding region binding to FGFR2 in the anti-FGFR2 antibody or the antigen-binding fragment thereof or the binding molecule of the present disclosure has cross-reactivity to human and monkey (e.g., cynomolgus) FGFR2 or an extracellular domain thereof; i.e., it is capable of binding to human and monkey (e.g., cynomolgus) FGFR2.

The term "VEGF" as used herein refers to an angiogenic factor. The VEGF family members include VEGF-A, VEGF-B, VEGF-C, VEGF-D, VEGF-E, placental growth factor (PIGF), and endocrine gland-derived VEGF (EG-VEGF). Active forms of VEGF are synthesized as homodimers or heterodimers with other VEGF family members. VEGF-A has six isoforms due to alternative splicing: VEGF121, VEGF145, VEGF165, VEGF183, VEGF189, and VEGF206. These isoforms mainly differ in their bioavailability, of which VEGF165 is the major isoform. In some embodiments, the VEGFA of the present disclosure refers to a VEGFA from a human, such as VEGF165 from a human. In one embodiment, the amino acid sequence of VEGFA of the present disclosure refers to the amino acid sequence under Accession No. P15692 (uniprot database).

The term "VEGF receptor" or "VEGFR" refers to the cellular receptor for VEGF, typically a cell surface receptor found on vascular endothelial cells, as well as variants thereof that retain the ability to bind to hVEGF. 3 VEGFR subtypes, VEGFR-1 (flt-1), VEGFR-2 (KDR), and VEGFR-3, constitute the VEGFR family. One example of VEGF receptor is VEGFR1, the fms-like tyrosine kinase (flt-1), which is a transmembrane receptor in the tyrosine kinase family. The Flt-1 receptor contains an extracellular domain, a transmembrane domain, and an intracellular domain with tyrosine kinase activity. The extracellular domain is involved in the binding of VEGF, while the intracellular domain is involved in signaling. Another example of VEGF receptor is VEGFR-2, i.e., flk-1 receptor (also known as KDR). The binding of VEGF to flt receptor results in the formation of at least two high-molecular-weight complexes with apparent molecular weights of 205,000 Daltons and 300,000 Daltons. The 300,000-Dalton complex is believed to be a dimer comprising two receptor molecules binding to a single VEGF molecule. In one embodiment, the VEGFR is VEGF-A receptor flt-1 or flk-1. In one embodiment, the VEGFR is flt-1. Wild-type VEGFR1 may be the polypeptide under Accession No. P17948 in UniProt. In one embodiment of the present disclosure, the "VEGFR" or "VEGF receptor" refers to a polypeptide having the amino acid sequence of wild-type human VEGF receptor, or a polypeptide having substantially the same sequence as the wild-type amino acid sequence. The VEGFR may retain at least 75%, 80%, 85%, 90%, 95%, or 99% of the VEGF-binding activity of the wild-type sequence, or a binding activity equal to or greater than that of the wild-type sequence. In one embodiment, the VEGFR1 of the present disclosure comprises the amino acid sequence set forth in SEQ ID NO: 101 or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 101, or comprises an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 101 and comprising the amino acid sequence set forth in SEQ ID NO: 100 or 102.

The "extracellular domain" or "ECD" refers to the extracellular domain of VEGFR, which is capable of binding to VEGF (e.g., VEGFA) and blocking its activity. In some embodiments, the ECD is determined according to Topological domain of Uniprot. In some embodiments, the ECD described herein also encompasses variant ECDs that retain the functionality of the original extracellular domain but have undergone certain modifications. In some embodiments, the ECD of VEGFR generally comprises or consists of the amino acid sequence set forth in SEQ ID NO: 102 or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 102, or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 102 and comprising ECD2 (e.g., the amino acid sequence set forth in SEQ ID NO: 100). In some embodiments, the ECD of VEGFR comprises ECD2. In some embodiments, ECD2 of the VEGFR generally comprises or consists of the amino acid sequence set forth in SEQ ID NO: 100, or an amino acid sequence having at least 85%, 86%, 87%, 88%, 89%, 90%, 91%, 92%, 93%, 94%, 95%, 96%, 97%, 98%, or 99% identity to the amino acid sequence set forth in SEQ ID NO: 100.

The term "functional fragment" of a protein comprises a portion of the protein, which is a portion or a segment of the intact or complete protein that has fewer amino acid residues than that of the intact or complete protein, but still retains the desired protein functionality. In some embodiments, the functional fragment comprises or consists of an extracellular domain. In some embodiments, the functional fragment comprises or consists of extracellular domain 2 (ECD2).

As used herein, the "VEGF antagonist" refers to a compound that can reduce or inhibit VEGF activity *in vivo*. The VEGF antagonist may bind to one or more VEGF or VEGF receptors or block the binding of one or more VEGF proteins to one or more VEGF receptors. The VEGF antagonist may be, for example, a small molecule, an anti-VEGF antibody or an antigen-binding fragment thereof, one or more VEGF receptors or extracellular domains thereof, or an antibody or an antigen-binding fragment thereof against a VEGF receptor. In one embodiment, the VEGF antagonist is any approved anti-VEGF drug, such as brolucizumab, ranibizumab, or aflibercept. In one example, the VEGF antagonist is an anti-VEGF antibody (such as brolucizumab, ranibizumab, bevacizumab, or a bispecific antibody such as faricimab), an anti-VEGF DARPin (such as abicipar), or a soluble VEGF receptor or a fragment thereof, e.g., a functional fragment, such as the ECD or ECD2.

The terms "full antibody", "full-length antibody", "complete antibody", and "intact antibody" may be used interchangeably herein to refer to a naturally occurring glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2, and CH3. Each light chain consists of a light chain variable region (abbreviated herein as VL) and a light chain constant region. The light chain constant region consists of one domain, CL. The VH region and the VL region can be further divided into hypervariable regions (complementarity-determining regions, or CDRs), with relatively conservative regions (framework regions, or FRs) inserted therebetween. Each VH or VL consists of three CDRs and four FRs, arranged from amino-terminus to carboxyl-terminus in the following order: FR1, CDR1, FR2, CDR2, FR3, CDR3, and FR4. The constant regions are not directly involved in the binding of antibodies to antigens, but exhibit a variety of effector functions.

In some embodiments, the antibody heavy chain constant region HC of the present disclosure is a heavy chain constant region of IgG1, IgG2, IgG3, or IgG4, preferably a heavy chain constant region of IgG1. In some preferred embodiments, the antibody heavy chain constant region HC of the present disclosure:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 91;
(ii) comprises or consists of an amino acid sequence selected from SEQ ID NO: 91; or
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared with an amino acid sequence selected from SEQ ID NO: 91. In some embodiments, the heavy chain constant region of the present disclosure further comprises one or more modifications capable of changing the effector function of the antibody (e.g., ADCC and/or CDC) or changing the half-life thereof.

In some embodiments, the antibody light chain constant region LC of the present disclosure is a lambda or kappa light chain constant region. In some embodiments, the antibody light chain constant region LC of the present disclosure:
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 92;
(ii) comprises or consists of an amino acid sequence selected from SEQ ID NO: 92; or
(iii) comprises or consists of an amino acid sequence having one or more (preferably no more than 20 or 10, and more preferably no more than 5, 4, 3, 2, or 1) amino acid modifications (preferably amino acid substitutions, and more preferably conservative amino acid substitutions) compared with an amino acid sequence selected from SEQ ID NO: 92.

The term "antibody fragment" comprises a portion of an intact antibody. In a preferred embodiment, the antibody fragment is an antigen-binding fragment.

The term "antigen-binding fragment" is a portion or segment of an intact antibody or a complete antibody that has fewer amino acid residues than the intact antibody or the complete antibody, which can bind to an antigen or compete with an intact antibody (i.e., an intact antibody from which the antigen-binding fragment is derived) for binding to an antigen. The antigen-binding fragment may be prepared by recombinant DNA techniques, or by enzymatic or chemical cleavage of an intact antibody. The antigen-binding fragments include, but are not limited to, Fab, Fab', F(ab')2, Fv, single-chain Fv, diabodies, and single-domain antibodies (sdAbs). The Fab fragment is a monovalent fragment consisting of VL, VH, CL, and CH1 domains and can be obtained, for example, by papain digestion of a complete antibody. In addition, the F(ab')2, a dimer of the Fab', is a bivalent antibody fragment produced by pepsin digestion of a portion below disulfide bonds in the hinge region of a complete antibody. The F(ab')2 can be reduced by disrupting the disulfide bonds in the hinge region in a neutral condition, and the F(ab')2 dimer is thus converted into Fab' monomers. The Fab' monomer is substantially a Fab fragment with a hinge region (for more detailed descriptions of other antibody fragments, see Fundamental Immunology, edited by W. E. Paul, Raven Press, N.Y. (1993)). The Fv fragment consists of the VL and VH domains of a single arm of an antibody. In addition, although the two domains VL and VH of the Fv fragment are encoded by separate genes, the domains can be linked, using recombinant methods, by a synthetic linker peptide capable of making these two domains produced as a single protein chain in which the VL and VH regions are paired to form a single-chain Fv (scFv). The antibody fragment can be obtained by a chemical method, a recombinant DNA method, or a protease digestion method.

The "Fab fragment" and "Fab" can be used interchangeably herein to refer to an immunoglobulin fragment consisting of two polypeptide chains and comprising an immunoglobulin heavy chain variable domain VH, a heavy chain constant domain CH1, a light chain variable domain VL, and a light chain constant domain CL, wherein one polypeptide chain comprises, from N-terminus to C-terminus, a VH and one constant region selected from CH1 and CL, and the other polypeptide chain comprises, from N-terminus to C-terminus, a VL and the other constant region selected from CL and CH1, wherein the VH and VL domains are paired to form an antigen-binding site. Herein, a Fab chain comprising a heavy chain constant region CH1 is also referred to as a "Fab heavy chain"; correspondingly, a Fab chain comprising a light chain constant region CL is also referred to as a "Fab light chain".

The term "target" refers to the bound substance against which the binding molecule is directed. The target may be an antigen, or may be a ligand or a receptor.

The term "antigen" refers to a molecule that induces an immune response. Such an immune response may involve antibody production or activation of specific immune cells, or both. Those skilled in the art will appreciate that any macromolecules, including substantially all proteins or peptides, can be used as antigens. In addition, an antigen may be derived from recombinant or genomic DNA.

As used herein, the term "epitope" refers to a portion of an antigen that specifically interacts with an antibody molecule.

The term "target-binding region" as used herein refers to a portion of a multispecific binding molecule (such as a fusion protein), e.g., a bispecific binding molecule, that binds to a particular target or an antigen. The target-binding region may be an antibody or immunoglobulin *per se* or an antibody fragment. Such a target-binding region may or may not have a tertiary structure independent of the remainder of the binding molecule, and may or may not bind to its target as a separate entity. The target-binding region may also be a receptor or a ligand, or a domain of a receptor capable of binding to a ligand. When a target-binding region specifically binds to an antigen, the "target-binding region" is also referred to as an "antigen-binding region". In one embodiment, the antigen-binding region used in the multispecific binding molecule of the present disclosure comprises a VH/VL pair consisting of an antibody light chain variable region (VL) and an antibody heavy chain variable region (VH), and the VH/VL pair may be contained in a single polypeptide chain (e.g., scFv) or in two separate polypeptide chains (e.g., contained in a Fab heavy chain and a Fab light chain, respectively).

When referring to "a target-binding region derived from an antibody", it means that the binding domain of the target-binding region is or is derived from the binding domain of the antibody that specifically binds to an antigen; for example, the heavy chain variable region and/or the light chain variable region of the target-binding region is or is derived from the heavy chain variable region and/or the light chain variable region of the antibody; or 1, 2, 3, 4, 5, or 6 CDRs of the target-binding region are the CDRs of the antibody.

The term "derived from" means that the fragment in the target-binding region is substantially identical to the fragment of the antibody from which it is derived, but has a mutation(s), such as a substitution, a deletion, or an addition, at one or more positions. In one specific embodiment, the mutation is not in the CDR of the antibody. In one specific embodiment, the mutation is not in the variable region of the antibody.

As used herein, the term "monospecific" antibody refers to an antibody having one or more binding regions, each of which binds to the same epitope of the same antigen. For example, the present disclosure provides a monospecific antibody against FGFR2.

The term "multispecific binding molecule" refers to a multispecific binding molecule with at least two specificities, e.g., a bispecific binding molecule, that is, the molecule comprises at least a first target-binding region and a second target-binding region, where the first target-binding region binds to one target, and the second target-binding region binds to another target. Accordingly, the multispecific binding molecule according to the present disclosure comprises specificities for binding to at least two different targets. The molecule according to the present disclosure further encompasses a multispecific molecule comprising multiple target-binding regions, e.g., a trispecific binding molecule.

In some embodiments, when the target-binding regions of the binding molecule are all derived from an antibody, the multispecific binding molecule is also referred to as a multispecific antibody. In the case of multispecific antibodies, the target is an antigen. In some embodiments, the bispecific binding molecule of the present disclosure is a bispecific antibody. As used herein, the term "multispecific antibody" refers to an antibody having at least two antigen-binding regions each binding to a different epitope of the same antigen or a different epitope of a different antigen. The multispecific antibody is an antibody having binding specificities for at least two different antigen epitopes. In one embodiment, provided herein is a bispecific antibody having binding specificities for a first antigen and a second antigen. For example, the present disclosure provides a bispecific antibody against FGFR2 and an additional antigen (e.g., VEGFR2).

In some embodiments, when the multispecific binding molecule of the present disclosure further comprises a target-binding region derived from a protein other than an antibody, the multispecific binding molecule is also referred to as a "fusion protein". For example, in some embodiments, the multispecific binding molecule of the present disclosure is a fusion protein comprising a target-binding region derived from the antibody or the antigen-binding fragment thereof of the present disclosure, and a target-binding region derived from another protein, e.g., a ligand or receptor. For example, the target-binding region is a VEGF receptor or a functional fragment thereof (ECD or ECD2).

Herein, the terms "antibody", "multispecific binding molecule", "multispecific antibody", and "fusion protein" are not mutually exclusive when referred to herein.

The term "variable region" or "variable domain" refers to a domain of a heavy chain or light chain of an antibody involved in the binding of the antibody to an antigen. Variable regions of heavy and light chains of native antibodies generally have similar structures, wherein each domain comprises four conserved framework regions (FRs) and three complementarity-determining regions.

The "complementarity-determining region" or "CDR region" or "CDR" is a region in an antibody variable domain that is highly variable in sequence and forms a structurally defined loop ("hypervariable loop") and/or comprises antigen-contacting residues ("antigen contact sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of the antibody are referred to as HCDR1, HCDR2, and HCDR3, whereas the CDRs located in the light chain variable domain of the antibody are referred to as LCDR1, LCDR2, and LCDR3. In a given amino acid sequence of a light chain variable region or a heavy chain variable region, the exact amino acid sequence boundary of each CDR may be determined using any one or a combination of many well-known antibody CDR assignment schemes including, e.g., Chothia based on the three-dimensional structure of antibodies and the topology of the CDR loops (Chothia et al., (1989) Nature, 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

The following are the regional ranges of the CDRs defined using the Kabat, AbM, Chothia, Contact, and IMGT schemes.

| CDR | Kabat scheme | AbM scheme | Chothia scheme | Contact scheme | IMGT scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L51 |
| LCDR3 (Kabat and Chothia numbering systems) | L89-L97 | L89-L97 | L91-L96 | L89-L96 | L89-L97 |
| HCDR1 (Kabat numbering system) | H31-H35B | H26-H35B | H26-H32...34 | H30-H35B | H26-H35B |
| HCDR1 (Chothia numbering system) | H31-H35 | H26-H35 | H26-H32 | H30-H35 | H26-H33 |
| HCDR2 (Kabat and Chothia numbering systems) | H50-H65 | H50-H58 | H52-H56 | H47-H58 | H51-H56 |
| HCDR3 (Kabat and Chothia numbering systems) | H95-H102 | H95-H102 | H96-H101 | H93-H101 | H93-H102 |

Unless otherwise stated, the term "CDR" or "CDR sequence" used in the present disclosure encompasses CDR sequences determined by any one of the schemes described above. CDRs may also be determined based on having the same Kabat numbering positions as a reference CDR sequence (e.g., any one of the exemplary CDRs of the present disclosure). Unless otherwise stated, in the present disclosure, residue positions of an antibody variable region (including heavy chain variable region residues and light chain variable region residues) are determined according to the Kabat numbering system (Kabat et al., Sequences of Proteins of Immunological Interest, 5th ed., Public Health Service, National Institutes of Health, Bethesda, Md. (1991)).

In one embodiment, the HCDRs and LCDRs in the antibody of the present disclosure are determined according to the Kabat scheme.

The term "Fc domain", "Fc region", or "Fc fragment" is used herein to define a C-terminal region of an immunoglobulin heavy chain, which comprises at least a portion of the constant region. The term includes Fc regions of native sequences and variant Fc regions. A native immunoglobulin "Fc domain" comprises two or three constant domains, i.e., a CH2 domain, a CH3 domain, and optionally a CH4 domain. For example, in native antibodies, an immunoglobulin Fc domain comprises the second and the third constant domains (CH2 domain and CH3 domain) derived from two heavy chains of IgG, IgA, and IgD antibodies; or comprises the second, the third, and the fourth constant domains (CH2 domain, CH3 domain, and CH4 domain) derived from two heavy chains of IgM and IgE antibodies. Unless otherwise stated herein, amino acid residues in the Fc region or the heavy chain constant region are numbered according to the EU numbering system (also known as the EU index) as described in, for example, Edelman, G.M. et al., Proc. Natl. Acad. USA, 63, 78-85 (1969) (https://pubmed.ncbi.nlm.nih.gov/5257969/), see also http://www.imgt.org/IMGTScientificChart/Numbering/Hu_IGHGnber.html. Herein, the term "Fc domain", "Fc region", or "Fc fragment" does not comprise a heavy chain variable region VH and a light chain variable region VL as well as a heavy chain constant region CH1 (with no hinge regions) and a light chain constant region CL of an immunoglobulin, but in some cases, it may comprise a hinge region at the N-terminus of the heavy chain constant region. In some embodiments, the heavy chain constant region Fc suitable for use in the present disclosure is from an antibody heavy chain constant region, e.g., a constant region of human IgG1, IgG2, IgG3, or IgG4, preferably from a constant region of IgG1. In some embodiments, the Fc region comprises or consists of the amino acid sequence set forth in SEQ ID NO: 93 or an amino acid sequence having at least 85%, 90%, 91%, 92%, 93%, 94%, 95%, 96%, 97%, 98%, or 99% identity thereto. In some embodiments of the present disclosure, the Fc fragments form an Fc dimer by dimerization. In some embodiments, the Fc fragments form an Fc heterodimer by heterodimerization. In the case of heterodimerization of the Fc fragments into a heterodimer, the Fc fragments may comprise a mutation(s) for heterodimerization, such as a knob-into-hole mutation. In some embodiments, the lysine at the C-terminus of the Fc region is substituted by alanine. In some embodiments, the Fc comprises or consists of the amino acid sequence set forth in SEQ ID NO: 97 or an amino acid sequence having at least 85%, 90%, 91%, 92%, 93%, 94%, 95%, 96%, 97%, 98%, or 99% identity thereto.

The term "CH1 region" refers to a portion of an antibody heavy chain polypeptide that extends from EU position 118 to EU position 216 (the EU numbering system). In some cases, e.g., when constructing a Fab, the CH1 region may also comprise a portion of the hinge region, e.g., comprising EPKSC. Thus, in some embodiments, the term "CH1" region refers to the portion of an antibody heavy chain polypeptide that extends from EU position 118 to EU position 220 (the EU numbering system). In one embodiment, the CH1 domain comprises the amino acid sequence of ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKVEPKSC (SEQ ID NO: 96).

Examples of "effector functions" of immunoglobulins include: C1q binding and complement-dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake in antigen-presenting cells, down-regulation of cell surface receptors (e.g., B-cell receptors), and B-cell activation.

The term "chimeric antibody" is an antibody molecule in which: (a) a constant region or a portion thereof is modified, substituted, or exchanged such that antigen-binding sites are linked to constant regions of different or modified classes, effector functions, and/or species, or disparate molecules imparting new properties (e.g., enzymes, toxins, hormones, growth factors, and drugs) to chimeric antibodies, etc.; or (b) a variable region or a portion thereof is modified, substituted, or exchanged by variable regions with different or modified antigen-binding specificities. For example, a mouse antibody can be modified by substituting its constant region with a constant region from a human immunoglobulin. Due to the substitution with a human constant region, the chimeric antibody may retain its specificity for recognizing antigens, while having reduced immunogenicity in humans as compared to the original mouse antibody.

The "humanized antibody" is an antibody that retains the antigen-specific reactivity of a non-human antibody (such as a murine antibody) and has lower immunogenicity when administered to humans as a therapeutic agent. This can be achieved, for example, by retaining non-human antigen-binding sites and substituting the remainder of the antibodies by their human counterparts (i.e., the portions of the variable regions not involved in binding are substituted with the corresponding parts of human antibodies).

As used herein, the term "anti", "binding", or "specific binding" means that the binding effect is selective for targets or antigens and may be distinguished from undesired or non-specific interactions. The ability of a binding site to bind to a particular target or an antigen may be determined by flow cytometry, enzyme-linked immunosorbent assay (ELISA), or conventional binding assays known in the art, such as radioimmunoassay (RIA), biolayer interferometry, MSD assay, or surface plasmon resonance (SPR).

The "affinity" or "binding affinity" refers to the inherent binding affinity that reflects the interaction between members of a binding pair. The affinity of molecule X for its partner Y may be generally represented by the dissociation constant (K_{D}), which is a ratio of the dissociation rate constant (K_{dis}) to the association rate constant (Kₒₙ). The affinity can be measured by common methods known in the art. One specific method for measuring affinity is the ForteBio kinetic binding assay.

The "percent identity (%)" of an amino acid sequence refers to the percentage of amino acid residues in a candidate sequence that are the same as those of a specific amino acid sequence shown in this description when aligning the candidate sequence with the specific amino acid sequence shown in this description, with gaps introduced if necessary to achieve maximum percent sequence identity and without considering any conservative substitutions as part of sequence identity. In some embodiments, the present disclosure considers variants of the antibody molecule of the present disclosure that have a considerable degree of identity to the antibody molecule and the sequence thereof specifically disclosed herein. For example, the identity is at least 80%, 85%, 90%, 95%, 97%, 98%, 99%, or higher. The variants may comprise conservative modifications, or be conservatively modified variants.

For polypeptide sequences, the "conservative modifications" include substitutions of, deletions of, or additions to a polypeptide sequence but do not substantially change the desired functional activity of the polypeptide sequence. For example, conservative substitutions often result in the substitution of an amino acid with a chemically similar amino acid. Conservative substitution tables providing functionally similar amino acids are well known in the art. 8 groups comprising amino acids that are conservatively substituted by each other are listed as follows: 1) alanine (A) and glycine (G); 2) aspartic acid (D) and glutamic acid (E); 3) asparagine (N) and glutamine (Q); 4) arginine (R) and lysine (K); 5) isoleucine (I), leucine (L), methionine (M), and valine (V); 6) phenylalanine (F), tyrosine (Y), and tryptophan (W); 7) serine (S) and threonine (T); and 8) cysteine (C) and methionine (M). In some embodiments, the term "conservative sequence modification" is used to refer to an amino acid modification that does not significantly affect or change the binding characteristics for an antigen of interest of the antibody molecule or the binding protein molecule of the present disclosure comprising the amino acid sequence. For example, conservatively modified variants retain at least 80%, 85%, 90%, 95%, 98%, 99%, or higher, such as 100%-110% or higher, binding affinity for an antigen of interest relative to the parent antibody or binding protein.

The term "linker" as used herein refers to any molecule that enables direct connection of different portions of a bispecific binding molecule. Examples of linkers to establish covalent linkages between different portions of a molecule include peptide linkers and non-protein polymers including, but not limited to, polyethylene glycol (PEG), polypropylene glycol, polyalkylene oxide, or copolymers of polyethylene glycol and polypropylene glycol. In some embodiments, the linker is a peptide linker (also referred to as a "linker peptide") and refers to a short amino acid sequence consisting of amino acids, such as glycine (G) and/or serine (S) and/or threonine (T) residues used alone or in combination, or a hinge region from an immunoglobulin, which is used for linking the amino acid sequence of a first portion of a binding molecule to a second portion of the binding molecule. For example, the peptide linker may link a first target-binding region of a binding molecule to a second target-binding region. For example, the peptide linker may also link one portion of an antibody to another portion of the antibody, e.g., a light chain variable region to a heavy chain variable region. Preferably, the peptide linker has a length sufficient to link two entities in a manner that maintains their conformation relative to each other without interference with the desired activities. In one embodiment, the linker peptide has a length of 5-50 amino acids, such as 10, 15, 20, 25, or 30 amino acids. In one embodiment, the linker peptide comprises the amino acid sequence (GS)n (SEQ ID NO: 112), (GGS)n (SEQ ID NO: 113), (GSGGS)n (SEQ ID NO: 114), (GGGGS)n (SEQ ID NO: 115), (GGGS)n (SEQ ID NO: 116), and (GGGGS)nG (SEQ ID NO: 117), wherein n is an integer equal to or greater than 1; for example, n is an integer of 2, 3, 4, 5, 6, 7, 8, 9, or 10. Useful linkers further include glycine-alanine polymers, alanine-serine polymers, and other flexible linkers. In some embodiments, the peptide linker is (GGGGS)n, wherein n = an integer of 1-10, e.g., 1, 2, 3, 4, 5, or 6, such as the sequence set forth in SEQ ID NO: 90.

The term "host cell" refers to a cell into which an exogenous polynucleotide has been introduced, including the progeny of such cells. The host cell includes "transformants" and "transformed cells", which include primary transformed cells and progeny derived therefrom. The host cell is any type of cell system that may be used to produce the antibody molecule of the present disclosure, including eukaryotic cells, e.g., mammalian cells, insect cells, and yeast cells, and prokaryotic cells, e.g., *Escherichia coli* cells. The host cell includes cultured cells, as well as cells within a transgenic animal, a transgenic plant, or a cultured plant tissue or animal tissue.

The term "vector" as used herein refers to a nucleic acid molecule capable of proliferating another nucleic acid to which it is linked. The term includes vectors that serve as self-replicating nucleic acid structures as well as vectors integrated into the genome of a host cell into which they have been introduced. The term "expression vector" refers to a vector comprising a recombinant polynucleotide, which comprises an expression control sequence effectively linked to a nucleotide sequence to be expressed. Expression vectors contain sufficient cis-regulatory elements for expression, and other elements for expression may be provided by a host cell or in an *in vitro* expression system. Expression vectors include all those known in the art, including cosmids, plasmids (e.g., naked or contained in liposomes), and viruses (e.g., lentiviruses, retroviruses, adenoviruses, and adeno-associated viruses) into which recombinant polynucleotides are incorporated.

The terms "individual" and "subject" are used interchangeably and refer to a mammal. The mammals include, but are not limited to, domesticated animals (e.g., cows, sheep, cats, dogs, and horses), primates (e.g., human and non-human primates such as monkeys), rabbits, and rodents (e.g., mice and rats). In particular, the individual is a human.

The term "treatment" (or "treat" or "treating") refers to slowing, interrupting, arresting, alleviating, stopping, lowering, or reversing the onset of symptoms, complications, or biochemical indications of a disease, or alleviating symptoms, or arresting or inhibiting the further progression of the disease, symptom, or disorder.

The term "prevention" (or "prevent" or "preventing") includes the inhibition of the development or progression of a disease or disorder, or symptoms of a specific disease or disorder. In some embodiments, subjects with a family history of cancer are candidates for preventive regimens. Generally, in the context of cancer, the term "prevention" refers to the administration of a drug prior to the appearance of signs or symptoms of cancer, particularly in subjects at risk of cancer.

The term "therapeutic agent" described herein encompasses any substance that is effective in preventing or treating a tumor, e.g., cancer, including a chemotherapeutic agent, a cytokine, a cytotoxic agent, an additional antibody, a small molecule drug, or an immunomodulatory agent (e.g., an immunosuppressant).

The term "cytotoxic agent" used in the present disclosure refers to a substance that inhibits or prevents cell functions and/or causes cell death or cell destruction.

The "chemotherapeutic agent" includes chemical compounds useful in the treatment of cancers or immune system diseases.

The term "small molecule drug" refers to a low-molecular-weight organic compound capable of regulating biological processes. The "small molecule" is defined as a molecule with a molecular weight of less than 10 kD, usually less than 2 kD, and preferably less than 1 kD. The small molecule includes but is not limited to inorganic molecules, organic molecules, organic molecules containing inorganic components, molecules containing radioactive atoms, synthetic molecules, peptide mimetics, and antibody mimetics. As therapeutic agents, small molecules penetrate cells better, are less susceptible to degradation, and are less likely to induce an immune response compared to large molecules.

The term "immunomodulatory agent" used herein refers to a natural or synthetic active agent or drug that suppresses or modulates an immune response. The immune response may be a humoral response or a cellular response. The immunomodulatory agent includes an immunosuppressant. In some embodiments, the immunomodulatory agent of the present disclosure includes an immune checkpoint inhibitor or an immune checkpoint agonist.

The term "effective amount" refers to an amount or dose of the antibody, fragment, composition, or combination of the present disclosure which generates expected effects in a patient in need of treatment or prevention after being administered to the patient in a single dose or multiple doses.

The term "therapeutically effective amount" refers to an amount effective to achieve a desired therapeutic result at a necessary dose for a necessary period of time. The therapeutically effective amount is also such an amount that any toxic or adverse effect of the antibody, fragment thereof, composition, or combination is inferior to the therapeutically beneficial effect. The "therapeutically effective amount" preferably inhibits a measurable parameter or improves a measurable parameter by at least about 40%, and even more preferably by at least about 50%, 55%, 60%, 65%, 70%, 75%, 80%, 85%, 90%, or even 100%, relative to untreated subjects.

The term "prophylactically effective amount" refers to an amount effective to achieve a desired prophylactic result at a necessary dose for a necessary period of time. Generally, since a prophylactic dose is administered to a subject before or at an earlier stage of a disease, a prophylactically effective amount will be less than a therapeutically effective amount.

The term "tumor" refers to all neoplastic cell growth and proliferation, whether malignant or benign, and all pre-cancerous and cancerous cells and tissues. The terms "cancer", "carcinoma", and "tumor" are not mutually exclusive when referred to herein. The term "tumor" encompasses both solid tumors and hematological tumors. The term "anti-tumor effect" or "tumor inhibitory effect" refers to a biological effect that can be demonstrated by a variety of means, including but not limited to, for example, decrease in tumor volume, decrease in the number of tumor cells, decrease in tumor cell proliferation, or decrease in tumor cell viability.

The term "pharmaceutical supplementary material" refers to a diluent, an adjuvant (e.g., Freund's adjuvant (complete and incomplete)), an excipient, a carrier, or a stabilizer, etc., that is administered with an active substance.

The term "pharmaceutical composition" refers to a composition that is present in a form allowing the effective biological activity of an active ingredient contained therein and does not contain additional ingredients having unacceptable toxicity to a subject to which the composition is administered.

The term "pharmaceutical combination or combination product" refers to a non-fixed combination product or a fixed combination product, including but not limited to, a kit. The term "non-fixed combination" means that the active ingredients (e.g., (i) the antibody or the multispecific binding molecule of the present disclosure, and (ii) an additional therapeutic agent) are administered to a patient as separate entities either simultaneously without specific time limitation or sequentially at identical or different time intervals, wherein such administration provides two or more active agents at prophylactically or therapeutically effective levels in the patient. The term "fixed combination" means that two or more active agents are administered to a patient simultaneously in the form of a single entity. The dose and/or time intervals of two or more active agents are preferably selected such that the combined use of the ingredients can result in a therapeutic effect on the disease or disorder greater than that achieved by the use of any one of the ingredients alone. Each ingredient may take a separate formulation form, and their formulation forms may be the same or different.

The term "combination therapy" refers to the administration of two or more therapeutic agents or modalities of treatment (e.g., radiotherapy or surgery) to treat the diseases as described herein. Such administration includes co-administration of these therapeutic agents in a substantially simultaneous manner, for example, in a single capsule with a fixed ratio of active ingredients. Alternatively, such administration includes co-administration of the active ingredients in a variety of or separate containers (e.g., tablets, capsules, powders, and liquids). The powder and/or liquid may be reconstituted or diluted to a desired dose before administration. In addition, such administration further includes using each type of therapeutic agent at approximately the same time or in a sequential manner at different times. In any case, the therapeutic regimen will provide the beneficial effect of the pharmaceutical combination in the treatment of disorders or symptoms described herein.

The "subject/patient/individual sample" refers to a collection of cells or fluids obtained from a patient or a subject. The source of tissue or cell samples can be solid tissues, e.g., from fresh, frozen, and/or preserved organ or tissue samples or biopsy samples or puncture samples; blood or any blood component; body fluids such as tears, vitreous humors, cerebrospinal fluids, amniotic fluids, peritoneal fluids, or interstitial fluids; and cells from a subject at any time during pregnancy or development. In some embodiments, the tissue sample is a tumor tissue. The tissue sample may comprise compounds that are not naturally mixed with tissues, such as preservatives, anticoagulants, buffers, fixatives, nutrients, and antibiotics.

### II. Anti-FGFR2 Antibody

In one aspect, the present disclosure provides an FGFR2 antibody having higher binding affinity for FGFR2. In some embodiments, the FGFR2 antibody of the present disclosure is suitable for use in constructing a target-binding region in a multispecific binding molecule (e.g., a fusion protein).

In some embodiments, the anti-FGFR2 antibody or the antigen-binding fragment thereof of the present disclosure binds to FGFR2 (e.g., human FGFR2 or monkey FGFR2, such as cynomolgus FGFR2) with higher affinity. In some embodiments, the anti-FGFR2 antibody or the antigen-binding fragment thereof of the present disclosure can bind to both human FGFR2 and monkey FGFR2 (e.g., cynomolgus FGFR2). In some embodiments, the affinity of the antibody is determined by biolayer interferometry or surface plasmon resonance. In some embodiments, the anti-FGFR2 antibody of the present disclosure binds to human FGFR2 or monkey FGFR2 (e.g., cynomolgus FGFR2) with an equilibrium dissociation constant (K_{D}) of less than about 10 nM, 5 nM, 4 nM, 3 nM, 2 nM, 1 nM, 0.5 nM, 0.2 nM, 0.1 nM, 0.01 nM, or 1 pM.

In some embodiments, the anti-FGFR2 antibody or the antigen-binding fragment thereof of the present disclosure binds to human or cynomolgus FGFR2 expressed by a cell. In some embodiments, the affinity of the anti-FGFR2 antibody for cell-expressed human or cynomolgus FGFR2 is determined by flow cytometry.

In some embodiments, the anti-FGFR2 antibody or the antigen-binding fragment thereof of the present disclosure can block the binding of FGFR2 to its ligand (e.g., FGF7, FGF10, and/or FGF22).

In some embodiments, the anti-FGFR2 antibody or the antigen-binding fragment thereof of the present disclosure can mediate the internalization of FGFR2 on the surface of a cell (e.g., a tumor cell).

In some embodiments, the anti-FGFR2 antibody or the antigen-binding fragment thereof of the present disclosure can mediate the trogocytosis (ADCT) of tumor cells by effector cells.

In some embodiments, the anti-FGFR2 antibody or the antigen-binding fragment thereof of the present disclosure can kill tumor cells.

In some embodiments, the anti-FGFR2 antibody or the antigen-binding fragment thereof of the present disclosure comprises 3 complementarity-determining regions from a heavy chain variable region (HCDRs): HCDR1, HCDR2, and HCDR3.

In some embodiments, the anti-FGFR2 antibody or the antigen-binding fragment thereof of the present disclosure comprises 3 complementarity-determining regions from a light chain variable region (LCDRs): LCDR1, LCDR2, and LCDR3.

In some embodiments, the anti-FGFR2 antibody or the antigen-binding fragment thereof of the present disclosure comprises 3 complementarity-determining regions (HCDRs) from a heavy chain variable region and 3 complementarity-determining regions (LCDRs) from a light chain variable region.

In some aspects, the anti-FGFR2 antibody or the antigen-binding fragment thereof of the present disclosure comprises a heavy chain variable region (VH). In some aspects, the anti-FGFR2 antibody or the antigen-binding fragment thereof of the present disclosure comprises a light chain variable region (VL). In some aspects, the anti-FGFR2 antibody or the antigen-binding fragment thereof of the present disclosure comprises a heavy chain variable region (VH) and a light chain variable region (VL). In some embodiments, the heavy chain variable region comprises 3 complementarity-determining regions (CDRs) from a heavy chain variable region: HCDR1, HCDR2, and HCDR3. In some embodiments, the light chain variable region comprises 3 complementarity-determining regions (CDRs) from a light chain variable region: LCDR1, LCDR2, and LCDR3.

In some embodiments, the anti-FGFR2 antibody or the antigen-binding fragment thereof of the present disclosure further comprises an antibody heavy chain constant region HC. In some embodiments, the anti-FGFR2 antibody or the antigen-binding fragment thereof of the present disclosure further comprises an antibody light chain constant region LC. In some embodiments, the anti-FGFR2 antibody or the antigen-binding fragment thereof of the present disclosure further comprises a heavy chain constant region HC and a light chain constant region LC.

In some embodiments, the heavy chain variable region VH of the anti-FGFR2 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 2, 12, 22, 27, 37, 44, 54, 63, 69, 71, 73, 75, 77, 79, or 104;
(ii) comprises or consists of the amino acid sequence of SEQ ID NO: 2, 12, 22, 27, 37, 44, 54, 63, 69, 71, 73, 75, 77, 79, or 104; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared with an amino acid sequence of SEQ ID NO: 2, 12, 22, 27, 37, 44, 54, 63, 69, 71, 73, 75, 77, 79, or 104, wherein preferably, the amino acid modifications do not occur in the CDRs.

In some embodiments, the light chain variable region VL of the anti-FGFR2 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 7, 17, 32, 41, 49, 58, 81, 86, 88, or 105;
(ii) comprises or consists of the amino acid sequence of SEQ ID NO: 7, 17, 32, 41, 49, 58, 81, 86, 88, or 105; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared with an amino acid sequence of SEQ ID NO: 7, 17, 32, 41, 49, 58, 81, 86, 88, or 105, wherein preferably, the amino acid modifications do not occur in the CDRs.

In some embodiments, the 3 complementarity-determining regions from the heavy chain variable region (HCDRs) of the anti-FGFR2 antibody of the present disclosure, HCDR1, HCDR2, and HCDR3,
(i) are the three-complementarity-determining regions HCDR1, HCDR2, and HCDR3 in the VH set forth in SEQ ID NO: 2, 12, 22, 27, 37, 44, 54, 63, 69, 71, 73, 75, 77, 79, or 104; or
(ii) jointly comprise a sequence having at least one and no more than 5, 4, 3, 2, or 1 amino acid modification (preferably amino acid substitutions, and preferably conservative substitutions) relative to the sequence of (i);
preferably, the HCDRs are determined according to Kabat.

In some embodiments, the 3 complementarity-determining regions from the light chain variable region (LCDRs) of the anti-FGFR2 antibody of the present disclosure, LCDR1, LCDR2, and LCDR3,
(i) are the three complementarity-determining regions LCDR1, LCDR2, and LCDR3 in the VL set forth in SEQ ID NO: 7, 17, 32, 41, 49, 58, 81, 86, 88, or 105; or
(ii) jointly comprise a sequence having at least one and no more than 5, 4, 3, 2, or 1 amino acid modification (preferably amino acid substitutions, and preferably conservative substitutions) relative to the sequence of (i);
preferably, the LCDRs are determined according to Kabat.

In some embodiments, the HCDR1 comprises or consists of the amino acid sequence set forth in SEQ ID NO: 3, 13, 23, 28, 45, or 64, or an amino acid sequence having 1, 2, or 3 amino acid modifications (preferably amino acid substitutions, and preferably conservative substitutions) compared with the amino acid sequence.

In some embodiments, the HCDR2 comprises or consists of the amino acid sequence set forth in SEQ ID NO: 4, 14, 24, 29, 46, 55, 65, or 66, or an amino acid sequence having 1, 2, or 3 amino acid modifications (preferably amino acid substitutions, and preferably conservative substitutions) compared with the amino acid sequence.

In some embodiments, the HCDR3 comprises or consists of the amino acid sequence set forth in SEQ ID NO: 5, 15, 25, 30, 39, 47, 56, or 67, or an amino acid sequence having 1, 2, or 3 amino acid modifications (preferably amino acid substitutions, and preferably conservative substitutions) compared with the amino acid sequence.

In some embodiments, the LCDR1 comprises or consists of the amino acid sequence set forth in SEQ ID NO: 8, 18, 33, 50, 59, or 82, or an amino acid sequence having 1, 2, or 3 amino acid modifications (preferably amino acid substitutions, and preferably conservative substitutions) compared with the amino acid sequence.

In some embodiments, the LCDR2 comprises or consists of the amino acid sequence set forth in SEQ ID NO: 9, 19, 34, 42, 51, 60, or 83, or an amino acid sequence having 1, 2, or 3 amino acid modifications (preferably amino acid substitutions, and preferably conservative substitutions) compared with the amino acid sequence.

In some embodiments, the LCDR3 comprises or consists of the amino acid sequence set forth in SEQ ID NO: 10, 20, 35, 52, 61, or 84, or an amino acid sequence having 1, 2, or 3 amino acid modifications (preferably amino acid substitutions, and preferably conservative substitutions) compared with the amino acid sequence.

In some embodiments, in the anti-FGFR2 antibody of the present disclosure,
the HCDR1 comprises or consists of the amino acid sequence set forth in SEQ ID NO: 64; the HCDR2 comprises or consists of the amino acid sequence set forth in SEQ ID NO: 65 or 66; the HCDR3 comprises or consists of the amino acid sequence set forth in SEQ ID NO: 67; the LCDR1 comprises or consists of the amino acid sequence set forth in SEQ ID NO: 82; the LCDR2 comprises or consists of the amino acid sequence set forth in SEQ ID NO: 83; and/or the LCDR3 comprises or consists of the amino acid sequence set forth in SEQ ID NO: 84;
the HCDR1 comprises or consists of the amino acid sequence set forth in SEQ ID NO: 3; the HCDR2 comprises or consists of the amino acid sequence set forth in SEQ ID NO: 4; the HCDR3 comprises or consists of the amino acid sequence set forth in SEQ ID NO: 5; the LCDR1 comprises or consists of the amino acid sequence set forth in SEQ ID NO: 8; the LCDR2 comprises or consists of the amino acid sequence set forth in SEQ ID NO: 9; and/or the LCDR3 comprises or consists of the amino acid sequence set forth in SEQ ID NO: 10;
the HCDR1 comprises or consists of the amino acid sequence set forth in SEQ ID NO: 23; the HCDR2 comprises or consists of the amino acid sequence set forth in SEQ ID NO: 24; the HCDR3 comprises or consists of the amino acid sequence set forth in SEQ ID NO: 25; the LCDR1 comprises or consists of the amino acid sequence set forth in SEQ ID NO: 82; the LCDR2 comprises or consists of the amino acid sequence set forth in SEQ ID NO: 83; and/or the LCDR3 comprises or consists of the amino acid sequence set forth in SEQ ID NO: 84;
the HCDR1 comprises or consists of the amino acid sequence set forth in SEQ ID NO: 3; the HCDR2 comprises or consists of the amino acid sequence set forth in SEQ ID NO: 38; the HCDR3 comprises or consists of the amino acid sequence set forth in SEQ ID NO: 39; the LCDR1 comprises or consists of the amino acid sequence set forth in SEQ ID NO: 8; the LCDR2 comprises or consists of the amino acid sequence set forth in SEQ ID NO: 42; and/or the LCDR3 comprises or consists of the amino acid sequence set forth in SEQ ID NO: 10;
the HCDR1 comprises or consists of the amino acid sequence set forth in SEQ ID NO: 28; the HCDR2 comprises or consists of the amino acid sequence set forth in SEQ ID NO: 29; the HCDR3 comprises or consists of the amino acid sequence set forth in SEQ ID NO: 30; the LCDR1 comprises or consists of the amino acid sequence set forth in SEQ ID NO: 33; the LCDR2 comprises or consists of the amino acid sequence set forth in SEQ ID NO: 34; and/or the LCDR3 comprises or consists of the amino acid sequence set forth in SEQ ID NO: 35;
the HCDR1 comprises or consists of the amino acid sequence set forth in SEQ ID NO: 13; the HCDR2 comprises or consists of the amino acid sequence set forth in SEQ ID NO: 14; the HCDR3 comprises or consists of the amino acid sequence set forth in SEQ ID NO: 15; the LCDR1 comprises or consists of the amino acid sequence set forth in SEQ ID NO: 18; the LCDR2 comprises or consists of the amino acid sequence set forth in SEQ ID NO: 19; and/or the LCDR3 comprises or consists of the amino acid sequence set forth in SEQ ID NO: 20;
the HCDR1 comprises or consists of the amino acid sequence set forth in SEQ ID NO: 45; the HCDR2 comprises or consists of the amino acid sequence set forth in SEQ ID NO: 46; the HCDR3 comprises or consists of the amino acid sequence set forth in SEQ ID NO: 47; the LCDR1 comprises or consists of the amino acid sequence set forth in SEQ ID NO: 50; the LCDR2 comprises or consists of the amino acid sequence set forth in SEQ ID NO: 51; and/or the LCDR3 comprises or consists of the amino acid sequence set forth in SEQ ID NO: 52; or
the HCDR1 comprises or consists of the amino acid sequence set forth in SEQ ID NO: 3; the HCDR2 comprises or consists of the amino acid sequence set forth in SEQ ID NO: 55; the HCDR3 comprises or consists of the amino acid sequence set forth in SEQ ID NO: 56; the LCDR1 comprises or consists of the amino acid sequence set forth in SEQ ID NO: 59; the LCDR2 comprises or consists of the amino acid sequence set forth in SEQ ID NO: 60; and/or the LCDR3 comprises or consists of the amino acid sequence set forth in SEQ ID NO: 61.

In some specific embodiments of the present disclosure, the anti-FGFR2 antibody or the antigen-binding fragment thereof of the present disclosure comprises a VH and a VL, wherein
the VH comprises or consists of the amino acid sequence set forth in SEQ ID NO: 2 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 7 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 22 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 37 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 41 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 27 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 32 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 12 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 17 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 44 or 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or 105 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 44 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 105 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 54 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 58 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 63, 69, 71, 73, 75, 77, or 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81, 86, or 88 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 63 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 71 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 73 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
the VH comprises or consists of the amino acid sequence set forth in SEQ ID NO: 77 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto; or
the VH comprises or consists of the amino acid sequence set forth in SEQ ID NO: 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto.

In some specific embodiments of the present disclosure, the anti-FGFR2 antibody or the antigen-binding fragment thereof of the present disclosure comprises
the three complementarity-determining regions HCDR1, HCDR2, and HCDR3 in the VH set forth in SEQ ID NO: 2, and the three complementarity-determining regions LCDR1, LCDR2, and LCDR3 in the VL set forth in SEQ ID NO: 7;
the three complementarity-determining regions HCDR1, HCDR2, and HCDR3 in the VH set forth in SEQ ID NO: 22, and the three complementarity-determining regions LCDR1, LCDR2, and LCDR3 in the VL set forth in SEQ ID NO: 81;
the three complementarity-determining regions HCDR1, HCDR2, and HCDR3 in the VH set forth in SEQ ID NO: 37, and the three complementarity-determining regions LCDR1, LCDR2, and LCDR3 in the VL set forth in SEQ ID NO: 41;
the three complementarity-determining regions HCDR1, HCDR2, and HCDR3 in the VH set forth in SEQ ID NO: 27, and the three complementarity-determining regions LCDR1, LCDR2, and LCDR3 in the VL set forth in SEQ ID NO: 32;
the three complementarity-determining regions HCDR1, HCDR2, and HCDR3 in the VH set forth in SEQ ID NO: 12, and the three complementarity-determining regions LCDR1, LCDR2, and LCDR3 in the VL set forth in SEQ ID NO: 17;
the three complementarity-determining regions HCDR1, HCDR2, and HCDR3 in the VH set forth in SEQ ID NO: 44 or 104, and the three complementarity-determining regions LCDR1, LCDR2, and LCDR3 in the VL set forth in SEQ ID NO: 49 or 105;
the three complementarity-determining regions HCDR1, HCDR2, and HCDR3 in the VH set forth in SEQ ID NO: 54, and the three complementarity-determining regions LCDR1, LCDR2, and LCDR3 in the VL set forth in SEQ ID NO: 58;
the three complementarity-determining regions HCDR1, HCDR2, and HCDR3 in the VH set forth in SEQ ID NO: 22, and the three complementarity-determining regions LCDR1, LCDR2, and LCDR3 in the VL set forth in SEQ ID NO: 81; or
the three complementarity-determining regions HCDR1, HCDR2, and HCDR3 in the VH set forth in SEQ ID NO: 63, 69, 71, 73, 75, 77, or 79, and the three complementarity-determining regions LCDR1, LCDR2, and LCDR3 in the VL set forth in SEQ ID NO: 81, 86, or 88.

In some specific embodiments of the present disclosure, the anti-FGFR2 antibody or the antigen-binding fragment thereof of the present disclosure comprises:
the HCDR1 set forth in SEQ ID NO: 64, the HCDR2 set forth in SEQ ID NO: 65 or 66, the HCDR3 set forth in SEQ ID NO: 67, the LCDR1 set forth in SEQ ID NO: 82, the LCDR2 set forth in SEQ ID NO: 83, and the LCDR3 set forth in SEQ ID NO: 84;
the HCDR1 set forth in SEQ ID NO: 3, the HCDR2 set forth in SEQ ID NO: 4, the HCDR3 set forth in SEQ ID NO: 5, the LCDR1 set forth in SEQ ID NO: 8, the LCDR2 set forth in SEQ ID NO: 9, and the LCDR3 set forth in SEQ ID NO: 31;
the HCDR1 set forth in SEQ ID NO: 23, the HCDR2 set forth in SEQ ID NO: 24, the HCDR3 set forth in SEQ ID NO: 25, the LCDR1 set forth in SEQ ID NO: 82, the LCDR2 set forth in SEQ ID NO: 83, and the LCDR3 set forth in SEQ ID NO: 84;
the HCDR1 set forth in SEQ ID NO: 3, the HCDR2 set forth in SEQ ID NO: 38, the HCDR3 set forth in SEQ ID NO: 39, the LCDR1 set forth in SEQ ID NO: 8, the LCDR2 set forth in SEQ ID NO: 42, and the LCDR3 set forth in SEQ ID NO: 10;
the HCDR1 set forth in SEQ ID NO: 28, the HCDR2 set forth in SEQ ID NO: 29, the HCDR3 set forth in SEQ ID NO: 30, the LCDR1 set forth in SEQ ID NO: 33, the LCDR2 set forth in SEQ ID NO: 34, and the LCDR3 set forth in SEQ ID NO: 35;
the HCDR1 set forth in SEQ ID NO: 13, the HCDR2 set forth in SEQ ID NO: 14, the HCDR3 set forth in SEQ ID NO: 15, the LCDR1 set forth in SEQ ID NO: 18, the LCDR2 set forth in SEQ ID NO: 19, and the LCDR3 set forth in SEQ ID NO: 20;
the HCDR1 set forth in SEQ ID NO: 45, the HCDR2 set forth in SEQ ID NO: 46, the HCDR3 set forth in SEQ ID NO: 47, the LCDR1 set forth in SEQ ID NO: 50, the LCDR2 set forth in SEQ ID NO: 51, and the LCDR3 set forth in SEQ ID NO: 52; or
the HCDR1 set forth in SEQ ID NO: 3, the HCDR2 set forth in SEQ ID NO: 55, the HCDR3 set forth in SEQ ID NO: 56, the LCDR1 set forth in SEQ ID NO: 59, the LCDR2 set forth in SEQ ID NO: 60, and the LCDR3 set forth in SEQ ID NO: 61.

In some specific embodiments of the present disclosure, the anti-FGFR2 antibody or the antigen-binding fragment thereof of the present disclosure comprises a VH and a VL, wherein
the VH comprises or consists of the amino acid sequence set forth in SEQ ID NO: 2, and the VL comprises or consists of the amino acid sequence set forth in SEQ ID NO: 7;
the VH comprises or consists of the amino acid sequence set forth in SEQ ID NO: 22, and the VL comprises or consists of the amino acid sequence set forth in SEQ ID NO: 81;
the VH comprises or consists of the amino acid sequence set forth in SEQ ID NO: 37, and the VL comprises or consists of the amino acid sequence set forth in SEQ ID NO: 41;
the VH comprises or consists of the amino acid sequence set forth in SEQ ID NO: 27, and the VL comprises or consists of the amino acid sequence set forth in SEQ ID NO: 32;
the VH comprises or consists of the amino acid sequence set forth in SEQ ID NO: 12, and the VL comprises or consists of the amino acid sequence set forth in SEQ ID NO: 17;
the VH comprises or consists of the amino acid sequence set forth in SEQ ID NO: 44 or 104, and the VL comprises or consists of the amino acid sequence set forth in SEQ ID NO: 49 or 105;
the VH comprises or consists of the amino acid sequence set forth in SEQ ID NO: 44, and the VL comprises or consists of the amino acid sequence set forth in SEQ ID NO: 49;
the VH comprises or consists of the amino acid sequence set forth in SEQ ID NO: 104, and the VL comprises or consists of the amino acid sequence set forth in SEQ ID NO: 105;
the VH comprises or consists of the amino acid sequence set forth in SEQ ID NO: 54, and the VL comprises or consists of the amino acid sequence set forth in SEQ ID NO: 58;
the VH comprises or consists of the amino acid sequence set forth in SEQ ID NO: 63, 69, 71, 73, 75, 77, or 79, and the VL comprises or consists of the amino acid sequence set forth in SEQ ID NO: 81, 86, or 88;
the VH comprises or consists of the amino acid sequence set forth in SEQ ID NO: 63, and the VL comprises or consists of the amino acid sequence set forth in SEQ ID NO: 81;
the VH comprises or consists of the amino acid sequence set forth in SEQ ID NO: 69, and the VL comprises or consists of the amino acid sequence set forth in SEQ ID NO: 86 or 88;
the VH comprises or consists of the amino acid sequence set forth in SEQ ID NO: 71, and the VL comprises or consists of the amino acid sequence set forth in SEQ ID NO: 86 or 88;
the VH comprises or consists of the amino acid sequence set forth in SEQ ID NO: 73, and the VL comprises or consists of the amino acid sequence set forth in SEQ ID NO: 86 or 88;
the VH comprises or consists of the amino acid sequence set forth in SEQ ID NO: 75, and the VL comprises or consists of the amino acid sequence set forth in SEQ ID NO: 86 or 88;
the VH comprises or consists of the amino acid sequence set forth in SEQ ID NO: 77, and the VL comprises or consists of the amino acid sequence set forth in SEQ ID NO: 86 or 88; or
the VH comprises or consists of the amino acid sequence set forth in SEQ ID NO: 79, and the VL comprises or consists of the amino acid sequence set forth in SEQ ID NO: 86 or 88.

In one embodiment of the present disclosure, the amino acid modifications described herein include amino acid substitutions, insertions, or deletions. In a preferred embodiment, the amino acid modification described in the present disclosure occurs in a region outside CDRs (e.g., in an FR). More preferably, the amino acid modification described in the present disclosure occurs in a region outside the heavy chain variable regions and/or outside the light chain variable regions. Preferably, the amino acid modification described herein is an amino acid substitution, preferably a conservative substitution.

In some embodiments, a deamidated mutation may be performed in the CDRs. For example, amino acid N may be mutated into another amino acid; for example, the HCDR2 comprises an N54E mutation.

In some embodiments, the anti-FGFR2 antibody or the antigen-binding fragment thereof of the present disclosure has one or more of the following properties:
(i) exhibiting identical or similar binding affinity and/or specificity for FGFR2 to the antibody of the present disclosure;
(ii) inhibiting (e.g., competitively inhibiting) the binding of the antibody of the present disclosure to FGFR2;
(iii) binding to the same or an overlapping epitope as the antibody of the present disclosure;
(iv) competing with the antibody of the present disclosure for binding to FGFR2; and
(v) having one or more biological properties of the antibody of the present disclosure.

In some embodiments, the anti-FGFR2 antibody or the antigen-binding fragment thereof of the present disclosure is an antibody in the form of IgG1, IgG2, IgG3, or IgG4, or an antigen-binding fragment thereof, e.g., an antibody in the form of IgG1 or an antigen-binding fragment thereof. In some embodiments, the heavy chain constant region of the anti-FGFR2 antibody of the present disclosure is a constant region of IgG1, IgG2, IgG3, or IgG4, e.g., a constant region of IgG1.

In some embodiments, the light chain constant region of the anti-FGFR2 antibody of the present disclosure is a kappa or lambda light chain constant region, e.g., a kappa light chain constant region.

In some embodiments, the anti-FGFR2 antibody or the antigen-binding fragment thereof of the present disclosure comprises an antibody heavy chain. In some embodiments, the anti-FGFR2 antibody or the antigen-binding fragment thereof of the present disclosure comprises an antibody light chain. In some embodiments, the anti-FGFR2 antibody or the antigen-binding fragment thereof of the present disclosure comprises a heavy chain and a light chain. In some embodiments, the anti-FGFR2 antibody or the antigen-binding fragment thereof of the present disclosure comprises two heavy chains and two light chains.

In one embodiment, the heavy chain of the anti-FGFR2 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 1, 11, 21, 26, 36, 43, 53, 62, 68, 70, 72, 74, 76, 78, or 106;
(ii) comprises or consists of the amino acid sequence of SEQ ID NO: 1, 11, 21, 26, 36, 43, 53, 62, 68, 70, 72, 74, 76, 78, or 106; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared with an amino acid sequence of SEQ ID NO: 1, 11, 21, 26, 36, 43, 53, 62, 68, 70, 72, 74, 76, 78, or 106, wherein preferably, the amino acid modifications do not occur in the CDRs, and more preferably, the amino acid modifications do not occur in the variable region.

In some embodiments, the lysine at the C-terminus of the heavy chain may be substituted by alanine, particularly when used in the construction of the multispecific binding molecule, e.g., when linked to other target-binding regions at the C-terminus, with or without a linker.

In some embodiments, the light chain of the anti-FGFR2 antibody of the present disclosure
(i) comprises or consists of an amino acid sequence having at least 90%, 91%, 92%, 93%, 94%, 95%, 96%, 97%, 98%, or 99% identity to the amino acid sequence of SEQ ID NO: 6, 16, 31, 40, 48, 57, 80, 85, 87, or 107;
(ii) comprises or consists of the amino acid sequence of SEQ ID NO: 6, 16, 31, 40, 48, 57, 80, 85, 87, or 107; or
(iii) comprises or consists of an amino acid sequence having one or more (preferably no more than 10, and more preferably no more than 5, 4, 3, 2, or 1) amino acid modifications (preferably amino acid substitutions, and more preferably conservative amino acid substitutions) compared with an amino acid sequence of SEQ ID NO: 6, 16, 31, 40, 48, 57, 80, 85, 87, or 107, wherein preferably, the amino acid modifications do not occur in the CDRs, and more preferably, the amino acid modifications do not occur in the variable region.

In some specific embodiments of the present disclosure, the anti-FGFR2 antibody or the antigen-binding fragment thereof of the present disclosure comprises a heavy chain and a light chain, wherein
i. the heavy chain comprises or consists of the amino acid sequence set forth in SEQ ID NO: 1 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 6 or an amino acid sequence having at least 90%, 91%, 92%, 93%, 94%, 95%, 96%, 97%, 98%, or 99% identity thereto;
ii. the heavy chain comprises or consists of the amino acid sequence set forth in SEQ ID NO: 21 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
iii. the heavy chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 40 or an amino acid sequence having at least 90%, 91%, 92%, 93%, 94%, 95%, 96%, 97%, 98%, or 99% identity thereto;
iv. the heavy chain comprises or consists of the amino acid sequence set forth in SEQ ID NO: 26 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
v. the heavy chain comprises or consists of the amino acid sequence set forth in SEQ ID NO: 11 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 16 or an amino acid sequence having at least 90%, 91%, 92%, 93%, 94%, 95%, 96%, 97%, 98%, or 99% identity thereto;
vi. the heavy chain comprises or consists of the amino acid sequence set forth in SEQ ID NO: 43 or 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or 107 or an amino acid sequence having at least 90%, 91%, 92%, 93%, 94%, 95%, 96%, 97%, 98%, or 99% identity thereto;
vii. the heavy chain comprises or consists of the amino acid sequence set forth in SEQ ID NO: 43 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or an amino acid sequence having at least 90%, 91%, 92%, 93%, 94%, 95%, 96%, 97%, 98%, or 99% identity thereto;
viii. the heavy chain comprises or consists of the amino acid sequence set forth in SEQ ID NO: 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 107 or an amino acid sequence having at least 90%, 91%, 92%, 93%, 94%, 95%, 96%, 97%, 98%, or 99% identity thereto;
ix. the heavy chain comprises or consists of the amino acid sequence set forth in SEQ ID NO: 53 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 57 or an amino acid sequence having at least 90%, 91%, 92%, 93%, 94%, 95%, 96%, 97%, 98%, or 99% identity thereto;
x. the heavy chain comprises or consists of the amino acid sequence set forth in SEQ ID NO: 62, 68, 70, 72, 74, 76, or 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80, 85, or 87 or an amino acid sequence having at least 90%, 91%, 92%, 93%, 94%, 95%, 96%, 97%, 98%, or 99% identity thereto;
xi. the heavy chain comprises or consists of the amino acid sequence set forth in SEQ ID NO: 62 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
xii. the heavy chain comprises or consists of the amino acid sequence set forth in SEQ ID NO: 68 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
xiii. the heavy chain comprises or consists of the amino acid sequence set forth in SEQ ID NO: 70 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
xiv. the heavy chain comprises or consists of the amino acid sequence set forth in SEQ ID NO: 72 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
xv. the heavy chain comprises or consists of the amino acid sequence set forth in SEQ ID NO: 74 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
xvi. the heavy chain comprises or consists of the amino acid sequence set forth in SEQ ID NO: 76 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
xvii. the heavy chain comprises or consists of the amino acid sequence set forth in SEQ ID NO: 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto; or
xviii. on the basis of the heavy chain and light chain in any one of (i)-(xvii) above, the lysine at the C-terminus of the heavy chain is substituted by alanine.

In some specific embodiments of the present disclosure, the anti-FGFR2 antibody or the antigen-binding fragment thereof of the present disclosure comprises a heavy chain and a light chain, wherein
i. the heavy chain comprises or consists of the amino acid sequence set forth in SEQ ID NO: 1, and the light chain comprises or consists of the amino acid sequence set forth in SEQ ID NO: 6;
ii. the heavy chain comprises or consists of the amino acid sequence set forth in SEQ ID NO: 21, and the light chain comprises or consists of the amino acid sequence set forth in SEQ ID NO: 80;
iii. the heavy chain comprises or consists of the amino acid sequence set forth in SEQ ID NO: 36, and the light chain comprises or consists of the amino acid sequence set forth in SEQ ID NO: 40;
iv. the heavy chain comprises or consists of the amino acid sequence set forth in SEQ ID NO: 26, and the light chain comprises or consists of the amino acid sequence set forth in SEQ ID NO: 31;
v. the heavy chain comprises or consists of the amino acid sequence set forth in SEQ ID NO: 11, and the light chain comprises or consists of the amino acid sequence set forth in SEQ ID NO: 16;
vi. the heavy chain comprises or consists of the amino acid sequence set forth in SEQ ID NO: 43 or 106, and the light chain comprises or consists of the amino acid sequence set forth in SEQ ID NO: 48 or 107;
vii. the heavy chain comprises or consists of the amino acid sequence set forth in SEQ ID NO: 43, and the light chain comprises or consists of the amino acid sequence set forth in SEQ ID NO: 48;
viii. the heavy chain comprises or consists of the amino acid sequence set forth in SEQ ID NO: 106, and the light chain comprises or consists of the amino acid sequence set forth in SEQ ID NO: 107;
ix. the heavy chain comprises or consists of the amino acid sequence set forth in SEQ ID NO: 53, and the light chain comprises or consists of the amino acid sequence set forth in SEQ ID NO: 57;
x. the heavy chain comprises or consists of the amino acid sequence set forth in SEQ ID NO: 62, 68, 70, 72, 74, 76, or 78, and the light chain comprises or consists of the amino acid sequence set forth in SEQ ID NO: 80, 85, or 87;
xi. the heavy chain comprises or consists of the amino acid sequence set forth in SEQ ID NO: 62, and the light chain comprises or consists of the amino acid sequence set forth in SEQ ID NO: 80;
xii. the heavy chain comprises or consists of the amino acid sequence set forth in SEQ ID NO: 68, and the light chain comprises or consists of the amino acid sequence set forth in SEQ ID NO: 85 or 87;
xiii. the heavy chain comprises or consists of the amino acid sequence set forth in SEQ ID NO: 70, and the light chain comprises or consists of the amino acid sequence set forth in SEQ ID NO: 85 or 87;
xiv. the heavy chain comprises or consists of the amino acid sequence set forth in SEQ ID NO: 72, and the light chain comprises or consists of the amino acid sequence set forth in SEQ ID NO: 85 or 87;
xv. the heavy chain comprises or consists of the amino acid sequence set forth in SEQ ID NO: 74, and the light chain comprises or consists of the amino acid sequence set forth in SEQ ID NO: 85 or 87;
xvi. the heavy chain comprises or consists of the amino acid sequence set forth in SEQ ID NO: 76, and the light chain comprises or consists of the amino acid sequence set forth in SEQ ID NO: 85 or 87;
xvii. the heavy chain comprises or consists of the amino acid sequence set forth in SEQ ID NO: 78, and the light chain comprises or consists of the amino acid sequence set forth in SEQ ID NO: 85 or 87; or
xviii. on the basis of the heavy chain and light chain in any one of (i)-(xvii) above, the lysine at the C-terminus of the heavy chain is substituted by alanine.

In some embodiments, the anti-FGFR2 antibody is a monoclonal antibody.

In some embodiments, the anti-FGFR2 antibody is humanized.

In one embodiment, the anti-FGFR2 antibody of the present disclosure also encompasses an antibody fragment (e.g., an antigen-binding fragment) thereof, preferably an antibody fragment selected from: a Fab, a Fab', a Fab'-SH, an Fv, a single-chain antibody (e.g., scFv), a (Fab')₂, a single-domain antibody (e.g., VHH), a domain antibody (dAb), a heavy-chain antibody, and a linear antibody. In one embodiment, the anti-FGFR2 antibody fragment of the present disclosure is a Fab comprising the VH and VL, and the CH1 and CL described herein.

In one embodiment, the anti-FGFR2 antibody of the present disclosure may also be a bispecific antibody or a multispecific antibody that specifically binds to FGFR2 and one or more additional antigens, such as VEGFA or VEGFR2.

### III. Multispecific Binding Molecule

In some embodiments, the present disclosure relates to a multispecific binding molecule, e.g., comprising one binding specificity for FGFR2 and additional binding specificities for one or more molecules (e.g., VEGFA such as VEGF165, or e.g., VEGFR2).

In some embodiments, the multispecific binding molecule is a bispecific binding molecule. In some embodiments, the multispecific binding molecule, e.g., the bispecific binding molecule, is a fusion protein.

Therefore, one aspect of the present disclosure relates to a bispecific binding molecule, comprising
a first target-binding region and a second target-binding region, wherein the first target-binding region specifically binds to FGFR2, and/or the second target-binding region specifically binds to VEGF (e.g., VEGFA such as VEGF165; or e.g., VEGFR2).

In some embodiments, the first target-binding region is derived from an anti-FGFR2 antibody or an antigen-binding fragment thereof, e.g., a Fab of an anti-FGFR2 antibody. In some embodiments, the anti-FGFR2 antibody or the antigen-binding fragment thereof is the anti-FGFR2 antibody or the antigen-binding fragment thereof of the present disclosure.

The first target-binding region suitable for use in the bispecific binding molecule of the present disclosure may comprise or consist of a full-length anti-FGFR2 antibody or an antigen-binding fragment thereof, as long as it can specifically bind to FGFR2, including, but not limited to, for example, a full-length antibody, a Fab, a Fab', a Fab'-SH, an Fv, a single-chain antibody (e.g., scFv), a (Fab')₂, a single-domain antibody (e.g., VHH), a domain antibody (dAb), a heavy-chain antibody, a linear antibody, or the like that specifically binds to FGFR2.

In some embodiments, the second target-binding region specifically binding to VEGF (e.g., VEGFA, such as VEGF165) is derived from a VEGF antagonist. In some embodiments, the VEGF antagonist is an anti-VEGF or anti-VEGFR2 antibody or an antigen-binding fragment thereof, a soluble VEGF receptor, or a fusion protein comprising same or a functional fragment thereof.

In some embodiments, the second target-binding region suitable for use in the bispecific binding molecule of the present disclosure may comprise or consist of a full-length anti-VEGF or anti-VEGFR2 antibody or an antigen-binding fragment thereof, as long as it can specifically bind to VEGF or VEGFR2, including, but not limited to, for example, a full-length antibody, a Fab, a Fab', a Fab'-SH, an Fv, a single-chain antibody (e.g., scFv), a (Fab')₂, a single-domain antibody (e.g., VHH), a domain antibody (dAb), a heavy-chain antibody, a linear antibody, or the like that specifically binds to VEGF or VEGFR2.

In some embodiments, the second target-binding region suitable for use in the bispecific binding molecule of the present disclosure may comprise a VEGF receptor or a functional fragment thereof, for example, comprising the extracellular domain (ECD) of the VEGF receptor or a functional fragment thereof (e.g., extracellular domain 2). In some embodiments, the VEGF receptor is VEGFR1. In some embodiments, the second target-binding region may comprise or consist of VEGFR1 or a functional fragment thereof, e.g., the extracellular domain of VEGFR1 or a fragment thereof, such as extracellular domain 2.

In some embodiments, the VEGF receptor is VEGFR1, comprising or consisting of the amino acid sequence set forth in SEQ ID NO: 101, or an amino acid sequence having at least 90%, 91%, 92%, 93%, 94%, 95%, 96%, 97%, 98%, or 99% identity thereto, or comprising an amino acid sequence having at least 90%, 91%, 92%, 93%, 94%, 95%, 96%, 97%, 98%, or 99% identity to the amino acid sequence set forth in SEQ ID NO: 101 and comprising the amino acid sequence set forth in SEQ ID NO: 100 or SEQ ID NO: 102.

In some embodiments, the extracellular domain of the VEGF receptor is an extracellular domain of VEGFR1, for example, comprising or consisting of the following amino acid sequence:
the amino acid sequence set forth in SEQ ID NO: 102; an amino acid sequence having at least 90%, 91%, 92%, 93%, 94%, 95%, 96%, 97%, 98%, or 99% identity to the amino acid sequence set forth in SEQ ID NO: 102; or an amino acid sequence having at least 90%, 91%, 92%, 93%, 94%, 95%, 96%, 97%, 98%, or 99% identity to the amino acid sequence set forth in SEQ ID NO: 102 and comprising the amino acid sequence set forth in SEQ ID NO: 100.

In some embodiments, the functional fragment of the extracellular domain of the VEGF receptor is extracellular domain 2 (ECD2). In some embodiments, the extracellular domain 2 is an extracellular domain 2 of VEGFR1, for example, comprising or consisting of the amino acid sequence set forth in SEQ ID NO: 100 or an amino acid sequence having at least 90%, 91%, 92%, 93%, 94%, 95%, 96%, 97%, 98%, or 99% identity thereto.

In some embodiments, when the second target-binding region contained in the multispecific binding molecule (e.g., bispecific binding molecule) of the present disclosure comprises a VEGF receptor or a functional fragment thereof (e.g., an extracellular domain or a fragment thereof, such as extracellular domain 2), the binding molecule is also referred to as a "fusion protein" or a "fusion protein molecule".

In some embodiments, the multispecific binding molecule (e.g., bispecific binding molecule) of the present disclosure comprises one or two or more Fc regions, e.g., two Fc regions.

The Fc region fragment suitable for use in the antibody molecule of the present disclosure may be any antibody Fc region. The Fc region may include a native sequence Fc region and a variant Fc region. The native sequence Fc domain encompasses naturally occurring Fc sequences of various immunoglobulins, such as Fc regions of various Ig subtypes and allotypes thereof (Gestur Vidarsson et al., IgG subclasses and allotypes: from structure to effector functions, 20 October 2014, doi: 10.3389/fimmu.2014.00520). For example, the Fc region of the antibody of the present disclosure may comprise two or three constant domains, i.e., a CH2 domain, a CH3 domain, and optionally a CH4 domain. In some embodiments, the antibody Fc region may also bear an IgG hinge region or a portion of the IgG hinge region, e.g., an IgG1 hinge region or a portion of the IgG1 hinge region, at the N-terminus.

Preferably, the Fc region of the antibody of the present disclosure comprises CH2-CH3 from the N-terminus to the C-terminus, or comprises hinge region-CH2-CH3 from the N-terminus to the C-terminus. In some embodiments, the Fc region suitable for use in the antibody or the bispecific antibody of the present disclosure is a human IgG Fc, such as a human IgG1 Fc, a human IgG2 Fc, a human IgG3 Fc, or a human IgG4 Fc.

In some embodiments, the Fc constituting the multispecific binding molecule may comprise a mutation that replaces the lysine at the C-terminus (e.g., with alanine), thereby preventing a fragment fused to the C-terminus thereof (e.g., a target-binding region) from cleavage.

In one embodiment, the Fc region is derived from a human IgG1 Fc, e.g., comprising or consisting of the amino acid sequence set forth in SEQ ID NO: 93 or an amino acid sequence having at least 90% identity, e.g., 95%, 96%, 97%, 99%, or higher identity, thereto.

In one embodiment, the Fc region is derived from a human IgG1 Fc, e.g., comprising or consisting of the amino acid sequence set forth in SEQ ID NO: 97 or an amino acid sequence having at least 90% identity, e.g., 95%, 96%, 97%, 99%, or higher identity, thereto, e.g., comprising a substitution of lysine to alanine at the C-terminus.

### ➢ Bispecific binding molecule

In some preferred embodiments, the present disclosure provides a bispecific binding molecule, comprising a first target-binding region specifically binding to FGFR2, a second target-binding region specifically binding to VEGF (e.g., VEGFA, such as VEGF165), and optionally an Fc region.

In some embodiments, the present disclosure provides a bispecific binding molecule, comprising a first target-binding region, a second target-binding region, and an Fc dimer, wherein the first target-binding region is a Fab fragment specifically binding to FGFR2, and the second target-binding region is a VEGF receptor or a functional fragment thereof specifically binding to VEGF, e.g., an extracellular domain.

In some embodiments, the bispecific binding molecule of the present disclosure comprises one or two first target-binding regions and one or two second target-binding regions.

In some embodiments, the bispecific binding molecule of the present disclosure comprises one first target-binding region and one second target-binding region. In some embodiments, the bispecific binding molecule of the present disclosure comprises two first target-binding regions and two second target-binding regions. In some embodiments, the two first target-binding regions are identical. In some embodiments, the two second target-binding regions are identical. In some embodiments, the bispecific binding molecule of the present disclosure comprises two identical first target-binding regions and two identical second target-binding regions.

In one embodiment, the bispecific binding molecule of the present disclosure is a fusion protein.

In some embodiments, the bispecific binding molecule of the present disclosure specifically binds to VEGF, e.g., human VEGFA, such as human VEGF165 protein, for example, with relatively high affinity.

In some embodiments, the bispecific binding molecule or the fragment thereof of the present disclosure binds to FGFR2 (e.g., human FGFR2) with high affinity and binds to VEGF, e.g., human VEGFA, such as human VEGF165 protein, with relatively high affinity.

In some embodiments, the bispecific binding molecule of the present disclosure binds to human VEGF165 protein with an equilibrium dissociation constant (K_{D}) of less than or equal to about 5 nM, 2.5 nM, 2 nM, 1.5 nM, 1 nM, 0.5 nM, 0.4 nM, 0.3 nM, 0.2 nM, or 0.15 nM.

In some embodiments, the bispecific binding molecule or the fragment thereof of the present disclosure binds to human FGFR2 with an equilibrium dissociation constant (K_{D}) of less than or equal to about 10 nM, 9 nM, 8 nM, 7 nM, 6 nM, or 5 nM.

In some embodiments, the bispecific binding molecule or the fragment thereof of the present disclosure is capable of blocking VEGF signaling.

In some embodiments, the bispecific binding molecule or the fragment thereof of the present disclosure is capable of blocking the binding of VEGF165 to its receptor VEGFR2.

In some embodiments, the bispecific binding molecule or the fragment thereof of the present disclosure mediates the internalization of FGFR2 and induces the trogocytosis by effector cells.

In some embodiments, the bispecific binding molecule or the fragment thereof of the present disclosure is capable of killing tumor cells.

In some embodiments, the molecule or the fragment thereof of the present disclosure is effective in treating a tumor.

In one embodiment, the first target-binding region is a Fab fragment of an anti-FGFR2 antibody. In one embodiment, the second target-binding region is a VEGFR receptor such as VEGFR1, or a functional fragment thereof; for example, the fragment comprises or consists of an extracellular domain or a fragment thereof (e.g., a functional fragment such as extracellular domain 2). In one embodiment, one or more second target-binding regions are separately linked to the N-terminus of the Fab fragment heavy chain of the first target-binding region.

In one embodiment, the bispecific binding molecule of the present disclosure comprises a first target-binding region, a second target-binding region, and an Fc region, wherein
the first target-binding region is a Fab fragment of an anti-FGFR2 antibody, comprising a Fab heavy chain VH-CH1 and a Fab light chain VL-CL;
the second target-binding region comprises or consists of a VEGF receptor such as VEGFR1, or a functional fragment thereof (the fragment comprises or consists of an extracellular domain or a fragment thereof, e.g., extracellular domain 2), wherein
the C-terminus of the CH1 of the Fab fragment heavy chain is fused to the N-terminus of the Fc region, and the N-terminus of the extracellular domain of the VEGF receptor is fused to the C-terminus of the Fc region, thereby constituting the heavy chain;
the Fab fragment light chain constitutes the light chain;
optionally, the bispecific binding molecule comprises two heavy chains and two light chains; the two heavy chains may be identical or different, or the two light chains may be identical or different.

In some embodiments, the Fab fragment constituting the first target-binding region is the Fab fragment of the anti-FGFR2 antibody of the present disclosure. When the binding molecule comprises two first target-binding regions, the two Fabs may be identical or different, e.g., binding to different FGFR2 epitopes, or binding to the same FGFR2 epitope but differing in sequence.

In some embodiments, the VEGF receptor is VEGFR1. In some embodiments, the functional fragment comprises or consists of an extracellular domain of VEGFR1 or a fragment thereof. In some embodiments, the fragment of the extracellular domain comprises or consists of ECD2.

In some embodiments, the C-terminal lysine of the Fc region is replaced by alanine. Thus, in some embodiments, the Fc region suitable for constructing the bispecific binding molecule of the present disclosure comprises or consists of the amino acid sequence set forth in SEQ ID NO: 97, or an amino acid sequence having at least 90%, 95%, 96%, 97%, 98%, or 99% identity thereto and comprising alanine at the C-terminus.

In some embodiments, the fusion is direct fusion or fusion via a linker or a linker peptide. In some embodiments, the linker peptide is selected from (GGGGS)n, where n =1, 2, 3, or 4.

In some embodiments, the heavy chain comprises or consists of the amino acid sequence set forth in SEQ ID NO: 89, or an amino acid sequence having at least 85%, 90%, 91%, 92%, 93%, 94%, 95%, 96%, 97%, 98%, or 99% identity thereto;
and/or
the light chain comprises or consists of the amino acid sequence set forth in SEQ ID NO: 85, or an amino acid sequence having at least 85%, 90%, 91%, 92%, 93%, 94%, 95%, 96%, 97%, 98%, or 99% identity thereto.

### IV. Modification of Anti-FGFR2 Antibody or Fragment Thereof or Multispecific Binding Molecule

The molecule of the present disclosure (e.g., the anti-FGFR2 antibody molecule or the fragment thereof or the multispecific binding molecule) may be mutated to give desired properties.

For example, the FR regions contained in the variable regions of the molecule of the present disclosure may be modified to reduce their immunogenicity or remove T cell epitopes.

The molecule of the present disclosure may also be engineered to introduce one or more modifications into the constant region, e.g., the heavy chain constant region, e.g., the Fc region, to alter one or more functional properties of the antibody, such as serum half-life, complement fixation, Fc receptor binding, and/or antigen-dependent cellular cytotoxicity. Furthermore, the molecule of the present disclosure may be chemically modified (e.g., one or more chemical moieties may be attached to the antibody) or be modified to alter its glycosylation, thereby altering one or more functional properties of the molecule again.

In one embodiment, cysteine residues may be increased or decreased, for example, to facilitate the assembly of different chains of the molecule or to increase or decrease the stability of the molecule.

In some embodiments, the heavy chain constant region or Fc region contained in the molecule of the present disclosure may be modified to adjust one or more properties of the antibody, for example, to alter its effector functions, such as ADCC and/or CDC activity, and/or to alter its *in vivo* half-life, such as to enhance ADCC activity, enhance CDC activity, and/or enhance ADCC and CDC activity, or to prolong the *in vivo* half-life of the antibody. In some embodiments, the modification is one or more amino acid substitutions.

In some preferred embodiments, the one or more amino acid substitutions occur at one or more of positions 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 328, 329, 330, 331, 333, 334, 335, 337, 338, 340, 360, 373, 376, 378, 382, 388, 389, 398, 414, 416, 419, 430, 434, 435, 437, 438, and 439 of the heavy chain constant region sequence according to the EU numbering system. In some preferred embodiments, the one or more amino acid substitutions occur at one or more of positions L234, L235, G236, S239, F243, T256, D265, H268, D270, K290, R292, S298, Y300, V305, K326, A330, I332, E333, K334, A339, and P396 of the heavy chain constant region sequence according to the EU numbering system. In some preferred embodiments, the one or more amino acid substitutions are one or more substitutions selected from G236A, S239D, F243L, T256A, K290A, R292P, S298A, Y300L, V305I, A330L, I332E, E333A, K334A, A339T, and P396L according to the EU numbering system. In some preferred embodiments, the one or more amino acid substitutions are one or more substitutions selected from an N297A substitution, an N297Q substitution, an L235A substitution together with an L237A substitution, an L234A substitution together with an L235A substitution, an E233P substitution, an L234V substitution, an L235A substitution, a C236 deletion, a P238A substitution, a D265A substitution, an A327Q substitution, and a P329A substitution according to the EU numbering system. In some preferred embodiments, the one or more amino acid substitutions occur at one or more of positions 235, 239, 243, 292, 300, 330, 332, and 396 of the heavy chain constant region sequence according to the EU numbering system. In some preferred embodiments, the one or more amino acid substitutions are at least one selected from S239D, L235V, F243L, R292P, Y300L, A330L, I332E, and P396L according to the EU numbering system.

In some preferred embodiments, the heavy chain constant region has one or more sets of mutations that occur simultaneously at combinations of positions selected from the following according to the EU numbering system: (1) L235/F243/R292/Y300/P396, (2) F243/R292/Y300/V305/P396, (3) D270/K326/A330/K334, (4) S239/A330/I332, (5) S298/E333/K334, (6) L234/L235/G236/S239/H268/D270/S298, (7) M252/S254/T256, (8) L234/L235/D265, (9) G236/S239/I332, and (10) S239/I332.

In some preferred embodiments, the heavy chain constant region has one or more sets of mutations selected from the following combinations of mutations according to the EU numbering system: (1) L235V/F243L/R292P/Y300L/P396L, (2) F243L/R292P/Y300L/V305I/P396L, (3) D270E/K326D/A330M/K334E, (4) S239D/A330L/I332E, (5) S298A/E333A/K334A, (6) L234Y/L235Q/G236W/S239M/H268D/D270E/S298A, (7) M252Y/S254T/T256E, (8) L234A/L235A/D265A, (9) L234F/L235E/D265A, (10) G236A/S239D/I332E, and (11) S239D/I332E.

In some embodiments, the molecule of the present disclosure may be subjected to post-translational modification to alter the effector function of the antibody, for example, to alter the glycosylation type of the molecule of the present disclosure.

In some embodiments, the glycosylation of the molecule of the present disclosure may be modified. For example, an aglycosylated antibody or binding molecule may be prepared (i.e., the antibody or binding molecule lacks glycosylation). Glycosylation may be altered to, for example, increase the affinity of the molecule of the present disclosure for an antigen. Such carbohydrate modifications may be achieved by, for example, altering one or more glycosylation sites within the antibody sequence. For example, one or more amino acid substitutions may be made to eliminate one or more variable region framework glycosylation sites, thereby eliminating the glycosylation at the site(s). Such aglycosylation may increase the affinity of the antibody for the antigen. See, e.g., U.S. Patent Nos. 5,714,350 and 6,350,861.

Additionally or optionally, an antibody or binding molecule having an altered type of glycosylation may be prepared, such as a hypofucosylated antibody having reduced amounts of fucosyl residues or an antibody or fusion protein having an increased proportion of a bisecting GlcNac structure. Such altered glycosylation has been demonstrated to increase the ADCC ability of the antibody or binding molecule. Such carbohydrate modifications may be achieved by, for example, expressing the antibody or binding molecule in a host cell with an altered glycosylation mechanism. Cells with an altered glycosylation mechanism have been described in the art and can be used as host cells for expressing the molecule of the present disclosure to produce antibodies or binding molecules with altered glycosylation. For example, cell lines Ms704, Ms705, and Ms709 lack the fucosyltransferase gene FUT8 (α(1,6)-fucosyltransferase), and thus antibodies or binding molecules expressed in the Ms704, Ms705, and Ms709 cell lines lack fucose on their carbohydrates. The Ms704, Ms705, and Ms709 FUT8^{-/-} cell lines were produced by the targeted disruption of the FUT8 gene in CHO/DG44 cells using two substitution-type vectors (see U.S. Patent Publication No. 20040110704 and Yamane-Ohnuki et al. (2004) Biotechnol Bioeng 87:614-22). As another example, EP 1,176,195 describes a cell line with a functionally disrupted FUT8 gene (encoding a fucosyltransferase); thus, by reducing or eliminating α-1,6 bond-related enzymes, antibodies expressed in such a cell line exhibit low fucosylation. EP 1,176,195 also describes cell lines with low or no enzymatic activity for adding fucose to the N-acetylglucosamine that binds to the Fc region of the antibody, such as the rat myeloma cell line YB2/0 (ATCC CRL 1662). PCT Publication WO 03/035835 describes Lec13 cells, a variant CHO cell line with a reduced ability to link fucose to Asn(297)-linked carbohydrates, which also causes antibodies expressed in the host cell to exhibit low fucosylation (see also Shields et al. (2002) J. Biol. Chem. 277:26733-26740). Antibodies or binding molecules with modified glycosylation profiles can also be produced in chicken eggs, as described in PCT Publication WO 06/089231. Optionally, antibodies or binding molecules with modified glycosylation profiles can be produced in plant cells such as duckweed. Methods for producing antibodies in a plant system are disclosed in a U.S. patent application corresponding to Alston & Bird LLP Attorney Docket No. 040989/314911, filed on August 11, 2006. PCT Publication WO 99/54342 describes cell lines engineered to express glycoprotein-modifying glycosyltransferases (e.g., β(1,4)-N-acetylglucosaminyltransferase III (GnTIII)) such that antibodies or binding molecules expressed in the engineered cell lines exhibit an increased proportion of bisecting GlcNac structures, which results in increased ADCC activity of the antibodies or binding molecules (see also Umana et al. (1999) Nat. Biotech. 17:176-180). Optionally, fucosidase can be used to cleave fucosyl residues of the antibody; for example, fucosidase, α-L-fucosidase, removes fucosyl residues from the antibody or binding molecule (Tarentino et al. (1975) Biochem. 14:5516-23).

Another modification contemplated by the present disclosure for the molecule herein is PEGylation. An antibody or binding molecule can be PEGylated to, for example, extend the biological (e.g., serum) half-life of the antibody or binding molecule. To PEGylate an antibody or binding molecule, the antibody or binding molecule is typically reacted with polyethylene glycol (PEG), such as a reactive ester or aldehyde derivative of PEG, under conditions in which one or more PEG groups are linked to the antibody or binding molecule. Preferably, PEGylation is performed via an acylation reaction or an alkylation reaction with a reactive PEG molecule (or an analogous reactive watersoluble polymer). The term "polyethylene glycol" as used herein is intended to encompass any one of the forms of PEG that have been used to derivatize other proteins, such as mono(C1-C10)alkoxy polyethylene glycol, aryloxy polyethylene glycol, or polyethylene glycol-maleimide. In certain embodiments, the antibody or binding molecule to be PEGylated is an aglycosylated antibody or binding molecule. Methods for PEGylating proteins are known in the art and can be applied to the molecule of the present disclosure. See, e.g., EPO 154 316 and EP 0 401 384.

Accordingly, the present disclosure relates to an antibody or a fragment thereof or a multispecific binding molecule with enhanced ADCC. In some embodiments, the antibody or the fragment thereof or the multispecific binding molecule with enhanced ADCC is an antibody or a fragment thereof or a multispecific binding molecule having an altered glycosylation type, e.g., an antibody or a fragment thereof or a multispecific binding molecule that is hypofucosylated or afucosylated, or an antibody or a fragment thereof or a multispecific binding molecule having an increased proportion of a bisecting GlcNac structure.

As described herein, they may be obtained by modifying an antibody or a fragment thereof or a multispecific binding molecule or expressing an antibody or a fragment thereof or a multispecific binding molecule in a host cell with an altered glycosylation mechanism.

### V. Encoding Nucleic Acid and Host Cell Comprising Same

In one aspect, the present disclosure provides a nucleic acid encoding any of the above anti-FGFR2 antibodies or fragments thereof or multispecific binding molecules or any one of the chains thereof.

For example, the nucleic acid of the present disclosure comprises a nucleic acid encoding an amino acid sequence selected from any one of SEQ ID NOs: 1, 2, 6, 7, 11, 12, 16, 17, 21, 22, 26, 27, 31, 32, 43, 44, 48, 49, 53, 54, 57, 58, 62, 63, 68-79, 80, 81, 85, 86, 87, 88, and 104-107, or a nucleic acid encoding an amino acid sequence having at least 85%, 90%, 91%, 92%, 93%, 94%, 95%, 96%, 97%, 98%, or 99% identity to an amino acid sequence selected from any one of SEQ ID NOs: 1, 2, 6, 7, 11, 12, 16, 17, 21, 22, 26, 27, 31, 32, 43, 44, 48, 49, 53, 54, 57, 58, 62, 63, 68-79, 80, 81, 85, 86, 87, 88, and 104-107.

As will be appreciated by those skilled in the art, each antibody or polypeptide amino acid sequence may be encoded by a variety of nucleic acid sequences because of codon degeneracy. Nucleic acid sequences encoding the molecule of the present disclosure may be generated using methods well known in the art, e.g., *de novo* solid-phase DNA synthesis, or PCR amplification.

In one aspect, the present disclosure provides a nucleic acid encoding any of the above antibodies or any of the above antibody chains. When expressed in a suitable expression vector, a polypeptide encoded by the nucleic acid is capable of exhibiting binding capacity to human and/or monkey (e.g., cynomolgus) FGFR2 antigen.

In another aspect, the present disclosure provides a nucleic acid encoding any of the above bispecific binding molecules or fusion proteins. When expressed in a suitable expression vector, a polypeptide encoded by the nucleic acid is capable of exhibiting binding capacity to human or monkey (e.g., cynomolgus) FGFR2 antigen.

In one embodiment, nucleic acids encoding each chain of the antibody or the fragment thereof or the multispecific binding molecule of the present disclosure may be in the same vector or in different vectors. In yet another embodiment, nucleic acids encoding each chain of the antibody or the fragment thereof or the multispecific binding molecule of the present disclosure may be introduced into the same or different host cells for expression. Thus, in some embodiments, the method for producing the antibody or the fragment thereof or the multispecific binding molecule of the present disclosure comprises a step of: culturing a host cell comprising a nucleic acid encoding each chain of the molecule in a condition suitable for the expression of the chains to produce the antibody or the fragment thereof or the multispecific binding molecule of the present disclosure.

In one embodiment, provided is a vector comprising the nucleic acid. In one embodiment, the vector is an expression vector, e.g., a eukaryotic expression vector. The vector includes, but is not limited to, a virus, a plasmid, a cosmid, a λ phage, or a yeast artificial chromosome (YAC). In one embodiment, the vector is, for example, a pcDNA vector, such as pcDNA3.1.

In one embodiment, provided is a host cell comprising the nucleic acid or the vector, e.g., for cloning or expressing a vector encoding the anti-FGFR2 antibody or the fragment thereof or the multispecific binding molecule. In one embodiment, the host cell is eukaryotic. In another embodiment, the host cell is selected from a yeast cell, a mammalian cell (e.g., a CHO cell (e.g., CHO-S, such as ExpiCHO-S) or 293 cell (e.g., 293F or HEK293 cell)), and other cells suitable for preparing an antibody or a fragment thereof. In one embodiment, the host cell is prokaryotic, e.g., a bacterium, such as *Escherichia coli.*

In some embodiments, eukaryotic microorganisms, such as filamentous fungi or yeast, are suitable cloning or expression hosts for the vector encoding the antibody. For example, fungus and yeast strains in which a glycosylation pathway has been "humanized" produce antibodies having a partial or full human glycosylation pattern. Host cells suitable for expressing a glycosylated antibody are also derived from multicellular organisms (invertebrates and vertebrates). Vertebrate cells may also be used as hosts. For example, a mammalian cell line engineered to be suitable for suspension growth may be used. Other examples of useful mammalian host cell lines are monkey kidney CV1 line (COS-7) transformed with SV40, human embryonic kidney line (HEK293, 293F, or 293T cells), and the like. Other useful mammalian host cell lines include Chinese hamster ovary (CHO) cells, including DHFR-CHO cells, CHO-S cells, ExpiCHO, and the like; and myeloma cell lines such as Y0, NS0, and Sp2/0. Mammalian host cell lines suitable for producing antibodies are known in the art.

Host cells may also be cells having an altered glycosylation mechanism as described herein, e.g., cells with complete or partial knockout of the FUT8 gene or complete or partial disruption of the FUT8 gene or protein function, e.g., CHO cells with knockout of the FUT8 gene.

### VI. Production and Purification of Anti-FGFR2 Antibody or Fragment Thereof or Multispecific Binding Molecule of the Present Disclosure

In one embodiment, provided is a method for preparing the anti-FGFR2 antibody or the fragment thereof or the multispecific binding molecule of the present disclosure, wherein the method comprises culturing a host cell comprising a nucleic acid encoding the anti-FGFR2 antibody or the fragment thereof or the multispecific binding molecule (e.g., any one and/or more polypeptide chains) or an expression vector comprising the nucleic acid, as provided above, under a condition suitable for expressing the anti-FGFR2 antibody or the fragment thereof or the multispecific binding molecule or the chain thereof, and optionally recovering the anti-FGFR2 antibody or the fragment thereof or the multispecific binding molecule from the host cell (or the host cell medium).

Polynucleotides encoding the polypeptide chains of the anti-FGFR2 antibody or the fragment thereof or the multispecific binding molecule of the present disclosure may be inserted into one or more vectors for further cloning and/or expression in host cells. Methods known to those skilled in the art can be used to construct expression vectors. Once the expression vector comprising one or more nucleic acid molecules of the present disclosure has been prepared for expression, the expression vector may be transfected or introduced into suitable host cells. Various techniques may be used for this purpose, for example, protoplast fusion, calcium phosphate precipitation, electroporation, retroviral transduction, viral transfection, biolistics, liposome-based transfection, or other conventional techniques.

The anti-FGFR2 antibody or the fragment thereof or the multispecific binding molecule prepared as described herein may be purified by known prior art such as high-performance liquid chromatography, ion exchange chromatography, gel electrophoresis, affinity chromatography, and size exclusion chromatography. The actual conditions used for purifying a particular protein also depend on factors such as net charge, hydrophobicity, and hydrophilicity, and these will be apparent to those skilled in the art.

The purity of the anti-FGFR2 antibody or the fragment thereof or the multispecific binding molecule of the present disclosure may be determined by any one of a variety of well-known analytical methods including size exclusion chromatography, gel electrophoresis, high performance liquid chromatography, and the like.

### VII. Assay for Anti-FGFR2 Antibody or Fragment Thereof or Multispecific Binding Molecule

The anti-FGFR2 antibody or the fragment thereof or the multispecific binding molecule provided herein may be identified, screened, or characterized for physical/chemical properties and/or biological activities thereof through a variety of assays known in the art.

In one aspect, the anti-FGFR2 antibody or the fragment thereof or the multispecific binding molecule of the present disclosure is assayed for its binding activity to a target (e.g., an antigen, e.g., a free antigen or an antigen expressed on a cell), for example, by known methods such as bio-layer interferometry, ELISA, and flow cytometry. The binding to VEGF and/or FGFR2 (or VEGF and/or FGFR2 expressed on a cell) can be determined using methods known in the art, and exemplary methods are disclosed herein, such as the method shown in Example 4 or 8. In some embodiments, the binding is determined by radioimmunoassay (RIA), biolayer interferometry (BLI), electrochemiluminescence (ECL), surface plasmon resonance (SPR), or flow cytometry (FACS).

The present disclosure also provides an assay for identifying the biological activity of the anti-FGFR2 antibody or the fragment thereof or the multispecific binding molecule. The biological activity is selected from the properties of the anti-FGFR2 antibody or the fragment thereof or the multispecific binding molecule of the present disclosure.

For example, the binding activity of the molecule of the present disclosure to a cell expressing VEGF and/or FGFR2 may be determined by methods known in the art, such as fluorescent reporter molecule assay and flow cytometry, or by the exemplary methods disclosed in the examples herein. For example, the binding of the antibody molecule of the present disclosure to VEGF and/or FGFR2 expressed on a cell is determined by the methods shown in Example 3 or 7.

For example, the blocking of the binding ability of FGFR2 to its ligand by the multispecific binding molecule of the present disclosure can be determined by methods known in the art, such as the method shown in Example 9.

For example, the blocking of the binding ability of VEGF (e.g., VEGF165) to its receptor (e.g., VEGFR2) by the multispecific binding molecule of the present disclosure may be determined by methods known in the art, such as the method shown in Example 13.

For example, the receptor internalization and trogocytosis mediated by the molecule of the present disclosure may be determined by methods known in the art, such as the method shown in Example 14.

For example, the inhibitory activity or structural safety of the molecule of the present disclosure against a tumor may be determined by methods known in the art, such as a killing assay against tumor cells (e.g., ADCC effect) or an *in vivo* efficacy study performed on a tumor model, such as the method shown in Example 15 or 16.

Cells for use in any of the above *in vitro* assay methods are primary cells or cell lines, including cells that naturally express or overexpress VEGF (e.g., human or monkey (e.g., cynomolgus)) or FGFR2 (e.g., human or monkey (e.g., cynomolgus) FGFR2), for example, cells overexpressing FGFR2, e.g., 293 cells, CHO cells, or SNU-16 cells.

It will be appreciated that any of the assay methods described above may be performed using a combination of the antibody of the present disclosure and other active agents.

### VIII. Immunoconjugate, Pharmaceutical Composition, Pharmaceutical Combination Product, and Kit of Anti-FGFR2 Antibody or Fragment thereof or Multispecific Binding Molecule of the Present Disclosure

In some embodiments, the present disclosure provides an immunoconjugate comprising any of the anti-FGFR2 antibody or the fragment thereof or the multispecific binding molecule described herein. Preferably, the immunoconjugate comprises one or more additional therapeutic agents (e.g., cytotoxins or small molecule compounds) or markers. In some embodiments, the immunoconjugate is an antibody-drug conjugate (ADC).

In some embodiments, the present disclosure provides a composition or a medicament or a formulation comprising any of the anti-FGFR2 antibody or the fragment thereof, the multispecific binding molecule, or the immunoconjugate of the present disclosure, and preferably the composition is a pharmaceutical composition.

In one embodiment, the composition, e.g., the pharmaceutical composition, comprises a combination of the anti-FGFR2 antibody or the fragment thereof, the multispecific binding molecule, or the immunoconjugate of the present disclosure, and one or more additional therapeutic agents.

The composition or the medicament or the formulation of the present disclosure may further comprise a suitable pharmaceutical supplementary material, such as a pharmaceutical carrier and a pharmaceutical excipient known in the art, including buffers. As used herein, the "pharmaceutical carrier" includes any and all solvents, dispersion media, isotonic agents, absorption delaying agents, and the like that are physiologically compatible.

For use and application of the pharmaceutical supplementary materials, see Hand book of Pharmaceutical Excipients, 8th ed., R. C. Rowe, P. J. Seskey, and S. C. Owen, Pharmaceutical Press, London, Chicago.

The composition or the medicament or the formulation of the present disclosure may be in a variety of forms. These forms include, for example, liquid, semi-solid, and solid dosage forms, such as liquid solutions (e.g., injectable solutions and eye drops), pulvis or suspensions, liposomes, and suppositories. The preferred form depends on the intended mode of administration and therapeutic use.

The medicament or the formulation comprising the anti-FGFR2 antibody or the fragment thereof, the multispecific binding molecule, or the immunoconjugate of the present disclosure may be prepared by mixing the anti-FGFR2 antibody or the fragment thereof or the multispecific binding molecule of the present disclosure having a desired purity with one or more optional pharmaceutical supplementary materials, e.g., in the form of a lyophilized formulation or an aqueous solution.

The composition or the medicament or the formulation of the present disclosure may further comprise more than one active ingredient required by a particular indication treated, preferably those having complementary activity without adversely affecting one another. For example, it is desirable to further provide additional therapeutic agents.

The present disclosure further provides a pharmaceutical combination or a pharmaceutical combination product comprising the anti-FGFR2 antibody or the fragment thereof, the multispecific binding molecule, or the immunoconjugate of the present disclosure and one or more additional therapeutic agents.

The present disclosure further provides a kit of parts, comprising the pharmaceutical combination, wherein, for example, the kit of parts comprises in the same package:
- a first container containing a pharmaceutical composition comprising the anti-FGFR2 antibody or the fragment thereof, the multispecific binding molecule, or the immunoconjugate of the present disclosure; and
- a second container containing a pharmaceutical composition comprising an additional therapeutic agent.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressant).

### IX. Use of Anti-FGFR2 Antibody or Fragment Thereof or Multispecific Binding Molecule and Method for Using Same

In one aspect, the present disclosure relates to the anti-FGFR2 antibody or the fragment thereof or the multispecific binding molecule (as well as the immunoconjugate, the composition, the pharmaceutical composition, the formulation, the combination product, or the like comprising same) of the present disclosure for use in a therapy.

In some embodiments, the present disclosure relates to the anti-FGFR2 antibody or the fragment thereof or the multispecific binding molecule (as well as the immunoconjugate, the composition, the pharmaceutical composition, the formulation, the combination product, or the like comprising same) of the present disclosure for use as a medicament.

In another aspect, the present disclosure provides a method for preventing or treating a disease in a subject, comprising administering to the subject the anti-FGFR2 antibody or the fragment thereof, the multispecific binding molecule, or the immunoconjugate, the composition, the medicament, or the formulation comprising same of the present disclosure. In some embodiments, the present disclosure provides a method for specifically activating a T cell in a subject, comprising administering to the subject the anti-FGFR2 antibody or the fragment thereof, the multispecific binding molecule, or the immunoconjugate, the composition, the medicament, or the formulation comprising same of the present disclosure.

In some embodiments, the disease is an FGFR2-related disease, e.g., a disease in which FGFR2 is abnormally expressed (e.g., highly expressed). In some embodiments, the disease is, for example, a tumor, e.g., a cancer. The cancer may be at an early, intermediate, or advanced stage, or may be a metastatic cancer. In some embodiments, the cancer may be a solid tumor or a hematological tumor. In some embodiments, the cancer is gastric cancer, lung cancer (such as non-small cell lung cancer), esophageal cancer, bile duct cancer (such as unresectable or metastatic bile duct cancer), breast cancer, pancreatic cancer, or colorectal cancer.

In some embodiments, the treatment of the disease will benefit from blocking the VEGF, e.g., VEGFA, signaling pathway.

In some embodiments, the cancer is an FGFR2-positive cancer. In some embodiments, the cancer is characterized by the presence of, or elevated levels of FGFR2 protein and/or nucleic acids in a patient with the cancer (e.g., in the tissues or cells of the patient's cancer) (e.g., compared to the FGFR2 protein and/or nucleic acid levels in the same tissue of a healthy individual, or compared to the FGFR2 protein and/or nucleic acid levels in the adjacent healthy tissue of the patient). For example, the tumor cells of the cancer may have or exhibit elevated levels of FGFR2 protein and/or nucleic acids (e.g., compared to the FGFR2 protein and/or nucleic acid levels in the cells of the same tissue of a healthy individual, or compared to the FGFR2 protein and/or nucleic acid levels in the healthy cells of the same tissue or in the cells of the adjacent healthy tissue of the patient).

The anti-FGFR2 antibody or the fragment thereof or the multispecific binding molecule of the present disclosure (as well as the immunoconjugate, the composition, the pharmaceutical composition, the formulation, the combination product, or the like comprising same) may be administered by any suitable method, including parenteral administration, intrapulmonary administration, intranasal administration, and, if required by locoregional treatment, intralesional administration. Parenteral injection or infusion includes intramuscular, intravenous, intra-arterial, intraperitoneal, or subcutaneous injection or infusion. The administration may be performed by any suitable route, such as injection, e.g., intravenous or subcutaneous injection, to some extent depending on short-term or long-term treatment. Various administration schedules are encompassed herein, including, but not limited to, single administration or multiple administrations at multiple time points, bolus injection, and pulse infusion.

In order to prevent or treat a disease, the appropriate dosage of the anti-FGFR2 antibody or the fragment thereof or the multispecific binding molecule (as well as the immunoconjugate, the composition, the pharmaceutical composition, the formulation, the combination product, or the like comprising same) of the present disclosure (when used alone or in combination with one or more additional therapeutic agents) will depend on the type of the disease to be treated, the type of the antibody, the severity and progression of the disease, purpose of administration (prophylactic or therapeutic), previous therapies, clinical histories of patients, responses to the antibody, and the discretion of an attending physician. The antibody is suitably administered to a patient through a single treatment or through a series of treatments.

In other aspects, the present disclosure provides the anti-FGFR2 antibody or the fragment thereof, the multispecific binding molecule, or the immunoconjugate, the composition, or the combination product comprising same of the present disclosure for the use described herein, e.g., for use in preventing or treating the related disease or disorder described herein.

In other aspects, the present disclosure provides use of the anti-FGFR2 antibody or the fragment thereof, the multispecific binding molecule, or the immunoconjugate, the composition, or the combination product comprising same of the present disclosure in producing or preparing a medicament for the use described herein, e.g., for use in preventing or treating the related disease or disorder described herein.

In some embodiments, the anti-FGFR2 antibody or the fragment thereof or the multispecific binding molecule (as well as the immunoconjugate, the composition, the pharmaceutical composition, the formulation, or the like comprising same) may also be administered in combination with one or more additional therapies, e.g., therapeutic modalities and/or additional therapeutic agents, for the use described herein, e.g., for use in preventing and/or treating the related disease or disorder mentioned herein.

In some embodiments, the therapeutic modality is, for example, surgical therapy or radiotherapy.

In some embodiments, the additional therapeutic agent is, for example, a chemotherapeutic agent, a cytokine, a cytotoxic agent, an additional antibody, a small molecule drug, or an immunomodulatory agent (e.g., an immunosuppressant).

### X. Diagnosis and Detection

In one aspect, the present disclosure further relates to methods for diagnosis and detection (e.g., for diagnostic or non-diagnostic purposes) of the anti-FGFR2 antibody or the fragment thereof or the multispecific binding molecule, and a composition comprising same for diagnosis and detection.

In certain embodiments, the anti-FGFR2 antibody provided herein may be used to detect the presence of FGFR2 in a biological sample. When used herein, the term "detection" includes quantitative and qualitative detections, and exemplary detection methods may involve immunohistochemistry, immunocytochemistry, flow cytometry (e.g., FACS), magnetic beads complexed with antibody molecules, ELISA, and PCR techniques (e.g., RT-PCR). In certain embodiments, the biological sample is a body fluid, such as blood, serum, or plasma.

In certain embodiments, the method comprises contacting a biological sample with the anti-FGFR2 antibody or the fragment thereof or the multispecific binding molecule described herein under a condition that allows the binding thereof to FGFR2, and detecting whether a complex is formed between the anti-FGFR2 antibody or the fragment thereof or the multispecific binding molecule and FGFR2. The formation of the complex indicates the presence of FGFR2. The method may be an *in vitro* or *in vivo* method.

In certain embodiments, provided is a labeled anti-FGFR2 antibody or fragment thereof or multispecific binding molecule. The label includes, but is not limited to, a label or moiety that is detected directly (such as a fluorescent label, a chromophoric label, an electron-dense label, a chemiluminescent label, and a radioactive label), and a moiety that is detected indirectly, such as an enzyme or a ligand, for example, by enzymatic reaction or molecular interaction. In some embodiments, the label is a marker such as biotin or hFc.

In some embodiments provided herein, the sample is obtained prior to the treatment with the anti-FGFR2 antibody or the fragment thereof or the multispecific binding molecule of the present disclosure. In some embodiments, the sample is acquired prior to other therapies. In some embodiments, the sample is acquired during the treatment with other therapies, or after the treatment with other therapies.

In some embodiments, FGFR2 is detected prior to a treatment, for example, prior to an initial treatment or prior to a certain treatment after a treatment interval.

### XI. Specific Embodiments

In one embodiment, the present disclosure relates to the following specific embodiments:
1. An anti-FGFR2 antibody or an antigen-binding fragment thereof, comprising
   i. the three complementarity-determining regions HCDR1, HCDR2, and HCDR3 in the heavy chain variable region VH set forth in SEQ ID NO: 63, 69, 71, 73, 75, 77, or 79, and the three complementarity-determining regions LCDR1, LCDR2, and LCDR3 in the light chain variable region VL set forth in SEQ ID NO: 81, 86, or 88;
   ii. the three complementarity-determining regions HCDR1, HCDR2, and HCDR3 in the heavy chain variable region VH set forth in SEQ ID NO: 2, and the three complementarity-determining regions LCDR1, LCDR2, and LCDR3 in the light chain variable region VL set forth in SEQ ID NO: 7;
   iii. the three complementarity-determining regions HCDR1, HCDR2, and HCDR3 in the heavy chain variable region VH set forth in SEQ ID NO: 22, and the three complementarity-determining regions LCDR1, LCDR2, and LCDR3 in the light chain variable region VL set forth in SEQ ID NO: 81;
   iv. the three complementarity-determining regions HCDR1, HCDR2, and HCDR3 in the heavy chain variable region VH set forth in SEQ ID NO: 37, and the three complementarity-determining regions LCDR1, LCDR2, and LCDR3 in the light chain variable region VL set forth in SEQ ID NO: 41;
   v. the three complementarity-determining regions HCDR1, HCDR2, and HCDR3 in the heavy chain variable region VH set forth in SEQ ID NO: 27, and the three complementarity-determining regions LCDR1, LCDR2, and LCDR3 in the light chain variable region VL set forth in SEQ ID NO: 32;
   vi. the three complementarity-determining regions HCDR1, HCDR2, and HCDR3 in the heavy chain variable region VH set forth in SEQ ID NO: 12, and the three complementarity-determining regions LCDR1, LCDR2, and LCDR3 in the light chain variable region VL set forth in SEQ ID NO: 17;
   vii. the three complementarity-determining regions HCDR1, HCDR2, and HCDR3 in the heavy chain variable region VH set forth in SEQ ID NO: 44 or 104, and the three complementarity-determining regions LCDR1, LCDR2, and LCDR3 in the light chain variable region VL set forth in SEQ ID NO: 49 or 105;
   viii. the three complementarity-determining regions HCDR1, HCDR2, and HCDR3 in the heavy chain variable region VH set forth in SEQ ID NO: 54, and the three complementarity-determining regions LCDR1, LCDR2, and LCDR3 in the light chain variable region VL set forth in SEQ ID NO: 58; or
   ix. the three complementarity-determining regions HCDR1, HCDR2, and HCDR3 in the heavy chain variable region VH set forth in SEQ ID NO: 22, and the three complementarity-determining regions LCDR1, LCDR2, and LCDR3 in the light chain variable region VL set forth in SEQ ID NO: 81.
2. An anti-FGFR2 antibody or an antigen-binding fragment thereof, comprising an HCDR1, an HCDR2, and an HCDR3, and an LCDR1, an LCDR2, and an LCDR3, wherein
   i. the HCDR1 comprises or consists of the amino acid sequence set forth in SEQ ID NO: 64; the HCDR2 comprises or consists of the amino acid sequence set forth in SEQ ID NO: 65 or 66; the HCDR3 comprises or consists of the amino acid sequence set forth in SEQ ID NO: 67; the LCDR1 comprises or consists of the amino acid sequence set forth in SEQ ID NO: 82; the LCDR2 comprises or consists of the amino acid sequence set forth in SEQ ID NO: 83; and the LCDR3 comprises or consists of the amino acid sequence set forth in SEQ ID NO: 84;
   ii. the HCDR1 comprises or consists of the amino acid sequence set forth in SEQ ID NO: 3; the HCDR2 comprises or consists of the amino acid sequence set forth in SEQ ID NO: 4; the HCDR3 comprises or consists of the amino acid sequence set forth in SEQ ID NO: 5; the LCDR1 comprises or consists of the amino acid sequence set forth in SEQ ID NO: 8; the LCDR2 comprises or consists of the amino acid sequence set forth in SEQ ID NO: 9; and the LCDR3 comprises or consists of the amino acid sequence set forth in SEQ ID NO: 10;
   iii. the HCDR1 comprises or consists of the amino acid sequence set forth in SEQ ID NO: 23; the HCDR2 comprises or consists of the amino acid sequence set forth in SEQ ID NO: 24; the HCDR3 comprises or consists of the amino acid sequence set forth in SEQ ID NO: 25; the LCDR1 comprises or consists of the amino acid sequence set forth in SEQ ID NO: 82; the LCDR2 comprises or consists of the amino acid sequence set forth in SEQ ID NO: 83; and the LCDR3 comprises or consists of the amino acid sequence set forth in SEQ ID NO: 84;
   iv. the HCDR1 comprises or consists of the amino acid sequence set forth in SEQ ID NO: 3; the HCDR2 comprises or consists of the amino acid sequence set forth in SEQ ID NO: 38; the HCDR3 comprises or consists of the amino acid sequence set forth in SEQ ID NO: 39; the LCDR1 comprises or consists of the amino acid sequence set forth in SEQ ID NO: 8; the LCDR2 comprises or consists of the amino acid sequence set forth in SEQ ID NO: 42; and the LCDR3 comprises or consists of the amino acid sequence set forth in SEQ ID NO: 10;
   v. the HCDR1 comprises or consists of the amino acid sequence set forth in SEQ ID NO: 28; the HCDR2 comprises or consists of the amino acid sequence set forth in SEQ ID NO: 29; the HCDR3 comprises or consists of the amino acid sequence set forth in SEQ ID NO: 30; the LCDR1 comprises or consists of the amino acid sequence set forth in SEQ ID NO: 33; the LCDR2 comprises or consists of the amino acid sequence set forth in SEQ ID NO: 34; and the LCDR3 comprises or consists of the amino acid sequence set forth in SEQ ID NO: 35;
   vi. the HCDR1 comprises or consists of the amino acid sequence set forth in SEQ ID NO: 13; the HCDR2 comprises or consists of the amino acid sequence set forth in SEQ ID NO: 14; the HCDR3 comprises or consists of the amino acid sequence set forth in SEQ ID NO: 15; the LCDR1 comprises or consists of the amino acid sequence set forth in SEQ ID NO: 18; the LCDR2 comprises or consists of the amino acid sequence set forth in SEQ ID NO: 19; and the LCDR3 comprises or consists of the amino acid sequence set forth in SEQ ID NO: 20;
   vii. the HCDR1 comprises or consists of the amino acid sequence set forth in SEQ ID NO: 45; the HCDR2 comprises or consists of the amino acid sequence set forth in SEQ ID NO: 46; the HCDR3 comprises or consists of the amino acid sequence set forth in SEQ ID NO: 47; the LCDR1 comprises or consists of the amino acid sequence set forth in SEQ ID NO: 50; the LCDR2 comprises or consists of the amino acid sequence set forth in SEQ ID NO: 51; and the LCDR3 comprises or consists of the amino acid sequence set forth in SEQ ID NO: 52; or
   viii. the HCDR1 comprises or consists of the amino acid sequence set forth in SEQ ID NO: 3; the HCDR2 comprises or consists of the amino acid sequence set forth in SEQ ID NO: 55; the HCDR3 comprises or consists of the amino acid sequence set forth in SEQ ID NO: 56; the LCDR1 comprises or consists of the amino acid sequence set forth in SEQ ID NO: 59; the LCDR2 comprises or consists of the amino acid sequence set forth in SEQ ID NO: 60; and the LCDR3 comprises or consists of the amino acid sequence set forth in SEQ ID NO: 61.
3. The antibody or the antigen-binding fragment thereof according to embodiment 1 or 2, comprising a heavy chain variable region VH, wherein the heavy chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 2, 12, 22, 27, 37, 44, 54, 63, 69, 71, 73, 75, 77, 79, or 104; or
   (ii) comprises or consists of the amino acid sequence of SEQ ID NO: 2, 12, 22, 27, 37, 44, 54, 63, 69, 71, 73, 75, 77, 79, or 104.
4. The antibody or the antigen-binding fragment thereof according to any one of embodiments 1-3, comprising a light chain variable region VL, wherein the light chain variable region
   (i) comprises or consists of an amino acid sequence having at least 90%, 91%, 92%, 93%, 94%, 95%, 96%, 97%, 98%, or 99% identity to the amino acid sequence of SEQ ID NO: 7, 17, 32, 41, 49, 58, 81, 86, 88, or 105; or
   (ii) comprises or consists of the amino acid sequence of SEQ ID NO: 7, 17, 32, 41, 49, 58, 81, 86, 88, or 105.
5. The antibody or the antigen-binding fragment thereof according to embodiment 1 or 2, comprising a heavy chain variable region VH and a light chain variable region VL, wherein
   i. the VH comprises or consists of the amino acid sequence set forth in SEQ ID NO: 63, 69, 71, 73, 75, 77, or 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81, 86, or 88 or an amino acid sequence having at least 90%, 91%, 92%, 93%, 94%, 95%, 96%, 97%, 98%, or 99% identity thereto;
   ii. the VH comprises or consists of the amino acid sequence set forth in SEQ ID NO: 63 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
   iii. the VH comprises or consists of the amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   iv. the VH comprises or consists of the amino acid sequence set forth in SEQ ID NO: 71 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   v. the VH comprises or consists of the amino acid sequence set forth in SEQ ID NO: 73 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   vi. the VH comprises or consists of the amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   vii. the VH comprises or consists of the amino acid sequence set forth in SEQ ID NO: 77 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   viii. the VH comprises or consists of the amino acid sequence set forth in SEQ ID NO: 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
   ix. the VH comprises or consists of the amino acid sequence set forth in SEQ ID NO: 2 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 7 or an amino acid sequence having at least 90%, 91%, 92%, 93%, 94%, 95%, 96%, 97%, 98%, or 99% identity thereto;
   x. the VH comprises or consists of the amino acid sequence set forth in SEQ ID NO: 22 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
   xi. the VH comprises or consists of the amino acid sequence set forth in SEQ ID NO: 37 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 41 or an amino acid sequence having at least 90%, 91%, 92%, 93%, 94%, 95%, 96%, 97%, 98%, or 99% identity thereto;
   xii. the VH comprises or consists of the amino acid sequence set forth in SEQ ID NO: 27 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 32 or an amino acid sequence having at least 90%, 91%, 92%, 93%, 94%, 95%, 96%, 97%, 98%, or 99% identity thereto;
   xiii. the VH comprises or consists of the amino acid sequence set forth in SEQ ID NO: 12 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 17 or an amino acid sequence having at least 90%, 91%, 92%, 93%, 94%, 95%, 96%, 97%, 98%, or 99% identity thereto;
   xiv. the VH comprises or consists of the amino acid sequence set forth in SEQ ID NO: 44 or 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or 105 or an amino acid sequence having at least 90%, 91%, 92%, 93%, 94%, 95%, 96%, 97%, 98%, or 99% identity thereto; the VH comprises or consists of the amino acid sequence set forth in SEQ ID NO: 44 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or an amino acid sequence having at least 90%, 91%, 92%, 93%, 94%, 95%, 96%, 97%, 98%, or 99% identity thereto;
   xv. the VH comprises or consists of the amino acid sequence set forth in SEQ ID NO: 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 105 or an amino acid sequence having at least 90%, 91%, 92%, 93%, 94%, 95%, 96%, 97%, 98%, or 99% identity thereto; or
   xvi. the VH comprises or consists of the amino acid sequence set forth in SEQ ID NO: 54 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 58 or an amino acid sequence having at least 90%, 91%, 92%, 93%, 94%, 95%, 96%, 97%, 98%, or 99% identity thereto.
6. The antibody or the antigen-binding fragment thereof according to embodiment 1 or 2, comprising a heavy chain variable region VH and a light chain variable region VL, wherein
   i. the VH comprises or consists of the amino acid sequence set forth in SEQ ID NO: 63, 69, 71, 73, 75, 77, or 79, and the VL comprises or consists of the amino acid sequence set forth in SEQ ID NO: 81, 86, or 88;
   ii. the VH comprises or consists of the amino acid sequence set forth in SEQ ID NO: 63, and the VL comprises or consists of the amino acid sequence set forth in SEQ ID NO: 81;
   iii. the VH comprises or consists of the amino acid sequence set forth in SEQ ID NO: 69, and the VL comprises or consists of the amino acid sequence set forth in SEQ ID NO: 86 or 88;
   iv. the VH comprises or consists of the amino acid sequence set forth in SEQ ID NO: 71, and the VL comprises or consists of the amino acid sequence set forth in SEQ ID NO: 86 or 88;
   v. the VH comprises or consists of the amino acid sequence set forth in SEQ ID NO: 73, and the VL comprises or consists of the amino acid sequence set forth in SEQ ID NO: 86 or 88;
   vi. the VH comprises or consists of the amino acid sequence set forth in SEQ ID NO: 75, and the VL comprises or consists of the amino acid sequence set forth in SEQ ID NO: 86 or 88;
   vii. the VH comprises or consists of the amino acid sequence set forth in SEQ ID NO: 77, and the VL comprises or consists of the amino acid sequence set forth in SEQ ID NO: 86 or 88;
   viii. the VH comprises or consists of the amino acid sequence set forth in SEQ ID NO: 79, and the VL comprises or consists of the amino acid sequence set forth in SEQ ID NO: 86 or 88;
   ix. the VH comprises or consists of the amino acid sequence set forth in SEQ ID NO: 2, and the VL comprises or consists of the amino acid sequence set forth in SEQ ID NO: 7;
   x. the VH comprises or consists of the amino acid sequence set forth in SEQ ID NO: 22, and the VL comprises or consists of the amino acid sequence set forth in SEQ ID NO: 81;
   xi. the VH comprises or consists of the amino acid sequence set forth in SEQ ID NO: 37, and the VL comprises or consists of the amino acid sequence set forth in SEQ ID NO: 41;
   xii. the VH comprises or consists of the amino acid sequence set forth in SEQ ID NO: 27, and the VL comprises or consists of the amino acid sequence set forth in SEQ ID NO: 32;
   xiii. the VH comprises or consists of the amino acid sequence set forth in SEQ ID NO: 12, and the VL comprises or consists of the amino acid sequence set forth in SEQ ID NO: 17;
   xiv. the VH comprises or consists of the amino acid sequence set forth in SEQ ID NO: 44 or 104, and the VL comprises or consists of the amino acid sequence set forth in SEQ ID NO: 49 or 105; the VH comprises or consists of the amino acid sequence set forth in SEQ ID NO: 44, and the VL comprises or consists of the amino acid sequence set forth in SEQ ID NO: 49;
   xv. the VH comprises or consists of the amino acid sequence set forth in SEQ ID NO: 104, and the VL comprises or consists of the amino acid sequence set forth in SEQ ID NO: 105; or
   xvi. the VH comprises or consists of the amino acid sequence set forth in SEQ ID NO: 54, and the VL comprises or consists of the amino acid sequence set forth in SEQ ID NO: 58.
7. The antibody or the antigen-binding fragment thereof according to any one of embodiments 1-6, further comprising a heavy chain constant region HC, wherein, for example, the heavy chain constant region HC of the antibody is a heavy chain constant region of IgG1, IgG2, IgG3, or IgG4, preferably a heavy chain constant region of IgG1.
8. The antibody or the antigen-binding fragment thereof according to embodiment 7, wherein the heavy chain constant region
   (i) comprises or consists of an amino acid sequence having at least 85%, 90%, 91%, 92%, 93%, 94%, 95%, 96%, 97%, 98%, or 99% identity to the amino acid sequence of SEQ ID NO: 91; or
   (ii) comprises or consists of the amino acid sequence of SEQ ID NO: 91.
9. The antibody or the antigen-binding fragment thereof according to any one of embodiments 1-8, further comprising a light chain constant region, wherein, for example, the light chain constant region is a lambda or kappa light chain constant region.
10. The antibody or the antigen-binding fragment thereof according to embodiment 9, wherein the light chain constant region
   (i) comprises or consists of an amino acid sequence having at least 85%, 90%, 91%, 92%, 93%, 94%, 95%, 96%, 97%, 98%, or 99% identity to an amino acid sequence selected from SEQ ID NO: 92; or
   (ii) comprises or consists of an amino acid sequence selected from SEQ ID NO: 92.
11. The antibody or the antigen-binding fragment thereof according to any one of embodiments 1-10, wherein the antibody comprises a heavy chain; the heavy chain
   (i) comprises or consists of an amino acid sequence having at least 90%, 91%, 92%, 93%, 94%, 95%, 96%, 97%, 98%, or 99% identity to the amino acid sequence of SEQ ID NO: 1, 11, 21, 26, 36, 43, 53, 62, 68, 70, 72, 74, 76, 78, or 106;
   (ii) comprises or consists of the amino acid sequence of SEQ ID NO: 1, 11, 21, 26, 36, 43, 53, 62, 68, 70, 72, 74, 76, 78, or 106; or
   (iii) is the heavy chain in (ii), with the lysine at the C-terminus of the heavy chain being substituted by alanine.
12. The antibody or the antigen-binding fragment thereof according to any one of embodiments 1-11, wherein the antibody comprises a light chain; the light chain
   (i) comprises or consists of an amino acid sequence having at least 90%, 91%, 92%, 93%, 94%, 95%, 96%, 97%, 98%, or 99% identity to the amino acid sequence of SEQ ID NO: 6, 16, 31, 40, 48, 57, 80, 85, 87, or 107; or
   (ii) comprises or consists of the amino acid sequence of SEQ ID NO: 6, 16, 31, 40, 48, 57, 80, 85, 87, or 107.
13. The antibody or the antigen-binding fragment thereof according to any one of embodiments 1-12, comprising a heavy chain and a light chain, wherein
   i. the heavy chain comprises or consists of the amino acid sequence set forth in SEQ ID NO: 62, 68, 70, 72, 74, 76, or 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80, 85, or 87 or an amino acid sequence having at least 90%, 91%, 92%, 93%, 94%, 95%, 96%, 97%, 98%, or 99% identity thereto;
   ii. the heavy chain comprises or consists of the amino acid sequence set forth in SEQ ID NO: 62 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
   iii. the heavy chain comprises or consists of the amino acid sequence set forth in SEQ ID NO: 68 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   iv. the heavy chain comprises or consists of the amino acid sequence set forth in SEQ ID NO: 70 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   v. the heavy chain comprises or consists of the amino acid sequence set forth in SEQ ID NO: 72 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   vi. the heavy chain comprises or consists of the amino acid sequence set forth in SEQ ID NO: 74 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   vii. the heavy chain comprises or consists of the amino acid sequence set forth in SEQ ID NO: 76 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   viii. the heavy chain comprises or consists of the amino acid sequence set forth in SEQ ID NO: 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
   ix. the heavy chain comprises or consists of the amino acid sequence set forth in SEQ ID NO: 1 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 6 or an amino acid sequence having at least 90%, 91%, 92%, 93%, 94%, 95%, 96%, 97%, 98%, or 99% identity thereto;
   x. the heavy chain comprises or consists of the amino acid sequence set forth in SEQ ID NO: 21 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
   xi. the heavy chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 40 or an amino acid sequence having at least 90%, 91%, 92%, 93%, 94%, 95%, 96%, 97%, 98%, or 99% identity thereto;
   xii. the heavy chain comprises or consists of the amino acid sequence set forth in SEQ ID NO: 26 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
   xiii. the heavy chain comprises or consists of the amino acid sequence set forth in SEQ ID NO: 11 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 16 or an amino acid sequence having at least 90%, 91%, 92%, 93%, 94%, 95%, 96%, 97%, 98%, or 99% identity thereto;
   xiv. the heavy chain comprises or consists of the amino acid sequence set forth in SEQ ID NO: 43 or 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or 107 or an amino acid sequence having at least 90%, 91%, 92%, 93%, 94%, 95%, 96%, 97%, 98%, or 99% identity thereto;
   xv. the heavy chain comprises or consists of the amino acid sequence set forth in SEQ ID NO: 43 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or an amino acid sequence having at least 90%, 91%, 92%, 93%, 94%, 95%, 96%, 97%, 98%, or 99% identity thereto;
   xvi. the heavy chain comprises or consists of the amino acid sequence set forth in SEQ ID NO: 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 107 or an amino acid sequence having at least 90%, 91%, 92%, 93%, 94%, 95%, 96%, 97%, 98%, or 99% identity thereto;
   xvii. the heavy chain comprises or consists of the amino acid sequence set forth in SEQ ID NO: 53 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 57 or an amino acid sequence having at least 90%, 91%, 92%, 93%, 94%, 95%, 96%, 97%, 98%, or 99% identity thereto; or
   xviii. on the basis of the heavy chain and light chain in any one of i-xvii, the lysine at the C-terminus of the heavy chain is substituted by alanine.
14. The antibody or the antigen-binding fragment thereof according to any one of embodiments 1-13, wherein the antibody is a humanized antibody.
15. The antibody or the antigen-binding fragment thereof according to any one of embodiments 1-14, wherein the antibody is a monoclonal antibody.
16. The antibody or the antigen-binding fragment thereof according to any one of embodiments 1-15, wherein the antigen-binding fragment is an antibody fragment selected from: a Fab, a Fab', a Fab'-SH, an Fv, a single-chain antibody (e.g., scFv), a (Fab')₂, a single-domain antibody (e.g., VHH), a domain antibody (dAb), a heavy-chain antibody, and a linear antibody.
17. The antibody or the antigen-binding fragment thereof according to embodiment 16, wherein the single-chain antibody is an scFv.
18. A multispecific binding molecule, comprising the antibody or the antigen-binding fragment thereof according to any one of embodiments 1-17.
19. The multispecific binding molecule according to embodiment 18, wherein the multispecific binding molecule is a bispecific binding molecule.
20. The multispecific binding molecule according to embodiment 19, wherein the multispecific binding molecule is a bispecific binding molecule, e.g., a fusion protein, specifically binding to FGFR2 and VEGF, e.g., VEGFA, such as VEGF165.
21. The multispecific binding molecule according to embodiment 20, comprising a first target-binding region specifically binding to FGFR2 and a second target-binding region specifically binding to VEGFA, wherein the first target-binding region comprises the antibody or the antigen-binding fragment thereof according to any one of embodiments 1-17.
22. The multispecific binding molecule according to embodiment 21, wherein the second target-binding region comprises a VEGF receptor or a functional fragment thereof, or an antibody or an antigen-binding fragment thereof specifically binding to VEGFR2.
23. The multispecific binding molecule according to embodiment 22, wherein the VEGF receptor is VEGFR1, and/or the functional fragment comprises or consists of:
   an extracellular domain of the VEGF receptor or a fragment thereof; for example, the fragment of the extracellular domain comprises or consists of extracellular domain 2.
24. The multispecific binding molecule according to embodiment 23, wherein
   the extracellular domain of VEGFR1 comprises or consists of the following amino acid sequence:
      i, the amino acid sequence set forth in SEQ ID NO: 102;
      ii, an amino acid sequence having at least 90%, 91%, 92%, 93%, 94%, 95%, 96%, 97%, 98%, or 99% identity to the amino acid sequence set forth in SEQ ID NO: 102; or
      iii, an amino acid sequence having at least 90%, 91%, 92%, 93%, 94%, 95%, 96%, 97%, 98%, or 99% identity to the amino acid sequence set forth in SEQ ID NO: 102 and comprising the amino acid sequence set forth in SEQ ID NO: 100;
   or, extracellular domain 2 of VEGFR1 comprises or consists of the following amino acid sequence:
      i, the amino acid sequence set forth in SEQ ID NO: 100;
      ii, an amino acid sequence having at least 90%, 91%, 92%, 93%, 94%, 95%, 96%, 97%, 98%, or 99% identity to the amino acid sequence set forth in SEQ ID NO: 100; or
      iii, an amino acid sequence having at least 90%, 91%, 92%, 93%, 94%, 95%, 96%, 97%, 98%, or 99% identity to the amino acid sequence set forth in SEQ ID NO: 100 and comprising the amino acid sequence set forth in SEQ ID NO: 100.
25. The multispecific binding molecule according to any one of embodiments 18-24, wherein the first target-binding region is a Fab fragment of the anti-FGFR2 antibody as defined in any one of embodiments 1-17.
26. The multispecific binding molecule according to embodiment 25, wherein the Fab comprises a CH1; the CH1 is a CH1 from IgG1, IgG2, IgG3, or IgG4, preferably a CH1 from IgG1.
27. The multispecific binding molecule according to embodiment 26, wherein the CH1
   (i) comprises or consists of an amino acid sequence having at least 85%, 90%, 91%, 92%, 93%, 94%, 95%, 96%, 97%, 98%, or 99% identity to the amino acid sequence of SEQ ID NO: 96; or
   (ii) comprises or consists of the amino acid sequence of SEQ ID NO: 96.
28. The multispecific binding molecule according to any one of embodiments 18-27, comprising an Fc region, e.g., an Fc dimer, wherein the two Fc regions constituting the Fc dimer are identical or different.
29. The multispecific binding molecule according to embodiment 28, wherein the Fc region is a CH1 from IgG1, IgG2, IgG3, or IgG4, preferably an Fc region from IgG1.
30. The multispecific binding molecule according to embodiment 29, wherein the Fc region comprises or consists of the amino acid sequence set forth in SEQ ID NO: 93 or an amino acid sequence having at least 90% identity, e.g., 95%, 96%, 97%, 99%, or higher identity, thereto.
31. The multispecific binding molecule according to embodiment 29, wherein the Fc region comprises a C-terminal lysine-to-alanine substitution.
32. The multispecific binding molecule according to embodiment 31, wherein the Fc region comprises or consists of the amino acid sequence set forth in SEQ ID NO: 97 or an amino acid sequence having at least 90% identity, e.g., 95%, 96%, 97%, 99%, or higher identity, thereto.
33. The multispecific binding molecule according to any one of embodiments 28-32, wherein the multispecific binding molecule is a bispecific binding molecule and comprises a first target-binding region, a second target-binding region, and an Fc region;
   the first target-binding region is a Fab fragment of an anti-FGFR2 antibody, comprising a Fab heavy chain VH-CH1 and a Fab light chain VL-CL;
   the second target-binding region comprises or consists of an extracellular domain of a VEGF receptor or a fragment thereof (e.g., comprising or consisting of ECD2);
   the C-terminus of the CH1 of the Fab fragment heavy chain is fused to the N-terminus of the Fc region, and the N-terminus of the extracellular domain of the VEGF receptor is fused to the C-terminus of the Fc region, thereby constituting the heavy chain;
   the Fab fragment light chain constitutes the light chain.
34. The multispecific binding molecule according to embodiment 33, wherein the bispecific binding molecule comprises two heavy chains and two light chains; the two heavy chains may be identical or different, or the two light chains may be identical or different.
35. The multispecific binding molecule according to embodiment 33 or 34, wherein the N-terminus of the extracellular domain of the VEGF receptor is fused to the C-terminus of the Fc region via a linker; for example, the linker comprises or consists of (GGGGS)n, wherein n = 1, 2, 3, or 4.
36. The multispecific binding molecule according to embodiment 35, wherein
   the heavy chain comprises or consists of the amino acid sequence set forth in SEQ ID NO: 89, or an amino acid sequence having at least 85%, 90%, 91%, 92%, 93%, 94%, 95%, 96%, 97%, 98%, or 99% identity thereto;
      and/or
   the light chain comprises or consists of the amino acid sequence set forth in SEQ ID NO: 85, or an amino acid sequence having at least 85%, 90%, 91%, 92%, 93%, 94%, 95%, 96%, 97%, 98%, or 99% identity thereto.
37. The antibody or the antigen-binding fragment thereof according to any one of embodiments 1-17 or the multispecific binding molecule according to any one of embodiments 18-36, wherein the antibody or the antigen-binding fragment thereof or the multispecific binding molecule is ADCC-enhanced; for example, the antibody or the antigen-binding fragment thereof or the multispecific binding molecule has a modified glycosylation type, e.g., is hypofucosylated or afucosylated, or has an increased bisecting GlcNac structure.
38. A nucleic acid molecule, encoding any one of the chains of the antibody or the antigen-binding fragment thereof according to any one of embodiments 1-17 or any one of the chains of the multispecific binding molecule according to any one of embodiments 18-36, or consisting of the nucleic acid sequence.
39. An expression vector, comprising the nucleic acid molecule according to embodiment 37, wherein preferably, the expression vector is pCDNA, e.g., pCDNA3.1.
40. A host cell, comprising the nucleic acid molecule according to embodiment 37 or the expression vector according to embodiment 38, wherein preferably, the host cell is prokaryotic or eukaryotic, e.g., a 293 cell or a CHO cell.
41. The host cell according to embodiment 40, wherein the host cell is a host cell with an altered glycosylation mechanism, e.g., a cell with complete or partial knockout of the FUT8 gene or complete or partial disruption of the FUT8 gene or protein functionality, such as a CHO cell with knockout of the FUT8 gene.
42. A method for preparing the antibody or the antigen-binding fragment thereof according to any one of embodiments 1-17 and 37 or the multispecific binding molecule according to any one of embodiments 18-37, comprising: culturing a host cell comprising the nucleic acid molecule according to embodiment 38 or the expression vector according to embodiment 39 or the host cell according to embodiment 40 or 41 in a condition suitable for expressing the chains of the antibody, and optionally recovering the antibody from the host cell (or the host cell culture).
43. An antibody or an antigen-binding fragment thereof or a multispecific binding molecule prepared by the method according to embodiment 42.
44. An immunoconjugate, comprising: the antibody or the antigen-binding fragment thereof according to any one of embodiments 1-17 and 37 or the multispecific binding molecule according to any one of embodiments 18-37.
45. A pharmaceutical composition or a medicament or a formulation, comprising the antibody or the antigen-binding fragment thereof according to any one of embodiments 1-17 and 37 or the multispecific binding molecule according to any one of embodiments 18-37, or the immunoconjugate according to embodiment 44, and optionally a pharmaceutical supplementary material.
46. A pharmaceutical combination product, comprising: the antibody or the antigen-binding fragment thereof according to any one of embodiments 1-17 and 37 or the multispecific binding molecule according to any one of embodiments 18-37, or the immunoconjugate according to embodiment 44, and one or more additional therapeutic agents (e.g., a chemotherapeutic agent, a cytokine, a cytotoxic agent, an additional antibody, a small molecule drug, or an immunomodulatory agent).
47. A method for preventing or treating a cancer in a subject, comprising: administering to the subject an effective amount of the antibody or the antigen-binding fragment thereof according to any one of embodiments 1-17 and 37 or the multispecific binding molecule according to any one of embodiments 18-37, or the immunoconjugate according to embodiment 44, or the pharmaceutical composition or the formulation according to embodiment 45, or the pharmaceutical combination product according to embodiment 46.
48. The method according to embodiment 47, wherein the cancer is an FGFR2-positive cancer; for example, the cancer is characterized by having a protein level and/or nucleic acid level of FGFR2 or having an elevated protein level and/or nucleic acid level of FGFR2 in a tumor cell of the cancer (e.g., compared with the protein level and/or nucleic acid level of FGFR2 in a cell of the same tissue of a healthy individual, or compared with the protein level and/or nucleic acid level of FGFR2 in a healthy cell of the same tissue or a cell of an adjacent healthy tissue of the patient).
49. The method according to embodiment 47 or 48, wherein the cancer is a solid tumor or a hematological tumor, for example, gastric cancer, lung cancer (such as non-small cell lung cancer), esophageal cancer, bile duct cancer (such as unresectable or metastatic bile duct cancer), breast cancer, pancreatic cancer, or colorectal cancer.
50. The method according to any one of embodiments 47-49, further comprising administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent (e.g., an immune checkpoint molecule inhibitor or agonist)).
51. A method for detecting the presence of FGFR2 in a biological sample, comprising
   (i) contacting a biological sample with the antibody or the antigen-binding fragment thereof according to any one of embodiments 1-17 and 37 or the multispecific binding molecule according to any one of embodiments 18-37 in a condition allowing the binding to FGFR2, and
   (ii) detecting whether a complex is formed by the antibody or the multispecific binding molecule and FGFR2,
   wherein formation of the complex indicates the presence of FGFR2.

### Examples

### Example 1. Generation of anti-human FGFR2 antibodies

The hybridoma of the anti-human FGFR2 antibody of the present disclosure may be prepared by the method described below. However, the method for preparing the anti-human FGFR2 antibody of the present disclosure is not limited to this method, and the antibody may also be prepared by other methods known in the art.

### 1.1 Antigen expression

The gene sequence of the extracellular region of the human FGFR2 protein (NP_001138385.1) and the gene sequence of the extracellular region of the cynomolgus FGFR2b protein (XP_028682763.1) were subjected to codon optimization and gene synthesis by General Biology (Anhui) Co., Ltd. The sequences were labeled with a 6×His tag, and then inserted into an expression vector pcDNA3.1(+) to give plasmids encoding huFGFR2-his (SEQ ID NO: 94) and cynoFGFR2-his (SEQ ID NO: 95). Then the two plasmids were separately transfected into ExpiCHO-S cells.

Specifically, the plasmids were transfected into ExpiCHO-S cells using the ExpiCHO^{™} expression system (ThermoFisher, Cat. No. A29133) according to the manufacturer's product instructions to express human FGFR2-his and cynomolgus FGFR2-his proteins. The cells were cultured for 10-12 days after transfection. When the cell viability decreased to 60%-70%, the supernatant was collected, and the expressed antigen with a histidine tag was purified using the NiSepharoseHighPerformance/NiSepharoseexcel affinity chromatography system (Cytiva, 71502810-EH). The purified antigens were concentrated and subjected to sterile filtration. The purity of the antigen was detected by SDS-PAGE and size exclusion chromatography. The results indicated that the purity of the antigens was greater than 90%, and the antigens could be used in the next step.

### 1.2 Animal immunization

5 BALB/c mice (Vital River) + 5 SJL mice (Shanghai SLAC) were immunized with the human FGFR2-his as the immunogen. The ten mice were subjected to four immunizations in total once every two weeks (Table 1). Blood was separately collected intravenously from the mice on day 7 after the second immunization and on day 7 after the third immunization. The titer of the antibody in the serum of the immunized animals was determined by ELISA. After a sufficient increase in the antibody titer was observed, the last booster immunization was performed. Finally, two immunized mice were selected for acquiring antibody-producing cells. Four days after the last booster immunization, spleens of the immunized mice were taken, and lymphocytes isolated from the spleens were fused with myeloma cells to produce hybridomas.

**Table 1. Immunization procedures for BALB/c mice**

| Procedure | Immunization route | Dose |
|---|---|---|
| Primary immunization | Subcutaneous injection | 50 µg protein/mouse |
| First booster immunization | Subcutaneous injection | 25 µg protein/mouse |
| Second booster immunization | Subcutaneous injection | 25 µg protein/mouse |
| Last booster immunization | Subcutaneous injection | 25 µg protein/mouse |

### 1.2 Screening of target hybridomas

Cell culture supernatants of 4800 hybridomas from Example 1.1 were subjected to primary screening by ELISA assay. After the ELISA assay, 198 positive supernatants were selected to detect a KATO-III cell line (a human gastric cancer cell line) by flow cytometry. The hybridoma clones corresponding to the positive supernatants binding to KATO-III cells were confirmed by the next round of ELISA assay. Finally, the above positive hybridoma clones in ELISA were selected for the next subcloning stage.

### 1.3 Subcloning of parental hybridomas

The screened hybridoma cell lines (parental clones) were subcloned to ensure the monoclonality. Subcloning was performed by reseeding the parental clones using a single-step cloning system. The positive subclones from Example 1.2 were transferred to a 96-well cell culture plate. The subclones were screened by ELISA using human FGFR2-his as the antigen, and hybridomas producing antibodies binding to human FGFR2 were obtained. The produced antibodies were designated as: mouse antibody F3F8, mouse antibody G11B8, mouse antibody H3F7-a, mouse antibody E3A1, mouse antibody D12D8, mouse antibody F6B6, mouse antibody A1B2, and mouse antibody A5H12.

### 1.4 Sequencing of anti-FGFR2 antibodies produced by hybridomas

The antibody sequencing was completed by GenScript NGS. The amino acid sequences of the heavy chain variable regions (VHs) and light chain variable regions (VLs) are shown in Table 2.

**Table 2: Amino acid sequences (SEQ ID NO) of heavy chain variable regions and light chain variable regions of anti-FGFR2 antibodies**

| Antibody name | Heavy chain | HC | VH | HCDR1 | HCDR2 | HCDR3 | Light chain | LC | VL | LCDR1 | LCDR2 | LCDR3 |
|---|---|---|---|---|---|---|---|---|---|---|---|---|
| F3F8 | F3F8-H | 1 | 2 | 3 | 4 | 5 | F3F8-L | 6 | 7 | 8 | 9 | 10 |
| G11B8 | G11B8-H | 62 | 63 | 64 | 65 | 67 | G11B8-L | 80 | 81 | 82 | 83 | 84 |
| H3F7-a | H3F7-a-H | 21 | 22 | 23 | 24 | 25 | G11B8-L | 80 | 81 | 82 | 83 | 84 |
| E3A1 | E3A1-H | 36 | 37 | 3 | 38 | 39 | E3A1-L | 40 | 41 | 8 | 42 | 10 |
| D12D8 | D12D8-H | 26 | 27 | 28 | 29 | 30 | D12D8-L | 31 | 32 | 33 | 34 | 35 |
| F6B6 | F6B6-H | 11 | 12 | 13 | 14 | 15 | F6B6-L | 16 | 17 | 18 | 19 | 20 |
| A1B2 | A1B2-H | 43 | 44 | 45 | 46 | 47 | A1B2-L | 48 | 49 | 50 | 51 | 52 |
| A5H12 | A5H12-H | 53 | 54 | 3 | 55 | 56 | A5H12-L | 57 | 58 | 59 | 60 | 61 |

### Example 2: Construction, expression, and purification of anti-FGFR2 chimeric antibodies

The DNA sequences of the mouse antibodies in Table 2 were subjected to codon optimization and gene synthesis by General Biology System (Anhui) Co., Ltd. The genes encoding the VH regions of the antibodies were separately inserted into an expression vector pcDNA3.1(+) comprising a gene encoding a human IgG1 heavy chain constant region (SEQ ID NO: 91) to give plasmids encoding the heavy chains of the anti-FGFR2 antibodies. The genes encoding the VL regions of the antibodies were separately inserted into an expression vector pcDNA3.1(+) comprising a gene encoding a human κ light chain constant region (SEQ ID NO: 92) to give plasmids encoding the light chains of the anti-FGFR2 antibodies.

The plasmids encoding the heavy chains and the light chains of the anti-FGFR2 antibodies were co-transfected into ExpiCHO-S cells to express the murine chimeric antibodies shown in Table 2.

Specifically, the plasmids encoding the heavy and light chains of the anti-FGFR2 antibodies shown in Table 2 were co-transfected into ExpiCHO-S cells using the ExpiCHO^{™} expression system (ThermoFisher, Cat. No. A29133) according to the manufacturer's product instructions to express the anti-FGFR2 chimeric antibodies, and a reference antibody was similarly expressed. The cells were cultured for 10-12 days after transfection. When the cell viability decreased to 60%-70%, the supernatant was collected, and the antibody expressed and secreted into the supernatant was purified using the MabSelectSure protein A affinity chromatography system (GE Healthcare). The purified antibodies were concentrated and subjected to sterile filtration. The purity of the antibody protein was detected by SDS-PAGE and size exclusion chromatography. The results indicated that the purity of the antibodies was greater than 90%, and the antibodies could be used in the next step.

Bemarituzumab, a fully human monoclonal antibody against FGFR2, having the heavy chain amino acid sequence of SEQ ID NO: 98 and the light chain amino acid sequence of SEQ ID NO: 99, derived from PCT Publication No. WO2018213304A1, was used as the reference antibody. The heavy and light chains were constructed into the same expression vector to give plasmids encoding the antibody heavy and light chains. The plasmids were transfected into CHO-K1 cells (ATCC, CCL-61^{TM}) in which the Fut-8 gene was knocked out for expression, and the obtained reference antibody is hereinafter referred to as bemarituzumab.

### Example 3. Binding of anti-FGFR2 chimeric antibodies to human tumor cells

Whether the anti-FGFR2 chimeric antibodies in the present disclosure could bind to tumor cells expressing human FGFR2 protein (e.g., human gastric cancer cell line KATO-III) was determined by flow cytometric cell binding assay.

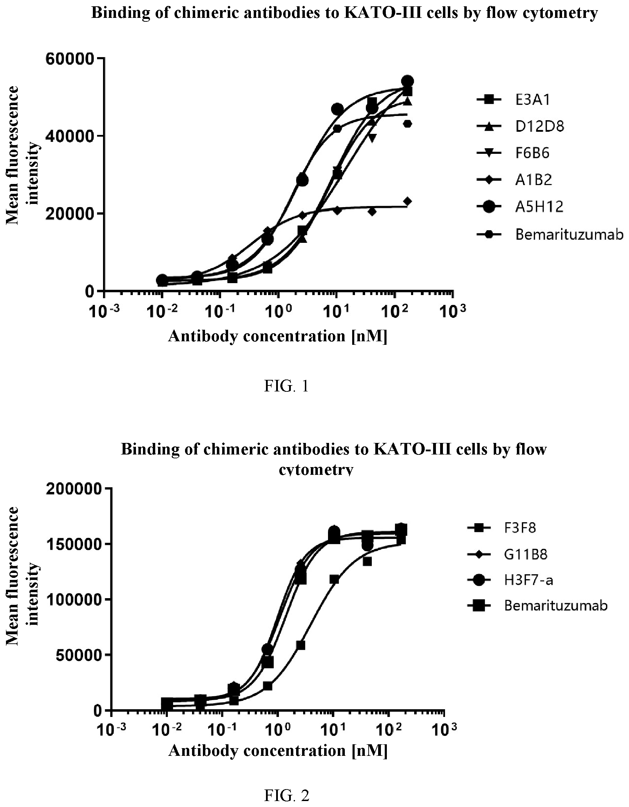

Semi-adherent KATO-III (Cell Bank of Type Culture Collection Committee of the Chinese Academy of Sciences, Cat. No. TCHu229) cells were subjected to enzymatic digestion to give a single-cell suspension. The single-cell suspension was centrifuged at 300× g at room temperature for 4 min, and then the medium was discarded. The cells were resuspended in PBS, seeded into a 96-well V-bottom plate (Nessie, Cat. No. 701211) at 1 × 10⁵ cells/well, and centrifuged at 300× g for 4 min at room temperature, and then the supernatant was discarded. The cells were resuspended in the serially diluted mouse chimeric anti-FGFR2 antibody solutions (serially 4-fold diluted from 25 µg/mL to the 8th concentration) and incubated at 4 °C for 30 min. The cells were washed once with PBS and centrifuged at 300× g for 4 min, and then the supernatant was discarded. The cells were resuspended in a fluorescent secondary antibody R-PE-conjugated AffiniPure Goat Anti-Human IgG, Fcγ Fragment Specific (Jackson ImmunoResearch, Cat. No. 109-116-098) solution diluted in a 1:200 ratio, and then incubated in the dark at 4 °C for 30 min. The cells were washed twice with PBS and then resuspended, and finally, PE-channel fluorescence signals were detected using a flow cytometer (Beckman, CytoFlex). The results are shown in FIGs. 1 and 2.

FIG. 1 shows that all the mouse chimeric anti-FGFR2 antibodies (i.e., antibodies E3A1, D12D8, F6B6, A1B2, and A5H12) bound to KATO-III cells expressing human FGFR2, with the EC₅₀ values for the binding of the antibodies to the cells being 8.321 nM, 7.373 nM, 14.43 nM, 0.3364 nM, and 2.299 nM, respectively.

FIG. 2 shows that the mouse chimeric anti-FGFR2b antibodies (i.e., antibodies F3F8, G11B8, and H3F7-a) bound to KATO-III cells expressing human FGFR2, with the EC₅₀ values for the binding of the antibodies to the cells being 3.969 nM, 0.9494 nM, and 1.086 nM, respectively.

### Example 4. Binding of anti-FGFR2 antibodies to FGFR family

The binding performance of the anti-FGFR2 antibodies to FGFR family members was determined by ELISA.

Human FGFR1 protein (ACRO Biosystems, Cat. No. FG1-H5223), human FGFR3 protein (Sino Biological, Cat. No. 16486-H08H), and human FGFR4 protein (Sino Biological, Cat. No. 10538-H08H) were separately immobilized onto 96-well plates by incubation at 4 °C overnight. The 96-well plate was then blocked by incubation with 1% BSA in PBS at 37 °C for 1 h. After blocking, the antibodies (i.e., F3F8, D12D8, G11B8, A1B2, H3F7-a, E3A1, A5H12, F6B6, and bemarituzumab) were incubated with each FGFR family protein immobilized onto 96-well plates for 1 h at 37 °C. After incubation, the 96-well plate was washed 3 times with PBST, and the cells were co-incubated at 37 °C for 1 h with peroxidase-labeled goat anti-human Fc IgG (Jackson Immuno Research, Cat. No. 109-035-098) diluted at 1/15000 in the binding buffer, washed again, and subjected to a chromogenic reaction with TMB. The reaction was terminated using 1 M H₂SO₄.

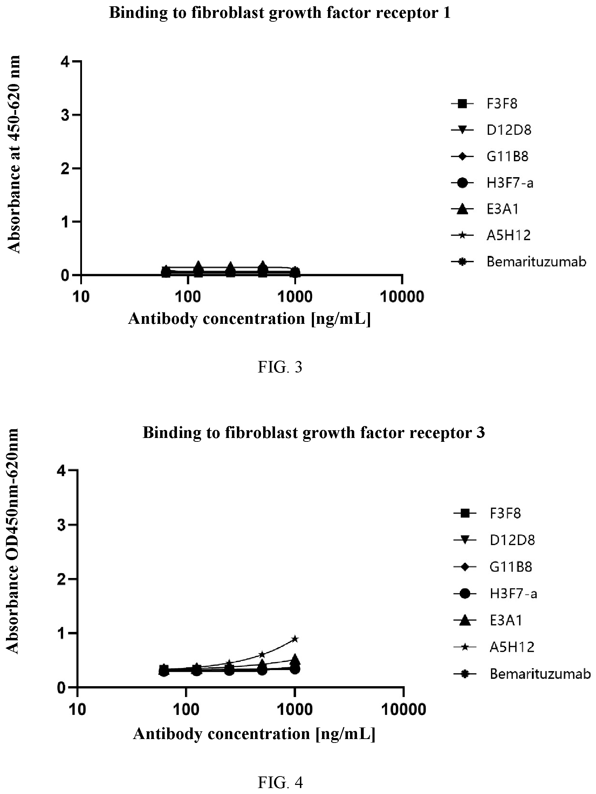

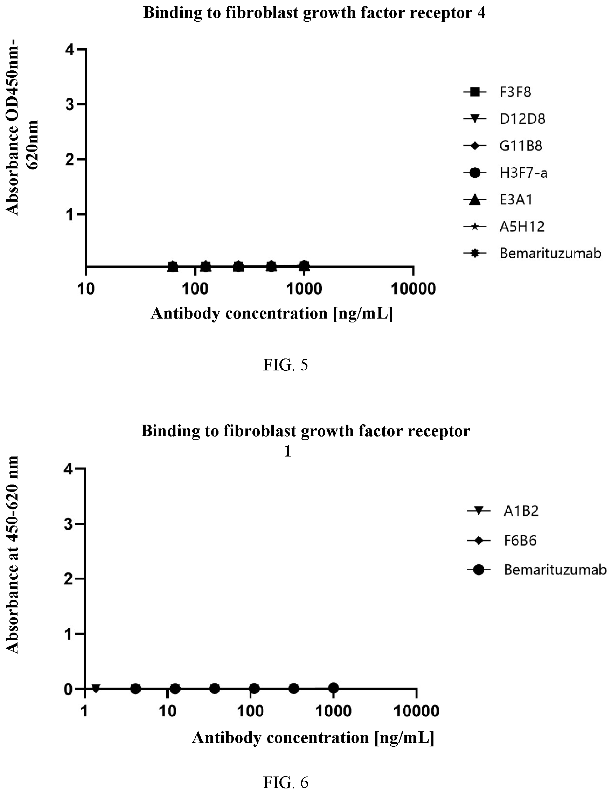

Representative binding curves of F3F8, D12D8, G11B8, A1B2, H3F7-a, E3A1, A5H12, F6B6, and bemarituzumab to the human FGFR family proteins are shown in FIGs. 3, 4, 5, 6, 7, and 8, respectively.

As can be seen from FIGs. 3, 5, 6, and 8, F3F8, D12D8, G11B8, A1B2, H3F7-a, E3A1, A5H12, F6B6 and bemarituzumab did not bind to FGFR1 and FGFR4.

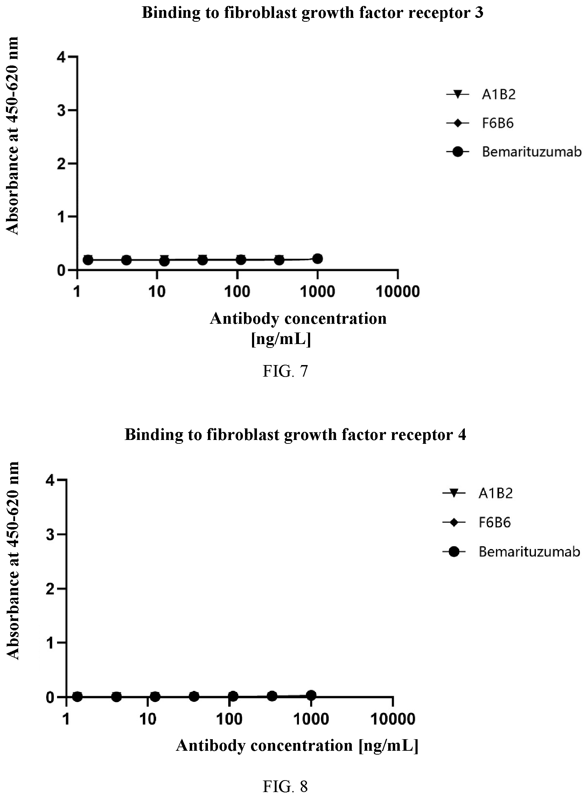

As can be seen from FIGs. 4 and 7, except for A5H12 and E3A1 which bound to some extent to FGFR3, the other antibodies did not bind to family member FGFR3.

### Example 5: Binding of anti-FGFR2 antibodies to human FGFR2c protein

The binding performance of the FGFR2 antibodies obtained in Example 2 to human FGFR2IIIc protein was determined by ELISA.

Human FGFR2IIIc (KACTUS, Cat. No. FGR-HM2CD) was immobilized onto a 96-well plate by incubation at 4 °C overnight. The 96-well plate was then blocked by incubation with 1% BSA in PBS at 37 °C for 1 h. After blocking, the antibodies (i.e., G11B8, A1B2, H3F7-a, A5H12, and bemarituzumab) were incubated with each FGFR family protein immobilized onto 96-well plates for 1 h at 37 °C. After incubation, the 96-well plate was washed 3 times with PBST, and the cells were co-incubated at 37 °C for 1 h with a peroxidase-labeled goat anti-human IgG Fab secondary antibody (Jackson Immuno Research, Cat. No. 109-035-097) diluted at 1/15000 in the binding buffer, washed again, and subjected to a chromogenic reaction with TMB. The reaction was terminated using 1 M H₂SO₄.

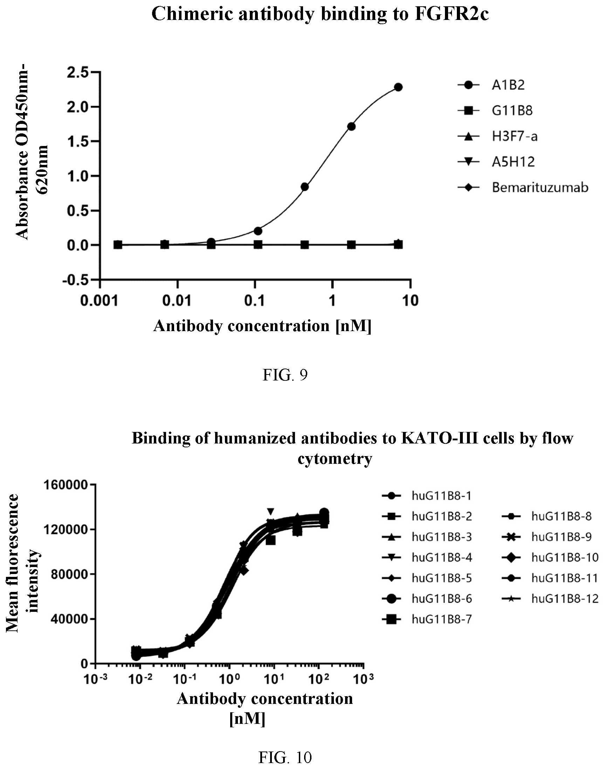

Representative binding curves of G11B8, A1B2, H3F7-a, A5H12, and bemarituzumab to FGFR2c protein are shown in FIG. 9.

As can be seen from FIG. 9, A1B2 bound to FGFR2-IIIc, while the other antibodies did not bind to FGFR2c.

### Example 6: Humanization of mouse anti-FGFR2 antibodies and characterization thereof

### 6.1 Humanization of mouse anti-FGFR2 antibody

The obtained antibody G11B8 was humanized. Specifically, the sequence of antibody G11B8 was searched and aligned in the IMGT database, thereby acquiring a human germline gene sequence IGHV1-69-2*01 with high homology to the heavy chain variable region of antibody G11B8 for use as a humanized framework of the heavy chain variable region, and acquiring a human germline gene sequence IGKV1-33*01 with high homology to the light chain variable region for use as a humanized framework of the light chain variable region. The CDRs (the boundaries of the three CDRs were defined by the IMGT scheme) in the heavy chain variable region and the light chain variable region of antibody G11B8 were grafted into the corresponding humanized frameworks to form humanized antibody G11B8 variable regions. In order to maintain the affinity of the humanized antibodies for G11B8, the humanized antibody variable regions obtained were subjected to back mutation.

The sequences of the humanized heavy chain variable regions and the humanized light chain variable regions were obtained. The variable region amino acid sequences were sent to General Biology System (Anhui) Co., Ltd. for codon optimization and gene synthesis. The genes encoding the humanized VH regions were inserted into an expression vector pcDNA3.1(+) comprising a gene encoding a human IgG1 heavy chain constant region (SEQ ID NO: 91) to give plasmids encoding the full-length heavy chains of the anti-FGFR2 humanized antibodies. The genes encoding the humanized VL regions were inserted into an expression vector pcDNA3.1(+) comprising a gene encoding a human κ light chain constant region (SEQ ID NO: 92) to give plasmids encoding the full-length light chains of the anti-FGFR2 humanized antibodies. Similarly, A1B2 was humanized and a corresponding expression plasmid was constructed. The names and the heavy chain variable region sequences of the heavy chains, as well as the names and the light chain variable region sequences of the light chains, of the mouse antibodies G11B8 and A1B2 after humanization are listed in Table 4.

**Table 3. Names and variable region sequences of heavy chains and light chains of humanized antibodies**

| Heavy chain name | Heavy chain |
|---|---|
| huG11B8H-1 | SEQ ID NO:68 |
| huG11B8H-2 | SEQ ID NO:70 |
| huG11B8H-3 | SEQ ID NO:72 |
| huG11B8H-4 | SEQ ID NO:74 |
| huG11B8H-5 | SEQ ID NO:76 |
| huG11B8H-6 | SEQ ID NO:78 |
| HuA1B2H-1 | SEQ ID NO:106 |

| Light chain name | Light chain |
|---|---|
| huG11B8L-1 | SEQ ID NO:85 |
| huG11B8L-2 | SEQ ID NO:87 |
| HuA1B2L-3 | SEQ ID NO:107 |

### 6.2 Expression and purification of humanized anti-FGFR2 antibodies

Similarly as described in Example 2, the plasmids encoding the full-length heavy chains of the anti-FGFR2 humanized antibodies and the plasmids encoding the full-length light chains of the anti-FGFR2 humanized antibodies were combined (Table 4) and co-transfected into ExpiCHO-S cells to express the respective anti-FGFR2 humanized antibodies, and the corresponding purification was performed as described in Example 2. The purified anti-FGFR2 humanized antibodies were concentrated and subjected to sterile filtration. The purity of the anti-FGFR2 humanized antibodies was detected by SDS-PAGE and size exclusion chromatography (SEC).

**Table 4: Humanized antibody expression combinations (SEQ ID NO)**

| Name of humanized antibody | Heavy chain | HC | VH | HCDR 1 | HCDR 2 | HCDR 3 | Light chain | LC | VL | LCD R1 | LCD R2 | LCD R3 |
|---|---|---|---|---|---|---|---|---|---|---|---|---|
| huG11B8-1 | huG11B8H-1 | 68 | 69 | 64 | 65 | 67 | huG11B8L-1 | 85 | 86 | 82 | 83 | 84 |
| huG11B8-2 | huG11B8H-1 | 68 | 69 | 64 | 65 | 67 | huG11B8L-2 | 87 | 88 | 82 | 83 | 84 |
| huG11B8-3 | huG11B8H-2 | 70 | 71 | 64 | 65 | 67 | huG11B8L-1 | 85 | 86 | 82 | 83 | 84 |
| huG11B8-4 | huG11B8H-2 | 70 | 71 | 64 | 65 | 67 | huG11B8L-2 | 87 | 88 | 82 | 83 | 84 |
| huG11B8-5 | huG11B8H-3 | 72 | 73 | 64 | 65 | 67 | huG11B8L-1 | 85 | 86 | 82 | 83 | 84 |
| huG11B8-6 | huG11B8H-3 | 72 | 73 | 64 | 65 | 67 | huG11B8L-2 | 87 | 88 | 82 | 83 | 84 |
| huG11B8-7 | huG11B8H-4 | 74 | 75 | 64 | 66 | 67 | huG11B8L-1 | 85 | 86 | 82 | 83 | 84 |
| huG11B8-8 | huG11B8H-4 | 74 | 75 | 64 | 66 | 67 | huG11B8L-2 | 87 | 88 | 82 | 83 | 84 |
| huG11B8-9 | huG11B8H-5 | 76 | 77 | 64 | 65 | 67 | huG11B8L-1 | 85 | 86 | 82 | 83 | 84 |
| huG11B8-10 | huG11B8H-5 | 76 | 77 | 64 | 65 | 67 | huG11B8L-2 | 87 | 88 | 82 | 83 | 84 |
| huG11B8-11 | huG11B8H-6 | 78 | 79 | 64 | 66 | 67 | huG11B8L-1 | 85 | 86 | 82 | 83 | 84 |
| huG11B8-12 | huG11B8H-6 | 78 | 79 | 64 | 66 | 67 | huG11B8L-2 | 87 | 88 | 82 | 83 | 84 |
| HuA1B2-3 | HuA1B2H-1 | 106 | 104 | 45 | 46 | 47 | HuA1B2L-3 | 107 | 105 | 50 | 51 | 52 |

### 6.3 Physicochemical analysis of humanized anti-FGFR2 antibodies

For the humanized anti-FGFR2 antibodies obtained, the purity of the antibodies in Table 4 was determined by size exclusion chromatography. Specifically, 20 µg of each humanized anti-FGFR2 antibody sample in Table 4 was injected onto a TSK G3000SWXL column using 100 mM sodium phosphate + 100 mM Na₂SO₄ (pH 7.0) as a running buffer for 30 min. The collected effluent was measured using the Agilent 1220 HPLC system, and the data were analyzed using OpenLAB software. The results indicate that the purities of the humanized anti-FGFR2 antibodies in Table 5 were greater than 90%, and the antibodies could be used in the next step.

### Example 7. Binding of humanized anti-FGFR2 antibodies to tumor cell lines

Whether the anti-FGFR2 humanized antibodies in the present disclosure could bind to tumor cells expressing human FGFR2b protein was determined by flow cytometric cell binding assay.

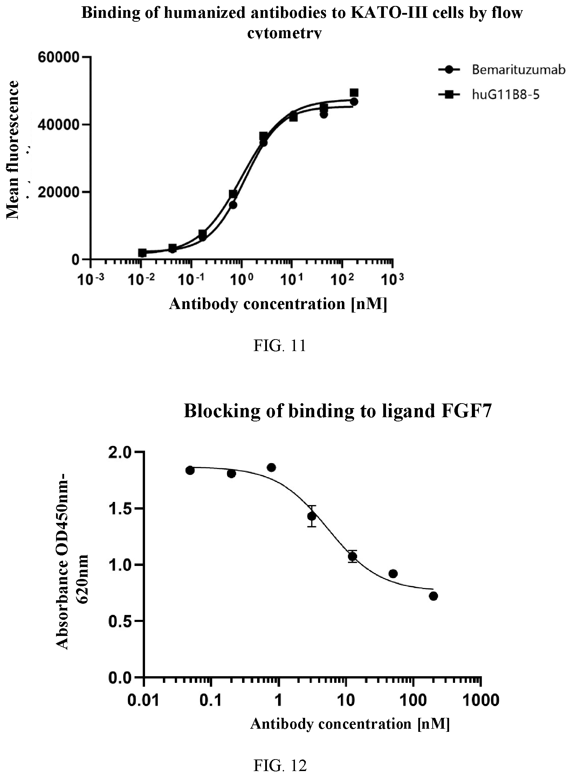

Semi-adherent KATO-III (Cell Bank of Type Culture Collection Committee of the Chinese Academy of Sciences, Cat. No. TCHu229) cells were subjected to enzymatic digestion to give a single-cell suspension. The single-cell suspension was centrifuged at 300× g at room temperature for 4 min, and then the medium was discarded. The cells were resuspended in PBS, seeded into a 96-well V-bottom plate (Nessie, Cat. No. 701211) at 1 × 10⁵ cells/well, and centrifuged at 300× g for 4 min at room temperature, and then the supernatant was discarded. The cells were resuspended in the serially diluted anti-FGFR2 humanized antibody solutions (serially 4-fold diluted from 20 µg/mL or 25 µg/mL to the 8th concentration) and incubated at 4 °C for 30 min. The cells were resuspended and incubated with the chimeric antibodies or bemarituzumab as the positive antibody using the same dilution method. The cells were washed once with PBS and centrifuged at 300× g for 4 min, and then the supernatant was discarded. The cells were resuspended in a fluorescent secondary antibody R-PE-conjugated Affini Pure Goat Anti-Human IgG, Fcγ Fragment Specific (Jackson Immuno Research, Cat. No. 109-116-098) solution diluted in a 1:200 ratio, and then incubated in the dark at 4 °C for 30 min. The cells were washed twice with PBS and then resuspended, and finally, PE-channel fluorescence signals were detected using a flow cytometer (Beckman, CytoFlex). The results are shown in FIGs. 10 and 11. The results show that all the anti-FGFR2 humanized antibodies could bind to KATO-III cells, with binding capacities being comparable to those of the chimeric antibodies and the positive reference antibody.

### Example 8. Assay on affinity of humanized monoclonal antibodies for human FGFR2 and cynomolgus FGFR2

In this study, the binding affinity of humanized monoclonal antibody huG11B8-5 for the human FGFR2-his protein (SEQ ID NO: 94) and the cynomolgus FGFR2-his protein (SEQ ID NO: 95) was detected using ForteBio Octet RED96e according to the manufacturer's instructions.

Briefly, an AHC sensor (ForteBio, Cat. No. 18-5060) was pre-equilibrated in a running buffer (1× PBS, XiGene, Cat. No. XG3650, containing 0.02% Tween 20, 0.1% BSA, pH 7.0) at room temperature for 10 min. In a 96-well plate, the kinetic assay was performed according to the following steps:
a) the system was equilibrated with the running buffer for 180 s to baseline;
b) humanized antibodies diluted with the running buffer at a final concentration of 5 µg/mL were added for 200 s of immobilization;
c) the system was equilibrated with the running buffer for 300 s to baseline;
d) the human FGFR2-his protein or the cynomolgus FGFR2-his protein diluted with the running buffer was added to each well at the following concentrations: 100 nM, 50 nM, 25 nM, 12.5 nM, 6.25 nM, 3.13 nM, and 1.56 nM, and subjected to 200 s of association and 600 s of dissociation.
e) The mixture was regenerated with a regeneration buffer (0.01 M Gly-HCl, pH 1.5) for 30 s. The experimental data were fitted and calculated using a 1:1 binding model in the Fortebio Data Analysis software.

Table 5 summarizes the binding affinities of humanized monoclonal antibody huG11B8-5 for human FGFR2 extracellular protein and cynomolgus FGFR2 extracellular protein.

**Table 5. Binding affinity of humanized monoclonal antibody huG11B8-5 for human FGFR2 extracellular protein and cynomolgus FGFR2 extracellular protein**

| Antigen | Antibody | KD (M) | Kon (1/Ms) | Kdis (1/s) |
|---|---|---|---|---|
| Human FGFR2 | huG11B8-5 | 2.62E-09 | 1.64E+05 | 4.31E-04 |
| Cynomolgus FGFR2 | | 1.62E-9 | 5.36E+05 | 8.66E-04 |

Table 5 shows that the humanized antibody of the present disclosure can specifically bind to human FGFR2 extracellular protein or cynomolgus FGFR2 extracellular protein with high affinity.

### Example 9. Blocking against ligands by humanized monoclonal antibody

The ability of the obtained humanized monoclonal antibody huG11B8-5 to block the binding of FGFR2 to its ligands was determined by ELISA.

Human FGF7 protein (Sino Biological, Cat. No. 10210-H07E), human FGF10 protein (Sino Biological, Cat. No. 10573-HNAE), and human FGF22 protein (R&D Systems, Cat. No. 3867-FG-035) were separately immobilized onto 96-well plates by incubation at 4 °C overnight. The 96-well plate was then blocked by incubation with 1% BSA in PBS at 37 °C for 1 h. After blocking, equal volumes of huG11B8-5 and FGFR2-his were mixed and incubated at 37 °C for 1 h to immobilize them on the 96-well plate. After incubation, the 96-well plate was washed 3 times with PBST, and the cells were co-incubated at 37 °C for 1 h with peroxidase-labeled mouse anti-His-Tag IgG (CwBio, Cat No. CW0285M) diluted at 1/10000 in the binding buffer, washed again, and subjected to a chromogenic reaction with TMB. The reaction was terminated using 1 M H₂SO₄.

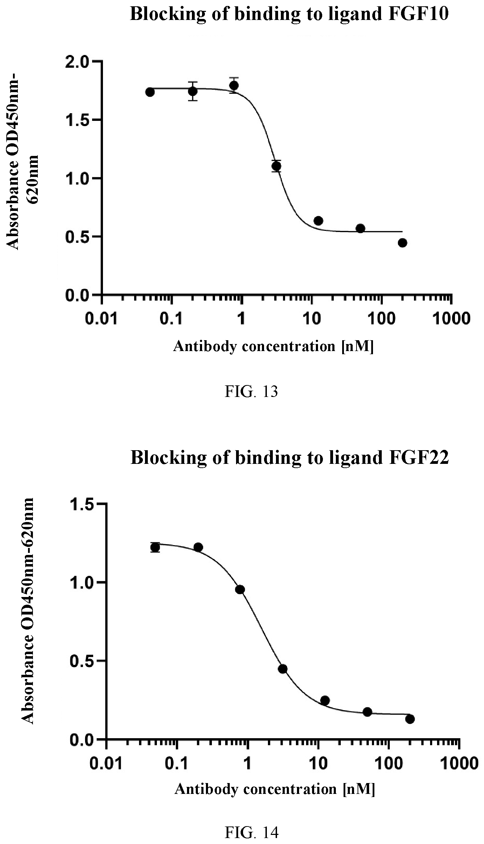

The curves for huG11B8-5 blocking the binding of FGFR2 protein to its ligands are shown in FIGs. 12, 13, and 14.

As can be seen from FIGs. 12, 13, and 14, huG11B8-5 can block the binding of FGFR2 to ligands FGF7, FGF10, and FGF22.

### Example 10. Classification and identification of epitopes for binding of humanized monoclonal antibodies to human FGFR2 protein

In this study, whether humanized monoclonal antibody huG11B8-5, humanized monoclonal antibody huA1B2-3, and bemarituzumab bind to the same epitope as human FGFR2-his protein (Y210630, SEQ ID NO: 94) was determined using ForteBio Octet RED96e according to the manufacturer's instructions.

Briefly, a HISK sensor (ForteBio, Cat. No. 18-5120) was pre-equilibrated in a running buffer (1× PBS, XiGene, Cat. No. XG3650, containing 0.02% Tween 20, 0.1% BSA, pH 7.0) at room temperature for 10 min. In a 96-well plate, the kinetic assay was performed according to the following steps:
a) the system was equilibrated with the running buffer for 180 s to baseline;
b) human FGFR2-his protein diluted with the running buffer at a final concentration of 5 µg/mL was added for immobilization to 0.3 nM;
c) the system was equilibrated with the running buffer for 30 s to baseline;
d) the humanized monoclonal antibodies huG11B8-5 and huA1B2-3 and bemarituzumab diluted to 200 nM with the running buffer were added to the wells as the first binding antibody, and subjected to 180 s of association and 30 s of baseline equilibration.
e) The humanized monoclonal antibodies huG11B8-5 and huA1B2-3 and bemarituzumab were diluted to 200 nM with the running buffer and used as the second binding antibody, and meanwhile, buffer wells were set as reference wells for subtraction.
e) The mixture was regenerated with a regeneration buffer (0.01 M Gly-HCl, pH 1.5) for 30 s. The experimental data were analyzed using process epitope;

The overlapping of the binding epitopes of the humanized monoclonal antibodies huG11B8-5 and huA1B2-3 and bemarituzumab with the human FGFR2-his protein was analyzed (Table 6).

**Table 6. Classification of binding epitopes of humanized monoclonal antibodies huG11B8-5 and huA1B2-3 and bemarituzumab for human FGFR2-his protein**

| | huG11B8-5 | huA1B2-3 | Bemarituzumab |
|---|---|---|---|
| huG11B8-5 | 6.20% | 10.50% | 8.40% |
| huA1B2-3 | 42.60% | 10.20% | 95.70% |
| Bemarituzumab | 36.80% | 105.50% | 7.80% |

Table 6 shows that the epitopes to which the humanized antibodies huG11B8-5 and huA1B2-3 of the present disclosure and bemarituzumab bind in the human FGFR2-his protein could be divided into two classes: huG11B8-5 and bemarituzumab bound to one class, and huA1B2-3 bound to the other class.

### Example 11. Construction of antibody-fusion protein

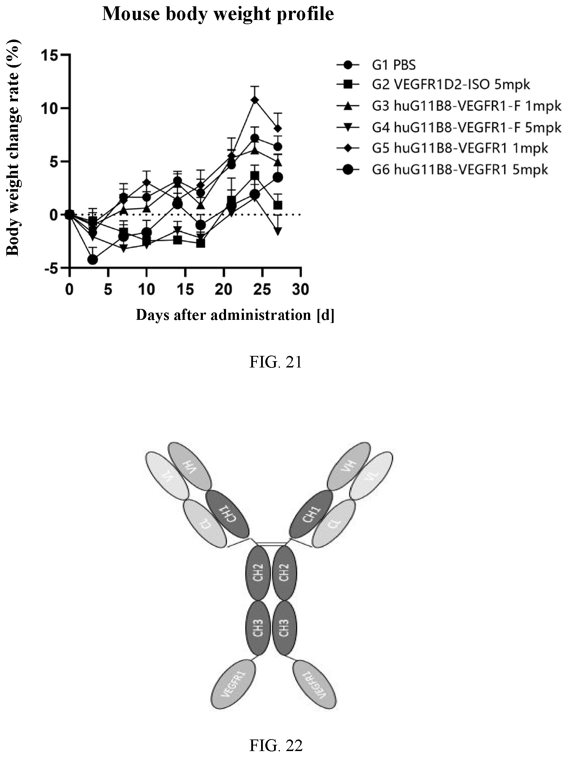

In the present application, an antibody fusion protein with the structure shown in FIG. 22 was obtained using standard construction methods:
Heavy chain of huG11B8-VEGFR1: the coding nucleotide sequence of VEGFR1 domain 2 (SEQ ID NO: 100) was synthesized and linked to the C-terminus of the huG11B8H-3 sequence (with a linker GGGGSGGGGSGGGGS (SEQ ID NO: 90) between the fragments); to prevent fragmentation and replacement, the lysine at the end of Fc was replaced by alanine to give an expression vector of the heavy chain of the anti-FGFR2-VEGFR1 fusion protein, wherein the heavy chain of the anti-FGFR2-VEGFR1 fusion protein sequentially contained, from N-terminus to C-terminus: an anti-FGFR2 heavy chain (with K at the end mutated to A) and VEGFR1.

Light chain of huG11B8-VEGFR1: huG11B8L-1 (SEQ ID NO: 85)

**Table 7. Antibody fusion protein**

| Molecule | Chain | Amino acid sequence |
|---|---|---|
| huG11B8-VEGFR1 | Heavy chain huG11B8-VEGFR1 | SEQ ID NO: 89 |
| | Light chain huG11B8L-1 | SEQ ID NO: 85 |

The heavy and light chain expression vectors obtained (see Table 6) were co-transfected into ExpiCHO-S cells for expression under appropriate conditions to obtain bispecific antibody proteins, and the expression, purification, and preliminary analysis steps were the same as those in Example 2. The obtained fusion protein was referred to as huG11B8-VEGFR1 below. Similarly, the expression of VEGFR1D2-ISO (heavy chain: SEQ ID NO: 110; light chain: SEQ ID NO: 111) is as described above.

In addition, ADCC-enhanced huG11B8-VEGFR1 and G11B8 antibodies (referred to herein as huG11B8-VEGFR1-F and G11B8-F) and bevacizumab analogs (heavy chain: SEQ ID NO: 108; light chain: SEQ ID NO: 109) were also expressed. The specific preparation was similar to that of bemarituzumab in Example 2.

### Example 12. Affinity assay of antibody-fusion proteins for FGFR2 and VEGF165

In this study, the binding affinity of antibody fusion protein huG1 1B8-VEGFR1-F for human FGFR2-his protein (SEQ ID NO: 94) and human VEGF165 protein (ACROBiosystems, Cat. No. VE5-H5248) was detected using ForteBioOctet RED96e according to the manufacturer's instructions.

Briefly, an AHC sensor (ForteBio, Cat. No. 18-5060) was pre-equilibrated in a running buffer (1× PBS, XiGene, Cat. No. XG3650, containing 0.02% Tween 20, 0.1% BSA, pH 7.0) at room temperature for 10 min. In a 96-well plate, the kinetic assay was performed according to the following steps:
a) the system was equilibrated with the running buffer for 180 s to baseline;
b) the antibody-fusion protein diluted with the running buffer at a final concentration of 5 µg/mL was added for 200 s of immobilization;
c) the system was equilibrated with the running buffer for 300 s to baseline;
d) the human FGFR2-his protein or the human VEGF165 protein diluted with the running buffer was added to each well at the following concentrations: 100 nM, 50 nM, 25 nM, 12.5 nM, 6.25 nM, 3.13 nM, and 1.56 nM, and subjected to 200 s of association and 600 s of dissociation.
e) The mixture was regenerated with a regeneration buffer (0.01 M Gly-HCl, pH 1.5) for 30 s. The experimental data were fitted and calculated using a 1:1 binding model in the Fortebio Data Analysis software.

Table 8 summarizes the binding affinity of antibody-fusion protein huG11B8-VEGFR1-F for FGFR2-his and VEGF165 proteins.

**Table 8: Binding affinity of antibody-fusion protein huG11B8-VEGFR1-F for human FGFR2 and VEGF165 proteins**

| Antigen | Fusion protein | KD (M) | Kon (1/Ms) | Kdis (1/s) |
|---|---|---|---|---|
| Human FGFR2 | huG11B8-VEGFR1-F | 4.53E-09 | 2.99E+05 | 1.36E-03 |
| Human VEGF165 | | 1.08E-10 | 5.68E+05 | 6.12E-05 |

Table 8 shows that the antibody-fusion protein of the present disclosure can specifically bind to human FGFR2 protein or human VEGF165 protein with high affinity.

### Example 13. Inhibition of binding of VEGF165 to VEGFR2 by huG11B8-VEGFR1 bispecific antibody

The eukaryotic expression vector encoding the human VEGFR2 (Uniprot: P35968) gene and the NFAT-luc2P reporter gene element (Promega, Cat. No. E8481) were transfected into 293T cells using a Lipofectamine^{™} 2000 (Invitrogen, Cat. No. 11668019) transfection reagent according to the manufacturer's instructions. 48 h after transfection, screening was performed with 0.3 µg/mL puromycin (Invitrogen, Cat. No. A1113802) and 85 µg/mL hygromycin B (Invitrogen, Cat. No. 10687010) to give a 293T polyclonal stable cell strain highly expressing human VEGFR2 and NFAT-luc2P. The polyclonal cells were subjected to limiting dilution to give monoclonal cells 293T/NFAT/VEGFR2 stably and highly expressing NFAT-luc2P and human VEGFR2.

293T/NFAT/VEGFR2 cells were digested and collected by centrifugation, and the supernatant was discarded. The cells were resuspended in an assay medium (90% DMEM + 10% fetal bovine serum) and counted on the Countstar cell counter. The cell suspension was diluted to 8 × 10⁵ cells/mL with the assay medium. The fusion proteins of interest at 3× final assay concentrations were prepared using an assay medium containing 6 ng/mL VEGF165. The final assay concentration of the antibodies of interest (huG11B8-VEGFR1 and bevacizumab analog) was 5 nM, and the antibodies were serially diluted 2-fold to the 9th concentration.

The 293T/NFAT/VEGFR2 cell suspension was seeded into a 384-well plate at 25 µL/well. The serially diluted antibody dilutions were loaded at 25 µL/well. The whole plate was centrifuged at a low speed of 600 rpm for 30 s on a centrifuge to ensure that no liquid was caught on the well wall. The plate was incubated in an incubator at 37 °C, 5% CO₂, and saturated humidity for 6 h. Bio-liteBuffer (Vazyme; Cat. No. DD1201-03) was thawed at room temperature in advance. The plate was taken out from the incubator and equilibrated at room temperature for 10-15 min. 25 µL of Bio-lite Buffer was added to each well. The mixture was well mixed for 3-5 min of lysis at room temperature, transferred to a 384-well white plate, and detected on a multi-mode microplate reader using the fluorescence detection module to measure the luminescence signal readings. The data were input into GraphpadPrism software with the x-axis data being log values of the molar concentration, and the "Dose-response-Stimulation→Log (inhibitor) vs. response-Variableslope (four parameters)" method was selected for fitting. The IC₅₀ values were recorded.

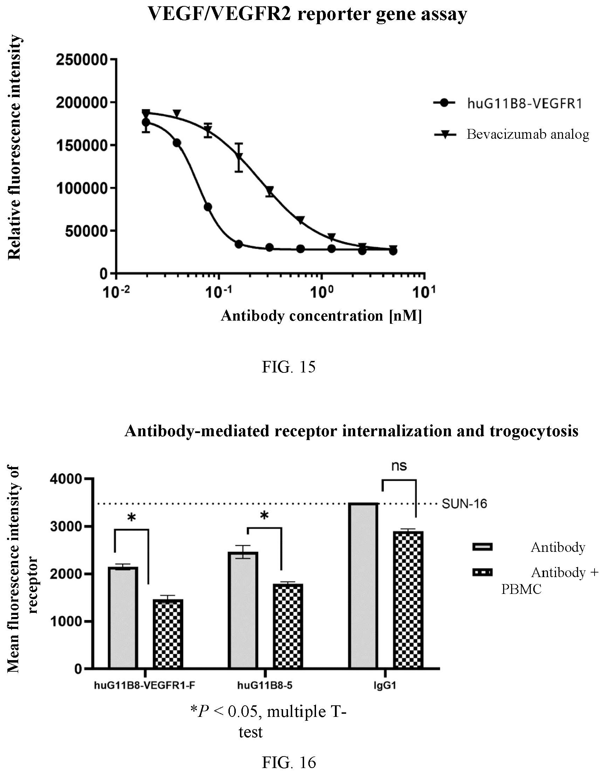

The experimental results are shown in FIG. 15. The huG11B8-VEGFR1 fusion protein could inhibit the binding of VEGF165 to VEGFR2 in a dose-dependent manner, with the IC₅₀ value being 0.06252 nM, and the inhibitory activity was significantly stronger than that of the reference antibody.

### Example 14. Antibody-mediated internalization of FGFR2 on tumor cell surface and ADCT effect

To detect the receptor internalization and trogocytosis effects mediated by the FGFR2 antibodies and the fusion proteins thereof, a primary PBMC-dependent trogocytosis assay system was established. The assay was performed using SNU-16 (Cobioer, CBP60502) endogenously expressing human FGFR2 as the target cells and human peripheral blood mononuclear cells (PBMCs) as the effector cells, as specifically shown below.

The PBMC cells were thawed, and adjusted to a cell density of 1 × 10 ⁶/mL using an RPMI-1640 (Gibco, 22400) + 10% FBS (Giboco, 10099) complete medium. The cells were seeded into a 96-well plate (Corning, 7007) at 100 µL per well (i.e., 100,000 cells/well). The cultured target cells SNU-16 were collected and centrifuged at 200 *g* for 5 min, and the supernatant was discarded. The cells were resuspended in PBS and centrifuged at 200 *g* for 5 min, and the supernatant was discarded. The cells were resuspended in PBS and counted. CFSE (Merck, SCT110) was added at a ratio of 1:5000. The mixture was left to stand at 37 °C for 15 min, and FBS was added. The mixture was left to stand at 37 °C for 5 min to stop staining. The mixture was centrifuged at 200 *g* for 5 min, and the supernatant was discarded. The cells were washed once with 3 mL of complete medium, resuspended in the complete medium, and counted. The target cell density was adjusted to 2 × 10⁵/mL, and the cells were seeded into the above 96-well plate at 50 µL per well (i.e., 10,000 cells/well) (effector-to-target ratio = 10:1). Antibodies of interest at 4× final assay concentration were prepared. The maximum assay concentration of antibodies IgG1 (Sino Biological, Cat. No. HG1K), bemarituzumab, and huG11B8-5 and the fusion protein (huG11B8-VEGFR1-F) was 100 nM, and the antibodies were serially 10-fold diluted to give 2 concentration points. 50 µL of antibody was added to each well. Blank (target cells plus complete medium) and antibody (target cells plus antibody) controls were set in the study. The final total volume of each well was 200 µL, and the plate was incubated in a cell incubator for another 72 h. After the incubation was completed, the plate was taken out, and all cell suspensions were transferred to a 96-well V-bottom plate (NEST, 701211). The plate was centrifuged at 400 *g* for 3 min, and the supernatant was discarded. The plate was washed once with PBS. A suspension of the FGFR2 polyclonal antibody (Thermo, PA5-14651) in PBS (1:100) was added at 100 µL/well to resuspend the cells, and the mixture was incubated at 4 °C for 30 min. The plate was washed twice with PBS, and a donkey anti-rabbit secondary antibody (Jackson, 711-116-152, 1:200) and a viability dye (Biolegend, 423114, 1:1000) were added at 100 µL/well. The plate was incubated at 4 °C for 30 min. The cells were washed twice with PBS, and the cell pellet was resuspended in 100 µL of PBS and determined using a flow cytometer.

The results, shown in FIG. 16, indicate that the FGFR2 antibody can induce down-regulation of FGFR2 receptor on the surface of SNU-16 cells. Moreover, the FGFR2 antibody and the fusion protein thereof can further down-regulate the FGFR2 receptor on the surface of SNU-16 cells through trogocytosis by PBMCs.

### Example 15. Antibody-mediated ADCC effect

To detect the ADCC effect of the FGFR2 antibody and the bispecific fusion protein thereof, an NK92MI-human CD16aV engineered cell line-dependent ADCC system was established, and experiments were performed using Ba/F3 engineered to express human FGFR2b (KYinno Biotechnology Co., Ltd., Cat. No. KC1654) as the target cells, as specifically shown below:
NK92MI (Nanjing Cobioer Biosciences Co., Ltd., Cat. No. CBP61113) was cultured in N500 NK cell serum-free medium (Dakewe, Cat. No. 6113031), followed by engineering. Specifically, a nucleotide sequence encoding a human CD16AV-FcR1γ sequence (SEQ ID NO: 103) was cloned into a stable transfection expression vector (PiggyBac Dual promoter (SBI, Cat. No. PB513-B1), and NK92MI cells were transfected with the sequence encoding the human CD16AV-FcR1γ using a Bio-RAD electroporator (BIO-RAD, Cat. No. Gene Pul Ser X Cell TM), followed by screening with 1 µg/mL puromycin to give NK92MI cells highly expressing human CD16a (also referred to herein as "NK92MI-huCD16aV" cells).

The normally cultured NK92MI-huCD16aV cells were collected and centrifuged at 200× for 5 min, and the supernatant was discarded. The cells were resuspended in a fresh medium and adjusted to a cell density of 3 × 10⁵/mL, and the cells were seeded into a 96-well plate (Corning, Cat. No. 7007) at 100 µL/well (i.e., 30,000 cells/well). Antibodies of interest at 4× final assay concentration were prepared. The maximum assay concentration of antibodies IgG1 (reference; Sino Biological, Cat. No. HG1K), bemarituzumab, huG11B8-5, huG11B8-VEGFR1-F, and huG11B8-VEGFR1 was 400 nM, and the antibodies were serially 5-fold diluted to give 8 concentration points. 50 µL of antibody was added to each well, mixed with the effector cells, and incubated for 30 min at 37 °C. Ba/F3 expressing human FGFR2b was cultured in a 1640 complete medium containing 5 µg/mL puromycin, 8 ng/mL murine IL-3 (Suzhou Novoprotein Scientific Co., Ltd., Cat. No. CP39), and 10% fetal bovine serum.

The target cells were collected and centrifuged at 200× g, and the supernatant was discarded. The cells were resuspended in PBS and counted. CFSE (Merck, Cat. No. SCT110) was added at a ratio of 1:5000. The cells were left to stand at 37 °C for 15 min, and fetal bovine serum was added. The cells were left to stand at 37 °C for 5 min before the staining was stopped. The cells were centrifuged at 200× *g* for 5 min, and the supernatant was discarded. The cells were resuspended in a 1640 complete medium containing 32 ng/mL IL-3 and 10% fetal bovine serum, and the target cell density was adjusted to 2 × 10⁵/mL. The cells were seeded into the above 96-well plate at 50 µL/well (i.e., 10,000 cells/well) (effector-to-target ratio = 3:1). Blank (target cells plus complete medium) and antibody (target cells plus antibody) controls were set in the study. The final total volume of each well was 200 µL, and the plate was incubated in a cell incubator for another 4 h. After the incubation was completed, the plate was centrifuged at 400× *g* for 3 min, and 20 µL of supernatant was discarded from each well. A propidium iodide (Beyotime, Cat. No. ST511) diluent (1:25 dilution) was prepared with PBS and added at 20 µL/well, and the mixture was incubated at room temperature for 10 min. The apoptosis rate of the target cells was measured by flow cytometry.

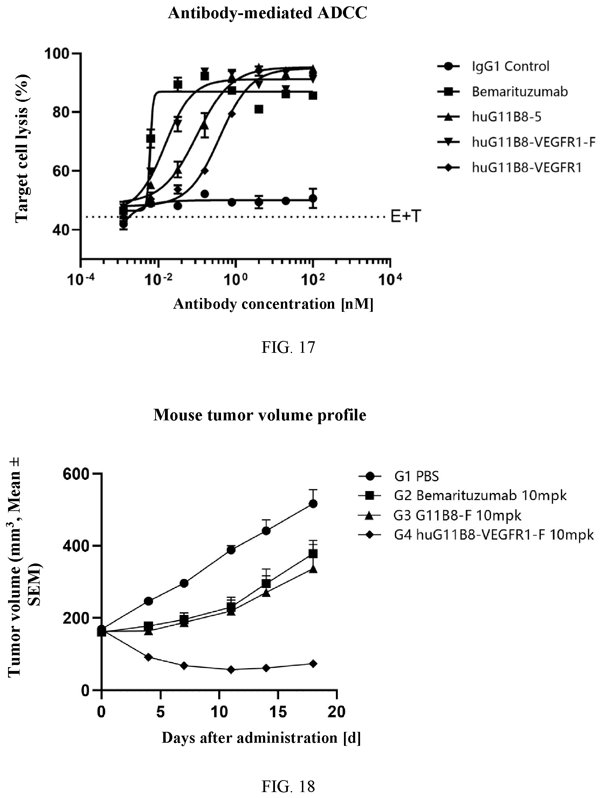

The results, as shown in FIG. 17, indicate that both the FGFR2 antibody and the bispecific fusion protein thereof can induce the killing of FGFR2b-expressing Ba/F3 cells by NK92MI-CD16aV as compared with the IgG1 reference.

### Example 16. In Vivo efficacy of antibody-fusion protein in tumor model

The *in vivo* antitumor effect of huG11B8-VEGFR1-F was investigated in a CB17-SCID mouse SNU16 tumor model.

CB17-SCID mice were purchased from Beijing Vital River Laboratory Animal Technology Co., Ltd. Mouse gastric cancer SNU16 cells were purchased from Nanjing Cobioer Biosciences Co., Ltd. The cells were cultured in an incubator containing 5% CO₂ at 37 °C, and the culture medium was an RPMI-1640 medium containing 10% inactivated fetal bovine serum.

CB17-SCID mice were grafted subcutaneously on the right side of the back with 10 × 10⁶ SNU16 cells/mouse. When the average tumor volume reached about 160 mm³, appropriate mice were selected for study and randomly divided into 4 experimental groups (6 mice per group) based on the mouse tumor volume and body weight. On Days 0, 4, 7, 11, and 14 after grouping, the blank control (PBS) (G1), bemarituzumab (10 mg/kg) (G2), G11B8-F (10 mg/kg) (G3), and fusion protein huG11B8-VEGFR1-F (10 mg/kg) (G4) were administered intraperitoneally to the mice. During the experiment, the maximum length of the major axis (L) and the maximum length of the minor axis (W) of tumors in tumor-bearing mice were measured twice weekly using a vernier caliper, and the tumor volume was calculated using the following formula: V = L×W2/2.

Treatment with G11B8-F and huG11B8-VEGFR1-F resulted in significant tumor growth inhibition as compared to the PBS group, and huG11B8-VEGFR1-F exhibited a stronger tumor inhibitory effect, with a tumor growth inhibition rate (TGI%) of 127.14% at the dose of 10 mg/kg. The results are shown in FIG. 18 and Table 9 below. FIG. 18 shows the change in tumor volume of mice after the administration of antibody fusion protein huG11B8-VEGFR1 to tumor-bearing mice.

**Table 9. Growth inhibition of SNU16 tumors by test substances**

| Group | Dose | Number of animals (N) | Tumor volume (mm³)^{a} (on day 18 after the first dose) | TGI(%)^{b} | P^{c} |
|---|---|---|---|---|---|
| Blank control (PBS) | / | 6 | 516.40±39.57 | - | / |
| Bemarituzumab | 10 mg/kg | 6 | 378.34±36.11 | 37.29 | 0.0832 |
| G11B8-F | 10 mg/kg | 6 | 336.96±66.35 | 49.94 | *0.0362 |
| huG11B8-VEGFR1-F | 10 mg/kg | 6 | 73.70±6.94 | 127.14 | ****<0.0001 |

| | | | | | |
|---|---|---|---|---|---|
| Note: a. Mean ± standard error; b. TGI = [1 - (change in tumor volume of administration group/change in tumor volume of control group)] × 100%; c. Tumor volumes in the administration group and the PBS control group were statistically analyzed (One-way ANOVA) on day 18 after grouping and administration, *: P < 0.05, ****: P < 0.0001. | | | | | |

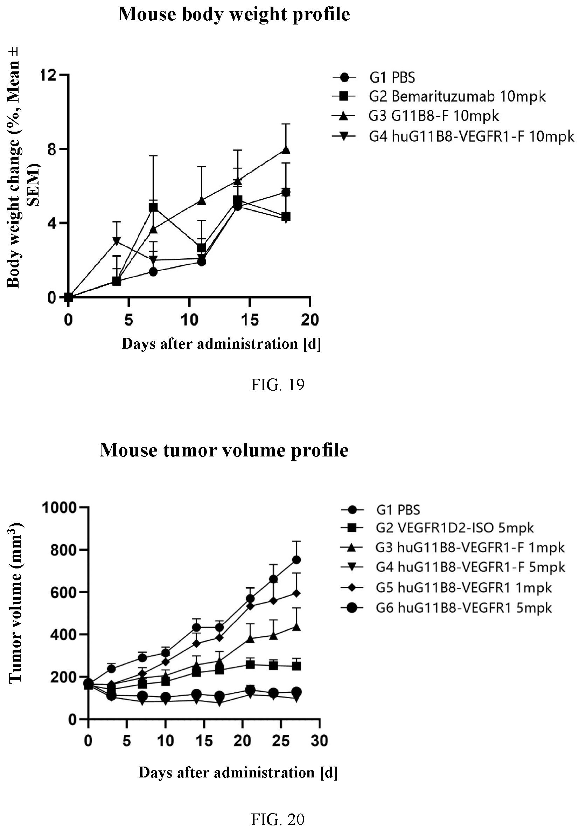

Throughout the course of the experiment, the experimental animals maintained good mobility and feeding consumption during the treatment period, and the body weight of the animals in groups G1-G4 increased to some extent, indicating that the test substances were well tolerated by the animals. The body weight changes of all animals are shown in FIG. 19 and Table 10. FIG. 19 shows the body weight changes in tumor-bearing mice after antibody-fusion protein huG11B8-VEGFR1-F administration.

**Table 10. Effect of test substances on mouse body weight**

| Group | Dose | Body weight (g)^{a} | | | Body weight change (g) on day 18 after administration |
|---|---|---|---|---|---|
| | | Before administration | Day 18 after grouping and administration | *P*^{b} | |
| Blank control (PBS) | / | 18.0±0.3 | 19.1±0.3 | - | +5.7 |
| Bemarituzumab | 10 mg/kg | 18.0±0.4 | 18.8±0.4 | 0.9926 | +4.4 |
| G11B8-F | 10 mg/kg | 17.5±0.3 | 18.9±0.4 | 0.6635 | +8.0 |
| huG11B8-VEGFR1-F | 10 mg/kg | 17.9±0.4 | 18.6±0.4 | 0.9997 | +4.2 |

| | | | | | |
|---|---|---|---|---|---|
| Note: a. Mean ± standard error; b. Body weights in the administration groups and the PBS control group were statistically analyzed (One-way ANOVA) on day 18 after grouping and administration. | | | | | |

In addition, CB17-SCID mice were grafted subcutaneously on the right side of the back with 10 × 10⁶ SNU16 cells/mouse. When the average tumor volume reached about 160 mm³, appropriate mice were selected for study and randomly divided into 6 experimental groups (6 mice per group) based on the mouse tumor volume and body weight. On Days 0, 3, 7, 10, 14, 17, 21, and 24 after grouping, the blank control (PBS) (G1), VEGFR1D2-ISO (5 mg/kg) (G2), huG11B8-VEGFR1-F (1 mg/kg) (G3), huG11B8-VEGFR1-F (5 mg/kg) (G4), huG11B8-VEGFR1 (1 mg/kg) (G5), and huG11B8-VEGFR1 (5 mg/kg) (G6) were administered intraperitoneally to the mice. During the experiment, the maximum length of the major axis (L) and the maximum length of the minor axis (W) of tumors in tumor-bearing mice were measured twice weekly using a vernier caliper, and the tumor volume was calculated using the following formula: V = L×W2/2.

On day 27 of tumor growth, VEGFR1D2-ISO at a dose of 5 mg/kg significantly inhibited tumor growth as compared with the PBS group, with a tumor growth inhibition rate (TGI%) of 84.59%. At 1 mg/kg, huG11B8-VEGFR1-F exhibited a stronger tumor inhibitory effect than that of huG11B8-VEGFR1, with a TGI of 53.97% vs. 26.36%. At 5 mg/kg, the anti-tumor effects of huG11B8-VEGFR1-F and huG11B8-VEGFR1 were comparable. The results are shown in FIG. 20 and Table 11 below.

**Table 11. Growth inhibition of SNU16 tumors by test substances**

| Group | Dose | Number of animals (N) | Tumor volume (mm³)^{a} (on day 27 after the first dose) | TGI(%)^{b} | P^{c} |
|---|---|---|---|---|---|
| Blank control (PBS) | / | 6 | 753.32±87.13 | - | / |
| VEGFR1D2-ISO | 5 mg/kg | 6 | 250.79±36.03 | 84.59 | ****<0.0001 |
| huG11B8-VEGFR1-F | 1 mg/kg | 6 | 436.77±90.20 | 53.97 | ** 0.0092 |
| huG11B8-VEGFR1-F | 5 mg/kg | 6 | 98.71±18.44 | 110.62 | ****<0.0001 |
| huG11B8-VEGFR1 | 1 mg/kg | 6 | 595.50±94.85 | 26.36 | 0.3347 |
| huG11B8-VEGFR1 | 5 mg/kg | 6 | 129.81±14.51 | 106.73 | ****<0.0001 |

| | | | | | |
|---|---|---|---|---|---|
| Note: a. Mean ± standard error; b. TGI = [1 - (change in tumor volume of administration group/change in tumor volume of control group)] × 100%; c. Tumor volumes in the administration group and the PBS control group were statistically analyzed (One-way ANOVA) on day 27 after grouping and administration, *: P < 0.05, **: P < 0.01, ****: P < 0.0001. | | | | | |

Throughout the course of the experiment, the experimental animals maintained good mobility and feeding consumption during the treatment period. The body weight changes of all animals are shown in FIG. 21 and Table 12.

**Table 12. Effect of test substances on mouse body weight**

| Group | Dose | Body weight (g)^{a} | | | Body weight change (g) on day 27 after administration |
|---|---|---|---|---|---|
| | | Before administration | Day 27 after grouping and administration | P^{b} | |
| Blank control (PBS) | / | 19.3±0.4 | 20.5±0.4 | - | +6.4 |
| VEGFR1D2-ISO | 5 mg/kg | 19.7±0.2 | 19.8±0.2 | *0.0381 | +0.9 |
| huG11B8-VEGFR1-F | 1 mg/kg | 19.1±0.3 | 20.0±0.4 | 0.9215 | +5.0 |
| huG11B8-VEGFR1-F | 5 mg/kg | 19.8±0.5 | 19.5±0.5 | **0.0016 | -1.6 |
| huG11B8-VEGFR1 | 1 mg/kg | 19.6±0.4 | 21.2±0.4 | 0.8539 | +8.1 |
| huG11B8-VEGFR1 | 5 mg/kg | 19.5±0.3 | 20.2±0.2 | 0.4700 | +3.5 |

| | | | | | |
|---|---|---|---|---|---|
| Note: a. Mean ± standard error; b. Body weights in the administration group and the PBS control group were statistically analyzed (One-way ANOVA) on day 27 after grouping and administration, *: P < 0.05, **: P < 0.01. | | | | | |

### SEQUENCE LISTING:

| Sequence No. | Description | Specific sequence |
|---|---|---|
| 1 | F3F8-H | QVTLKESGPGILQSSQTLSLTCSFSGFSLSTSGMGVSWIRQPSGKGLEWLAHIYWDNDKRYNPSLKSRLTIS KDTSRNQVFLKITSVDTADTATYYCTRSDSSGSFDYWGQGTTLTVSSASTKGPSVFPLAPSSKSTSGGTAAL GCLVKDYFPEPVTVSWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREPQVYTLPPS RDELTKNQVSLTCLVKGFYPSDIAVEWESNGQPENNYKTTPPVLDSDGSFFLYSKLTVDKSRWQQGNVFS CSVMHEALHNHYTQKSLSLSPGK K V S S |
| 2 | F3F8-H-VH | QVTLKESGPGILQSSQTLSLTCSFSGFSLSTSGMGVSWIRQPSGKGLEWLAHIYWDNDKRYNPSLKSRLTI KDTSRNQVFLKITSVDTADTATYYCTRSDSSGSFDYWGQGTTLTVSS S |
| 3 | F3F8-H-CDR1 | TSGMGVS |
| 4 | F3F8-H-CDR2 | HIYWDNDKRYNPSLKS |
| 5 | F3F8-H-CDR3 | SDSSGSFDY |
| 6 | F3F8-L | DIQMTQTTSSLSASLGDRVTISCRASQDISNYLNWYQQKPDGTVKFLIYYTSTLHSGVPSRFSGSGSGTDY LTISNLEQEDIATYFCQQGNTLPWTFGGGTKLEIKRTVAAPSVFIFPPSDEQLKSGTASVVCLLNNFYPREA VQWKVDNALQSGNSQESVTEQDSKDSTYSLSSTLTLSKADYEKHKVYACEVTHQGLSSPVTKSFNRGEC |
| 7 | F3F8-L-VL | DIQMTQTTSSLSASLGDRVTISCRASQDISNYLNWYQQKPDGTVKFLIYYTSTLHSGVPSRFSGSGSGTDY LTISNLEQEDIATYFCQQGNTLPWTFGGGTKLEIK S |
| 8 | F3F8-L-CDR1 | RASQDISNYLN |
| 9 | F3F8-L-CDR2 | YTSTLHS |
| 10 | F3F8-L-CDR3 | QQGNTLPWT |
| 11 | F6B6-H | QVQLKQSGPGLVQPSQSLSITCTVSGFSLTNYGVHWVRQSPGKGLEWLGVIWSDGSTDYDAAFISRLSIS[?] NSKSQVFFKMNSLQAADTAIYYCARKGDYDYDNYAMDYWGQGTSVTVSSASTKGPSVFPLAPSSKSTS[?] GTAALGCLVKDYFPEPVTVSWNSGALTSGVHTFPAVLQSSGLYSLSSVVTVPSSSLGTQTYICNVNHKPS[?] TKVDKKVEPKSCDKTHTCPPCPAPELLGGPSVFLFPPKPKDTLMISRTPEVTCVVVDVSHEDPEVKFNWY DGVEVHNAKTKPREEQYNSTYRVVSVLTVLHQDWLNGKEYKCKVSNKALPAPIEKTISKAKGQPREPQ[?] TLPPSRDELTKNQVSLTCLVKGFYPSDIAVEWESNGQPENNYKTTPPVLDSDGSFFLYSKLTVDKSRWQ[?] NVFSCSVMHEALHNHYTQKSLSLSPGK |
| 12 | F6B6-H-VH | QVQLKQSGPGLVQPSQSLSITCTVSGFSLTNYGVHWVRQSPGKGLEWLGVIWSDGSTDYDAAFISRLSIS[?] NSKSQVFFKMNSLQAADTAIYYCARKGDYDYDNYAMDYWGQGTSVTVSS |
| 13 | F6B6-H-CDR1 | NYGVH |
| 14 | F6B6-H-CDR2 | VIWSDGSTDYDAAFIS |
| 15 | F6B6-H-CDR3 | KGDYDYDNYAMDY |
| 16 | F6B6-L | DVVITQSPATLSVTPGDSVSLSCRASQSISNNLHWYQQKSHESPRLLIKYASQSISGIPSRFSGSGSGTDFTL NSVETEDFGMYFCQQSNSWPLTFGAGTKLELKRTVAAPSVFIFPPSDEQLKSGTASVVCLLNNFYPREAK QWKVDNALQSGNSQESVTEQDSKDSTYSLSSTLTLSKADYEKHKVYACEVTHQGLSSPVTKSFNRGEC SI V |
| 17 | F6B6-L-VL | DVVITQSPATLSVTPGDSVSLSCRASQSISNNLHWYQQKSHESPRLLIKYASQSISGIPSRFSGSGSGTDFTL NSVETEDFGMYFCQQSNSWPLTFGAGTKLELK SI |
| 18 | F6B6-L-CDR1 | RASQSISNNLH |
| 19 | F6B6-L-CDR2 | YASQSIS |
| 20 | F6B6-L-CDR3 | QQSNSWPLT |
| 21 | H3F7-a-H | EVQLQQSGPELVKPGASVKIPCKASGYTFTDYNMDWVKQSHGKSLEWIGDINPKNGETFYNQKFKGKAT TVDRSSSTAYMELRSLTSEDTAVYFCAKRDLNWYFDYWGQGTTLTVSSASTKGPSVFPLAPSSKSTSGGT ALGCLVKDYFPEPVTVSWNSGALTSGVHTFPAVLQSSGLYSLSSVVTVPSSSLGTQTYICNVNHKPSNTK DKKVEPKSCDKTHTCPPCPAPELLGGPSVFLFPPKPKDTLMISRTPEVTCVVVDVSHEDPEVKFNWYVDG EVHNAKTKPREEQYNSTYRVVSVLTVLHQDWLNGKEYKCKVSNKALPAPIEKTISKAKGQPREPQVYTL PSRDELTKNQVSLTCLVKGFYPSDIAVEWESNGQPENNYKTTPPVLDSDGSFFLYSKLTVDKSRWQQGN SCSVMHEALHNHYTQKSLSLSPGK |
| 22 | H3F7-a-VH | EVQLQQSGPELVKPGASVKIPCKASGYTFTDYNMDWVKQSHGKSLEWIGDINPKNGETFYNQKFKGKA[?] TVDRSSSTAYMELRSLTSEDTAVYFCAKRDLNWYFDYWGQGTTLTVSS |
| 23 | H3F7-a-H-CDR1 | DYNMD |
| 24 | H3F7-a-H-CDR2 | DINPKNGETFYNQKFKG |
| 25 | H3F7-a-H-CDR3 | RDLNWYFDY |
| 26 | D12D8-H | QVQLQQSGAELARPGASVKLSCKASGYTFTNYGISWVKQRTGQGLEWIGEIYPRSGNTYYNEKFKGKAT TADKSSSTAYMELRSLTSEDSAVYFCARGDYSNSDYWGQGTTLTVSSASTKGPSVFPLAPSSKSTSGGTAA LGCLVKDYFPEPVTVSWNSGALTSGVHTFPAVLQSSGLYSLSSVVTVPSSSLGTQTYICNVNHKPSNTKVD KKVEPKSCDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVDKSRWQQGNVFS CSVMHEALHNHYTQKSLSLSPGK |
| 27 | D12D8-H-VH | QVQLQQSGAELARPGASVKLSCKASGYTFTNYGISWVKQRTGQGLEWIGEIYPRSGNTYYNEKFKGKATL TADKSSSTAYMELRSLTSEDSAVYFCARGDYSNSDYWGQGTTLTVSS |
| 28 | D12D8-H-CDR1 | NYGIS |
| 29 | D12D8-H-CDR2 | EIYPRSGNTYYNEKFKG |
| 30 | D12D8-H-CDR3 | GDYSNSDY |
| 31 | D12D8-L | DIQMTQTTSSLSASLGDRVTISCRASQDITDHLNWYQQKPDGTVKLLIYYTSRLHSGVPSRFSGSGSGTDYS LTISNLEQEDIATYFCQQGNTLPRTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 32 | D12D8-VL | DIQMTQTTSSLSASLGDRVTISCRASQDITDHLNWYQQKPDGTVKLLIYYTSRLHSGVPSRFSGSGSGTDYS LTISNLEQEDIATYFCQQGNTLPRTFGGGTKLEIK |
| 33 | D12D8-L-CDR1 | RASQDITDHLN |
| 34 | D12D8-L-CDR2 | YTSRLHS |
| 35 | D12D8-L-CDR3 | QQGNTLPRT |
| 36 | E3A1-H | QVTLKESGPGILQSSQTLSLTCSFSGFSLTTSGMGVSWIRQPSGKGLEWLAHIYWDDDKRYNPSLKSRLTIS KDTSRNQVFLKITSVDTADTATYYCAGCDSSGSFDYWGQGTTLTVSSASTKGPSVFPLAPSSKSTSGGTAA LGCLVKDYFPEPVTVSWNSGALTSGVHTFPAVLQSSGLYSLSSVVTVPSSSLGTQTYICNVNHKPSNTKVD KKVEPKSCDKTHTCPPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTVDKSRWQQGNVFS CSVMHEALHNHYTQKSLSLSPGK |
| 37 | E3A1-VH | QVTLKESGPGILQSSQTLSLTCSFSGFSLTTSGMGVSWIRQPSGKGLEWLAHIYWDDDKRYNPSLKSRLTIS KDTSRNQVFLKITSVDTADTATYYCAGCDSSGSFDYWGQGTTLTVSS |
| 3 | E3A1-H-CDR1 | TSGMGVS |
| 38 | E3A1-H-CDR2 | HIYWDDDKRYNPSLKS |
| 39 | E3A1-H-CDR3 | CDSSGSFDY |
| 40 | E3A1-L | DIQMTQTTSSLSASLGDRVTITCRASQDISNYLNWYQQKPDGTVKLLIYYRSRLHSGVPSRFSGSGSGPEYS LTISNLEQEDIATYFCQQGNTLPWTFGGGTKLEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 41 | E3A1-VL | DIQMTQTTSSLSASLGDRVTITCRASQDISNYLNWYQQKPDGTVKLLIYYRSRLHSGVPSRFSGSGSGPEYS LTISNLEQEDIATYFCQQGNTLPWTFGGGTKLEIK |
| 8 | E3A1-L-CDR1 | RASQDISNYLN |
| 42 | E3A1-L-CDR2 | YRSRLHS |
| 10 | E3A1-L-CDR3 | QQGNTLPWT |
| 43 | A1B2-H | EVQLQQSGAELVRPGASVKLSCTASGFNIKDYYMHWVKQRPEQGLEWIGRIDPEDGDTEYAPKFQGKAT MTADTSSNTAYLQLSSLTSEDTAVYYCTTYYYVSTYEDYWGQGTTLTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 44 | A1B2-H-VH | EVQLQQSGAELVRPGASVKLSCTASGFNIKDYYMHWVKQRPEQGLEWIGRIDPEDGDTEYAPKFQGKAT MTADTSSNTAYLQLSSLTSEDTAVYYCTTYYYVSTYEDYWGQGTTLTVSS |
| 45 | A1B2-H-CDR1 | GFNIKDYY |
| 46 | A1B2-H-CDR2 | IDPEDGDT |
| 47 | A1B2-H-CDR3 | TTYYYVSTYEDY |
| 48 | A1B2-L | QIVLTQSPAIMSASLGEEITLTCSASSSVSHMHWYQQKSGTSPKLLIYSTSNLASGVPSRFSGSGSGTFYSLTI SSVEAEDAADYYCHHWSSWTFGGGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC |
| 49 | A1B2-L-VL | QIVLTQSPAIMSASLGEEITLTCSASSSVSHMHWYQQKSGTSPKLLIYSTSNLASGVPSRFSGSGSGTFYSLTI SSVEAEDAADYYCHHWSSWTFGGGTKLEIK |
| 50 | A1B2-L-CDR1 | SASSSVSHMH |
| 51 | A1B2-L-CDR2 | STSNLAS |
| 52 | A1B2-L-CDR3 | HHWSSWT |
| 53 | A5H12-H | QVTLKESGPGILQSSQTLSLTCSFSGFSLSTSGMGVSWIRQPSGKGLEWLAHIYWDDDKRYNSSLKSRLTIS KDTSRNQVFLKITSVDTADTATYYCARSSDWDDYTMAYWGQGTSVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 54 | A5H12-H-VH | QVTLKESGPGILQSSQTLSLTCSFSGFSLSTSGMGVSWIRQPSGKGLEWLAHIYWDDDKRYNSSLKSRLTIS KDTSRNQVFLKITSVDTADTATYYCARSSDWDDYTMAYWGQGTSVTVSS |
| 3 | A5H12-H-CDR1 | TSGMGVS |
| 55 | A5H12-H-CDR2 | HIYWDDDKRYNSSLKS |
| 56 | A5H12-H-CDR3 | SSDWDDYTMAY |
| 57 | A5H12-L | DIVLTQSPASLAVSLGQRATISCRASESIDDFGISFMHWYQQKPGQPPKLLIYRASNLESGIPARFSGSGSRAD FTLTINPVETDDVATYYCQQSNKDPPTFGAGTNLELKRTVAAPSVFIFPPSDEQLKSGTASVVCLLNNFYPR EAKVQWKVDNALQSGNSQESVTEQDSKDSTYSLSSTLTLSKADYEKHKVYACEVTHQGLSSPVTKSFNRG EC |
| 58 | A5H12-L-VL | DIVLTQSPASLAVSLGQRATISCRASESIDDFGISFMHWYQQKPGQPPKLLIYRASNLESGIPARFSGSGSRAD FTLTINPVETDDVATYYCQQSNKDPPTFGAGTNLELK |
| 59 | A5H12-L-CDR1 | RASESIDDFGISFMH |
| 60 | A5H12-L-CDR2 | RASNLES |
| 61 | A5H12-L-CDR3 | QQSNKDPPT |
| 62 | G11B8-H | EVQLQQSGPELVKPGASVKIPCKASGYTFTDYNMDWVKQSHGKSLEWIGDINPKNGETFYNQKFKGKATL TVDRSSNTAYMELRSLTSEDTAVYFCAKRDLNWYFDYWGQGTTLTVSSASTKGPSVFPLAPSSKSTSGGT AALGCLVKDYFPEPVTVSWNSGALTSGVHTFPAVLQSSGLYSLSSVVTVPSSSLGTQTYICNVNHKPSNTK VDKKVEPKSCDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQPREPQVYTL PPSRDELTKNQVSLTCLVKGFYPSDIAVEWESNGQPENNYKTTPPVLDSDGSFFLYSKLTVDKSRWQQGNV FSCSVMHEALHNHYTQKSLSLSPGK |
| 63 | G11B8-VH | EVQLQQSGPELVKPGASVKIPCKASGYTFTDYNMDWVKQSHGKSLEWIGDINPKNGETFYNQKFKGKATL TVDRSSNTAYMELRSLTSEDTAVYFCAKRDLNWYFDYWGQGTTLTVSS |
| 64 | G11B8-H-CDR1 huG11B8H-1/2/3/4/5/6-CDR1 | GYTFTDYN |
| 65 | G11B8-H-CDR2 huG11B8H-1/2/3/5-CDR2 | INPKNGET |
| 66 | huG11B8H-4/6-CDR2 | INPKNEET |
| 67 | G11B8-H-CDR3 huG11B8H-1/2/3/4/5/6-CDR3 | AKRDLNWYFDY |
| 68 | huG11B8H-1 | EVQLVQSGAEVKKPGATVKISCKASGYTFTDYNMDWVQQAPGKGLEWIGDINPKNGETFYAEKFQGRVT LTVDRSTNTAYMELSSLRSEDTAVYFCAKRDLNWYFDYWGQGTTVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 69 | VH huG11B8H-1- | EVQLVQSGAEVKKPGATVKISCKASGYTFTDYNMDWVQQAPGKGLEWIGDINPKNGETFYAEKFQGRVT LTVDRSTNTAYMELSSLRSEDTAVYFCAKRDLNWYFDYWGQGTTVTVSS |
| 70 | huG11B8H-2 | EVQLVQSGAEVKKPGATVKISCKASGYTFTDYNMDWVQQAPGKGLEWIGDINPKNGETFYAEKFQGRVT LTVDRSTNTAYMELSSLRSEDTAVYYCAKRDLNWYFDYWGQGTTVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 71 | huG11B8H-2-VH | EVQLVQSGAEVKKPGATVKISCKASGYTFTDYNMDWVQQAPGKGLEWIGDINPKNGETFYAEKFQGRVT LTVDRSTNTAYMELSSLRSEDTAVYYCAKRDLNWYFDYWGQGTTVTVSS |
| 72 | huG11B8H-3 | EVQLVQSGAEVKKPGATVKISCKASGYTFTDYNMDWVQQAPGKGLEWMGDINPKNGETFYAEKFQGRV TLTVDRSTNTAYMELSSLRSEDTAVYFCAKRDLNWYFDYWGQGTTVTVSSASTKGPSVFPLAPSSKSTSG GTAALGCLVKDYFPEPVTVSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKSRWQQG NVFSCSVMHEALHNHYTQKSLSLSPGK |
| 73 | huG11B8H-3-VH | EVQLVQSGAEVKKPGATVKISCKASGYTFTDYNMDWVQQAPGKGLEWMGDINPKNGETFYAEKFQGRV TLTVDRSTNTAYMELSSLRSEDTAVYFCAKRDLNWYFDYWGQGTTVTVSS |
| 74 | huG11B8H-4 | EVQLVQSGAEVKKPGATVKISCKASGYTFTDYNMDWVQQAPGKGLEWMGDINPKNEETFYAEKFQGRV TLTVDRSTNTAYMELSSLRSEDTAVYFCAKRDLNWYFDYWGQGTTVTVSSASTKGPSVFPLAPSSKSTSG GTAALGCLVKDYFPEPVTVSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKSRWQQG NVFSCSVMHEALHNHYTQKSLSLSPGK |
| 75 | huG11B8H-4-VH | EVQLVQSGAEVKKPGATVKISCKASGYTFTDYNMDWVQQAPGKGLEWMGDINPKNEETFYAEKFQGRV TLTVDRSTNTAYMELSSLRSEDTAVYFCAKRDLNWYFDYWGQGTTVTVSS |
| 76 | huG11B8H-5 | EVQLVQSGAEVKKPGASVKVSCKASGYTFTDYNMDWVRQAPGQRLEWMGDINPKNGETFYSQKFQGRV TITVDTSASTAYMELSSLRSEDTAVYYCAKRDLNWYFDYWGQGTTVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 77 | huG11B8H-5-VH | EVQLVQSGAEVKKPGASVKVSCKASGYTFTDYNMDWVRQAPGQRLEWMGDINPKNGETFYSQKFQGRV TITVDTSASTAYMELSSLRSEDTAVYYCAKRDLNWYFDYWGQGTTVTVSS |
| 78 | huG11B8H-6 | EVQLVQSGAEVKKPGASVKVSCKASGYTFTDYNMDWVRQAPGQRLEWMGDINPKNEETFYSQKFQGRV TITVDTSASTAYMELSSLRSEDTAVYYCAKRDLNWYFDYWGQGTTVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK |
| 79 | huG11B8H-6-VH | EVQLVQSGAEVKKPGASVKVSCKASGYTFTDYNMDWVRQAPGQRLEWMGDINPKNEETFYSQKFQGRV TITVDTSASTAYMELSSLRSEDTAVYYCAKRDLNWYFDYWGQGTTVTVSS |
| 80 | G11B8-L | DIQMTQSSSYLSVSLGGRVTITCKASDHINNWLAWYQQKPGNAPRLLISGATSLETGVPSRFSGSGSGKDYT LSITSLQTEDVATYYCQQYWSTPWTFGGGTKLEIKRTVAAPSVFIFPPSDEQLKSGTASVVCLLNNFYPREA KVQWKVDNALQSGNSQESVTEQDSKDSTYSLSSTLTLSKADYEKHKVYACEVTHQGLSSPVTKSFNRGEC |
| 81 | G11B8-VL | DIQMTQSSSYLSVSLGGRVTITCKASDHINNWLAWYQQKPGNAPRLLISGATSLETGVPSRFSGSGSGKDYT LSITSLQTEDVATYYCQQYWSTPWTFGGGTKLEIK |
| 82 | G11B8-L-CDR1 huG11B8L-1/2-CDR1 | DHINNW |
| 83 | G11B8-L-CDR2 huG11B8L-1/2-CDR2 | GAT |
| 84 | G11B8-L-CDR3 huG11B8L-1/2-CDR3 | QQYWSTPWT |
| 85 | huG11B8L-1 | DIQMTQSPSSLSASVGDRVTITCQASDHINNWLAWYQQKPGKAPKLLISGATSLETGVPSRFSGSGSGKDYT FTISSLQPEDIATYYCQQYWSTPWTFGGGTKVEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 86 | huG11B8L-1-VL | DIQMTQSPSSLSASVGDRVTITCQASDHINNWLAWYQQKPGKAPKLLISGATSLETGVPSRFSGSGSGKDYT FTISSLQPEDIATYYCQQYWSTPWTFGGGTKVEIK |
| 87 | huG11B8L-2 | DIQMTQSPSSLSASVGDRVTITCQASDHINNWLAWYQQKPGKAPKLLISGATSLETGVPSRFSGSGSGKDFT FTISSLQPEDIATYYCQQYWSTPWTFGGGTKVEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 88 | huG11B8L-2-VL | DIQMTQSPSSLSASVGDRVTITCQASDHINNWLAWYQQKPGKAPKLLISGATSLETGVPSRFSGSGSGKDFT FTISSLQPEDIATYYCQQYWSTPWTFGGGTKVEIK |
| 89 | HuG11B8-VEGFR1-heavy chain | EVQLVQSGAEVKKPGATVKISCKASGYTFTDYNMDWVQQAPGKGLEWMGDINPKNGETFYAEKFQGRV TLTVDRSTNTAYMELSSLRSEDTAVYFCAKRDLNWYFDYWGQGTTVTVSSASTKGPSVFPLAPSSKSTSG GTAALGCLVKDYFPEPVTVSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKSRWQQG NVFSCSVMHEALHNHYTQKSLSLSPGAGGGGSGGGGSGGGGSGRPFVEMYSEIPEIIHMTEGRELVIPCRVT SPNITVTLKKFPLDTLIPDGKRIIWDSRKGFIISNATYKEIGLLTCEATVNGHLYKTNYLTHRQTNTI |
| 85 | HuG11B8-VEGFR1-light chain | DIQMTQSPSSLSASVGDRVTITCQASDHINNWLAWYQQKPGKAPKLLISGATSLETGVPSRFSGSGSGKDYT FTISSLQPEDIATYYCQQYWSTPWTFGGGTKVEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 90 | Linker | GGGGSGGGGSGGGGS |
| 91 | IgG1 constant region | |
| 92 | Kappa constant region | |
| 93 | Fc region | DKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLTVDKSRWQQGNVFSCSVMHEAL HNHYTQKSLSLSPGK |
| 94 | huFGFR2-his | RAPYWTNTEKMEKRLHAVPAANTVKFRCPAGGNPMPTMRWLKNGKEFKQEHRIGGYKVRNQHWSLIME SVVPSDKGNYTCVVENEYGSINHTYHLDVVERSPHRPILQAGLPANASTVVGGDVEFVCKVYSDAQPHIQ WIKHVEKNGSKYGPDGLPYLKVLKHSGINSSNAEVLALFNVTEADAGEYICKVSNYIGQANQSAWLTVLP KQQAPGREKEITASPDYLEHHHHHH |
| 95 | cynoFGFR2-his | RAPYWTNTEKMEKRLHAVPAANTVKFRCPAGGNPTPTMRWLKNGKEFKQEHRIGGYKVRNQHWSLIME SVVPSDKGNYTCVVENEYGSINHTYHLDVVERSPHRPILQAGLPANASTVVGGDVEFVCKVYSDAQPHIQ WIKHVEKNGSKYGPDGLPYLKVLKHSGINSSNAEVLALFNVTEADAGEYICKVSNYIGQANQSAWLTVLP KQQAPGREKEITASPDYLEHHHHHH |
| 98 | Bemarituzuma b-H | QVQLVQSGAEVKKPGSSVKVSCKASGYIFTTYNVHWVRQAPGQGLEWIGSIYPDNGDTSYNQNFKGRATI TADKSTSTAYMELSSLRSEDTAVYYCARGDFAYWGQGTLVTVSSASTKGPSVFPLAPSSKSTSGGTAALGC LVKDYFPEPVTVSWNSGALTSGVHTFPAVLQSSGLYSLSSVVTVPSSSLGTQTYICNVNHKPSNTKVDKKV EPKSCDKTHTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVYTLPPSRDE LTKNQVSLTCLVKGFYPSDIAVEWESNGQPENNYKTTPPVLDSDGSFFLYSKLTVDKSRWQQGNVFSCSV MHEALHNHYTQKSLSLSPGK |
| 99 | Bemarituzuma b-L | DIQMTQSPSSLSASVGDRVTITCKASQGVSNDVAWYQQKPGKAPKLLIYSASYRYTGVPSRFSGSGSGTDF TFTISSLQPEDIATYYCQQHSTTPYTFGQGTKLEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 100 | VEGFR1 receptor extracellular domain 2 | GRPFVEMYSEIPEIIHMTEGRELVIPCRVTSPNITVTLKKFPLDTLIPDGKRIIWDSRKGFIISNATYKEIGLLTC EATVNGHLYKTNYLTHRQTNTI |
| 101 | VEGFR1 receptor | SKLKDPELSLKGTQHIMQAGQTLHLQCRGEAAHKWSLPEMVSKESERLSITKSACGRNGKQFCSTLTLNTA QANHTGFYSCKYLAVPTSKKKETESAIYIFISDTGRPFVEMYSEIPEIIHMTEGRELVIPCRVTSPNITVTLKKF PLDTLIPDGKRIIWDSRKGFIISNATYKEIGLLTCEATVNGHLYKTNYLTHRQTNTIIDVQISTPRPVKLLRGH TLVLNCTATTPLNTRVQMTWSYPDEKNKRASVRRRIDQSNSHANIFYSVLTIDKMQNKDKGLYTCRVRSG PSFKSVNTSVHIYDKAFITVKHRKQQVLETVAGKRSYRLSMKVKAFPSPEVVWLKDGLPATEKSARYLTR GYSLIIKDVTEEDAGNYTILLSIKQSNVFKNLTATLIVNVKPQIYEKAVSSFPDPALYPLGSRQILTCTAYGIP QPTIKWFWHPCNHNHSEARCDFCSNNEESFILDADSNMGNRIESITQRMAIIEGKNKMASTLVVADSRISGI YICIASNKVGTVGRNISFYITDVPNGFHVNLEKMPTEGEDLKLSCTVNKFLYRDVTWILLRTVNNRTMHYSI SKQKMAITKEHSITLNLTIMNVSLQDSGTYACRARNVYTGEEILQKKEITIRDQEAPYLLRNLSDHTVAISSS TTLDCHANGVPEPQITWFKNNHKIQQEPGIILGPGSSTLFIERVTEEDEGVYHCKATNQKGSVESSAYLTVQ GTSDKSNLELITLTCTCVAATLFWLLLTLFIRKMKRSSSEIKTDYLSIIMDPDEVPLDEQCERLPYDASKWEF ARERLKLGKSLGRGAFGKVVQASAFGIKKSPTCRTVAVKMLKEGATASEYKALMTELKILTHIGHHLNVV NLLGACTKQGGPLMVIVEYCKYGNLSNYLKSKRDLFFLNKDAALHMEPKKEKMEPGLEQGKKPRLDSVT SSESFASSGFQEDKSLSDVEEEEDSDGFYKEPITMEDLISYSFQVARGMEFLSSRKCIHRDLAARNILLSENN VVKICDFGLARDIYKNPDYVRKGDTRLPLKWMAPESIFDKIYSTKSDVWSYGVLLWEIFSLGGSPYPGVQM DEDFCSRLREGMRMRAPEYSTPEIYQIMLDCWHRDPKERPRFAELVEKLGDLLQANVQQDGKDYIPINAIL TGNSGFTYSTPAFSEDFFKESISAPKFNSGSSDDVRYVNAFKFMSLERIKTFEELLPNATSMFDDYQGDSSTL LASPMLKRFTWTDSKPKASLKIDLRVTSKSKESGLSDVSRPSFCHSSCGHVSEGKRRFTYDHAELERKIACC SPPPDYNSVVLYSTPPI |
| 96 | CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC |
| 97 | comprising replacement Fc region, of lysine by alanine | DKTHTCPPCPAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPPSRDELTKNQ VSLTCLVKGFYPSDIAVEWESNGQPENNYKTTPPVLDSGSFFLYSKLTVDKSRWQQGNVFSCSVMHEAL HNHYTQKSLSLSPGA |
| 102 | VEGFR1 receptor extracellular domain | SKLKDPELSLKGTQHIMQAGQTLHLQCRGEAAHKWSLPEMVSKESERLSITKSACGRNGKQFCSTLTLNTA QANHTGFYSCKYLAVPTSKKKETESAIYIFISDTGRPFVEMYSEIPEIIHMTEGRELVIPCRVTSPNITVTLKKF PLDTLIPDGKRIIWDSRKGFIISNATYKEIGLLTCEATVNGHLYKTNYLTHRQTNTIIDVQISTPRPVKLLRGH TLVLNCTATTPLNTRVQMTWSYPDEKNKRASVRRRIDQSNSHANIFYSVLTIDKMQNKDKGLYTCRVRSG PSFKSVNTSVHIYDKAFITVKHRKQQVLETVAGKRSYRLSMKVKAFPSPEVVWLKDGLPATEKSARYLTR GYSLIIKDVTEEDAGNYTILLSIKQSNVFKNLTATLIVNVKPQIYEKAVSSFPDPALYPLGSRQILTCTAYGIP QPTIKWFWHPCNHNHSEARCDFCSNNEESFILDADSNMGNRIESITQRMAIIEGKNKMASTLVVADSRISGI YICIASNKVGTVGRNISFYITDVPNGFHVNLEKMPTEGEDLKLSCTVNKFLYRDVTWILLRTVNNRTMHYSI SKQKMAITKEHSITLNLTIMNVSLQDSGTYACRARNVYTGEEILQKKEITIRDQEAPYLLRNLSDHTVAISSS TTLDCHANGVPEPQITWFKNNHKIQQEPGIILGPGSSTLFIERVTEEDEGVYHCKATNQKGSVESSAYLTVQ GTSDKSNLE |
| 103 | CD16AV-FcR1γ | * |
| 104 | HuA1B2H-1-VH | EVQLVQSGAEVKKPGATVKISCKASGFNIKDYYMHWVQQAPGKGLEWMGRIDPEDGDTEYAEKFQGRVT ITADTSTNTAYMELSSLRSEDTAVYYCTTYYYVSTYEDYWGQGTTVTVSS |
| 105 | HuA1B2L-3-VL | EIVLTQSPATLSLSPGERATLSCRASSSVSHMHWYQQKPGQAPKLLIYSTSNLASGVPARFSGSGSGTDYTL TISSLEPEDFAVYYCHHWSSWTFGGGTKVEIK |
| 106 | HuA1B2H-1 | EVQLVQSGAEVKKPGATVKISCKASGFNIKDYYMHWVQQAPGKGLEWMGRIDPEDGDTEYAEKFQGRVT ITADTSTNTAYMELSSLRSEDTAVYYCTTYYYVSTYEDYWGQGTTVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAKGQPREPQVYT LPPSRDELTKNQVSLTCLVKGFYPSDIAVEWESNGQPENNYKTTPPVLDSDGSFFLYSKLTVDKSRWQQGN VFSCSVMHEALHNHYTQKSLSLSPGK* |
| 107 | HuA1B2L-3 | EIVLTQSPATLSLSPGERATLSCRASSSVSHMHWYQQKPGQAPKLLIYSTSNLASGVPARFSGSGSGTDYTL TISSLEPEDFAVYYCHHWSSWTFGGGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVTHQGLSSPVTKSFNRGEC* |
| 108 | Bevacizumab analog-H | EVQLVESGGGLVQPGGSLRLSCAASGYTFTNYGMNWVRQAPGKGLEWVGWINTYTGEPTYAADFKRRFT FSLDTSKSTAYLQMNSLRAEDTAVYYCAKYPHYYGSSHWYFDVWGQGTLVTVSSASTKGPSVFPLAPSSK STSGGTAALGCLVKDYFPEPVTVSWNSGALTSGVHTFPAVLQSSGLYSLSSVVTVPSSSLGTQTYICNVNH KPSNTKVDKKVEPKSCDKTHTCPPCPAPEAAGGPSVFLFPPKPKDTLMISRTPEVTCVVVAVSHEDPEVKFN WYVDGVEVHNAKTKPREEQYNSTYRVVSVLTVLHQDWLNGKEYKCKVSNKALAAPIEKTISKAKGQPRE PQVYTLPPSRDELTKNQVSLTCLVKGFYPSDIAVEWESNGQPENNYKTTPPVLDSDGSFFLYSKLTVDKSR WQQGNVFSCSVMHEALHNHYTQKSLSLSPGK |
| 109 | Bevacizumab analog-L | DIQMTQSPSSLSASVGDRVTITCSASQDISNYLNWYQQKPGKAPKVLIYFTSSLHSGVPSRFSGSGSGTDFTL TISSLQPEDFATYYCQQYSTVPWTFGQGTKVEIKRTVAAPSVFIFPPSDEQLKSGTASVVCLLNNFYPREAK VQWKVDNALQSGNSQESVTEQDSKDSTYSLSSTLTLSKADYEKHKVYACEVTHQGLSSPVTKSFNRGEC |
| 110 | VEGFR1D2-ISO-H | QMQLVQSGAEVKKPGASVKLSCKASGYPFQSYYIHWVRQAPGQGLEWVGVINPSGGSTTYAQKFQGRVT MTRDTSISTAYMELSRLRSDDTVVYYCARGSYSSGWDYYYYYGMDVWGKGTLVTVSSASTKGPSVFPLA PSSKSTSGGTAALGCLVKDYFPEPVTVSWNSGALTSGVHTFPAVLQSSGLYSLSSVVTVPSSSLGTQTYICN VNHKPSNTKVDKKVEPKSCDKTHTCPPCPAPEAAGGPSVFLFPPKPKDTLMISRTPEVTCVVVAVSHEDPE VKFNWYVDGVEVHNAKTKPREEQYNSTYRVVSVLTVLHQDWLNGKEYKCKVSNKALAAPIEKTISKAKG QPREPQVYTLPPSRDELTKNQVSLTCLVKGFYPSDIAVEWESNGQPENNYKTTPPVLDSDGSFFLYSKLTVD KSRWQQGNVFSCSVMHEALHNHYTQKSLSLSPGASPAPELLGGPSGRPFVEMYSEIPEIIHMTEGRELVIPC RVTSPNITVTLKKFPLDTLIPDGKRIIWDSRKGFIISNATYKEIGLLTCEATVNGHLYKTNYLTHRQTNTI |
| 111 | VEGFR1D2-ISO-L | DVVMTQSPLSLPVTPGEPASISCRSSQSLLHSQGYNYLDWYLQKPGQSPQLLIYLGSNRASGVPDRFSGSGS GTDFTLKISRVEAEDVGVYYCMQALQTPWTFGQGTKVEIKRTVAAPSVFIFPPSDEQLKSGTASVVCLLNN FYPREAKVQWKVDNALQSGNSQESVTEQDSKDSTYSLSSTLTLSKADYEKHKVYACEVTHQGLSSPVTKS FNRGEC |
| 112 | Linker | (GS)n, where n is an integer equal to or greater than 1; for example, n is an integer of 2, 3, 4, 5, 6, 7, 8, 9, or 10 |
| 113 | Linker | (GGS)n, where n is an integer equal to or greater than 1; for example, n is an integer of 2, 3, 4, 5, 6, 7, 8, 9, or 10 |
| 114 | Linker | (GSGGS)n, where n is an integer equal to or greater than 1; for example, n is an integer of 2, 3, 4, 5, 6, 7, 8, 9, or 10 |
| 115 | Linker | (GGGGS)n, where n is an integer equal to or greater than 1; for example, n is an integer of 2, 3, 4, 5, 6, 7, 8, 9, or 10 |
| 116 | Linker | (GGGS)n, where n is an integer equal to or greater than 1; for example, n is an integer of 2, 3, 4, 5, 6, 7, 8, 9, or 10 |
| 117 | Linker | (GGGGS)nG, where n is an integer equal to or greater than 1; for example, n is an integer of 2, 3, 4, 5, 6, 7, 8, 9, or 10 |

## Claims

1. An anti-FGFR2 antibody or an antigen-binding fragment thereof, comprising
i. the three complementarity-determining regions HCDR1, HCDR2, and HCDR3 in the heavy chain variable region VH set forth in SEQ ID NO: 63, 69, 71, 73, 75, 77, or 79, and the three complementarity-determining regions LCDR1, LCDR2, and LCDR3 in the light chain variable region VL set forth in SEQ ID NO: 81, 86, or 88;
ii. the three complementarity-determining regions HCDR1, HCDR2, and HCDR3 in the heavy chain variable region VH set forth in SEQ ID NO: 2, and the three complementarity-determining regions LCDR1, LCDR2, and LCDR3 in the light chain variable region VL set forth in SEQ ID NO: 7;
iii. the three complementarity-determining regions HCDR1, HCDR2, and HCDR3 in the heavy chain variable region VH set forth in SEQ ID NO: 22, and the three complementarity-determining regions LCDR1, LCDR2, and LCDR3 in the light chain variable region VL set forth in SEQ ID NO: 81;
iv. the three complementarity-determining regions HCDR1, HCDR2, and HCDR3 in the heavy chain variable region VH set forth in SEQ ID NO: 37, and the three complementarity-determining regions LCDR1, LCDR2, and LCDR3 in the light chain variable region VL set forth in SEQ ID NO: 41;
v. the three complementarity-determining regions HCDR1, HCDR2, and HCDR3 in the heavy chain variable region VH set forth in SEQ ID NO: 27, and the three complementarity-determining regions LCDR1, LCDR2, and LCDR3 in the light chain variable region VL set forth in SEQ ID NO: 32;
vi. the three complementarity-determining regions HCDR1, HCDR2, and HCDR3 in the heavy chain variable region VH set forth in SEQ ID NO: 12, and the three complementarity-determining regions LCDR1, LCDR2, and LCDR3 in the light chain variable region VL set forth in SEQ ID NO: 17;
vii. the three complementarity-determining regions HCDR1, HCDR2, and HCDR3 in the heavy chain variable region VH set forth in SEQ ID NO: 44 or 104, and the three complementarity-determining regions LCDR1, LCDR2, and LCDR3 in the light chain variable region VL set forth in SEQ ID NO: 49 or 105;
viii. the three complementarity-determining regions HCDR1, HCDR2, and HCDR3 in the heavy chain variable region VH set forth in SEQ ID NO: 54, and the three complementarity-determining regions LCDR1, LCDR2, and LCDR3 in the light chain variable region VL set forth in SEQ ID NO: 58; or
ix. the three complementarity-determining regions HCDR1, HCDR2, and HCDR3 in the heavy chain variable region VH set forth in SEQ ID NO: 22, and the three complementarity-determining regions LCDR1, LCDR2, and LCDR3 in the light chain variable region VL set forth in SEQ ID NO: 81.

2. An anti-FGFR2 antibody or an antigen-binding fragment thereof, comprising an HCDR1, an HCDR2, and an HCDR3, and an LCDR1, an LCDR2, and an LCDR3, wherein
i. the HCDR1 comprises or consists of the amino acid sequence set forth in SEQ ID NO: 64; the HCDR2 comprises or consists of the amino acid sequence set forth in SEQ ID NO: 65 or 66; the HCDR3 comprises or consists of the amino acid sequence set forth in SEQ ID NO: 67; the LCDR1 comprises or consists of the amino acid sequence set forth in SEQ ID NO: 82; the LCDR2 comprises or consists of the amino acid sequence set forth in SEQ ID NO: 83; and the LCDR3 comprises or consists of the amino acid sequence set forth in SEQ ID NO: 84;
ii. the HCDR1 comprises or consists of the amino acid sequence set forth in SEQ ID NO: 3; the HCDR2 comprises or consists of the amino acid sequence set forth in SEQ ID NO: 4; the HCDR3 comprises or consists of the amino acid sequence set forth in SEQ ID NO: 5; the LCDR1 comprises or consists of the amino acid sequence set forth in SEQ ID NO: 8; the LCDR2 comprises or consists of the amino acid sequence set forth in SEQ ID NO: 9; and the LCDR3 comprises or consists of the amino acid sequence set forth in SEQ ID NO: 10;
iii. the HCDR1 comprises or consists of the amino acid sequence set forth in SEQ ID NO: 23; the HCDR2 comprises or consists of the amino acid sequence set forth in SEQ ID NO: 24; the HCDR3 comprises or consists of the amino acid sequence set forth in SEQ ID NO: 25; the LCDR1 comprises or consists of the amino acid sequence set forth in SEQ ID NO: 82; the LCDR2 comprises or consists of the amino acid sequence set forth in SEQ ID NO: 83; and the LCDR3 comprises or consists of the amino acid sequence set forth in SEQ ID NO: 84;
iv. the HCDR1 comprises or consists of the amino acid sequence set forth in SEQ ID NO: 3; the HCDR2 comprises or consists of the amino acid sequence set forth in SEQ ID NO: 38; the HCDR3 comprises or consists of the amino acid sequence set forth in SEQ ID NO: 39; the LCDR1 comprises or consists of the amino acid sequence set forth in SEQ ID NO: 8; the LCDR2 comprises or consists of the amino acid sequence set forth in SEQ ID NO: 42; and the LCDR3 comprises or consists of the amino acid sequence set forth in SEQ ID NO: 10;
v. the HCDR1 comprises or consists of the amino acid sequence set forth in SEQ ID NO: 28; the HCDR2 comprises or consists of the amino acid sequence set forth in SEQ ID NO: 29; the HCDR3 comprises or consists of the amino acid sequence set forth in SEQ ID NO: 30; the LCDR1 comprises or consists of the amino acid sequence set forth in SEQ ID NO: 33; the LCDR2 comprises or consists of the amino acid sequence set forth in SEQ ID NO: 34; and the LCDR3 comprises or consists of the amino acid sequence set forth in SEQ ID NO: 35;
vi. the HCDR1 comprises or consists of the amino acid sequence set forth in SEQ ID NO: 13; the HCDR2 comprises or consists of the amino acid sequence set forth in SEQ ID NO: 14; the HCDR3 comprises or consists of the amino acid sequence set forth in SEQ ID NO: 15; the LCDR1 comprises or consists of the amino acid sequence set forth in SEQ ID NO: 18; the LCDR2 comprises or consists of the amino acid sequence set forth in SEQ ID NO: 19; and the LCDR3 comprises or consists of the amino acid sequence set forth in SEQ ID NO: 20;
vii. the HCDR1 comprises or consists of the amino acid sequence set forth in SEQ ID NO: 45; the HCDR2 comprises or consists of the amino acid sequence set forth in SEQ ID NO: 46; the HCDR3 comprises or consists of the amino acid sequence set forth in SEQ ID NO: 47; the LCDR1 comprises or consists of the amino acid sequence set forth in SEQ ID NO: 50; the LCDR2 comprises or consists of the amino acid sequence set forth in SEQ ID NO: 51; and the LCDR3 comprises or consists of the amino acid sequence set forth in SEQ ID NO: 52; or
viii. the HCDR1 comprises or consists of the amino acid sequence set forth in SEQ ID NO: 3; the HCDR2 comprises or consists of the amino acid sequence set forth in SEQ ID NO: 55; the HCDR3 comprises or consists of the amino acid sequence set forth in SEQ ID NO: 56; the LCDR1 comprises or consists of the amino acid sequence set forth in SEQ ID NO: 59; the LCDR2 comprises or consists of the amino acid sequence set forth in SEQ ID NO: 60; and the LCDR3 comprises or consists of the amino acid sequence set forth in SEQ ID NO: 61.

3. The antibody or the antigen-binding fragment thereof according to claim 1 or 2, comprising a heavy chain variable region VH, wherein the heavy chain variable region
(i) comprises or consists of an amino acid sequence having at least 90%, 91%, 92%, 93%, 94%, 95%, 96%, 97%, 98%, or 99% identity to the amino acid sequence of SEQ ID NO: 2, 12, 22, 27, 37, 44, 54, 63, 69, 71, 73, 75, 77, 79, or 104; or
(ii) comprises or consists of the amino acid sequence of SEQ ID NO: 2, 12, 22, 27, 37, 44, 54, 63, 69, 71, 73, 75, 77, 79, or 104.

4. The antibody or the antigen-binding fragment thereof according to any one of claims 1-3, comprising a light chain variable region VL, wherein the light chain variable region
(i) comprises or consists of an amino acid sequence having at least 90%, 91%, 92%, 93%, 94%, 95%, 96%, 97%, 98%, or 99% identity to the amino acid sequence of SEQ ID NO: 7, 17, 32, 41, 49, 58, 81, 86, 88, or 105; or
(ii) comprises or consists of the amino acid sequence of SEQ ID NO: 7, 17, 32, 41, 49, 58, 81, 86, 88, or 105.

5. The antibody or the antigen-binding fragment thereof according to claim 1 or 2, comprising a heavy chain variable region VH and a light chain variable region VL, wherein
i. the VH comprises or consists of the amino acid sequence set forth in SEQ ID NO: 63, 69, 71, 73, 75, 77, or 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81, 86, or 88 or an amino acid sequence having at least 90%, 91%, 92%, 93%, 94%, 95%, 96%, 97%, 98%, or 99% identity thereto;
ii. the VH comprises or consists of the amino acid sequence set forth in SEQ ID NO: 63 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
iii. the VH comprises or consists of the amino acid sequence set forth in SEQ ID NO: 69 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
iv. the VH comprises or consists of the amino acid sequence set forth in SEQ ID NO: 71 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
v. the VH comprises or consists of the amino acid sequence set forth in SEQ ID NO: 73 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
vi. the VH comprises or consists of the amino acid sequence set forth in SEQ ID NO: 75 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
vii. the VH comprises or consists of the amino acid sequence set forth in SEQ ID NO: 77 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
viii. the VH comprises or consists of the amino acid sequence set forth in SEQ ID NO: 79 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 86 or 88 or an amino acid sequence having at least 90%, 91%, 92%, 93%, 94%, 95%, 96%, 97%, 98%, or 99% identity thereto;
ix. the VH comprises or consists of the amino acid sequence set forth in SEQ ID NO: 2 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 7 or an amino acid sequence having at least 90%, 91%, 92%, 93%, 94%, 95%, 96%, 97%, 98%, or 99% identity thereto;
x. the VH comprises or consists of the amino acid sequence set forth in SEQ ID NO: 22 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 81 or an amino acid sequence having at least 90%, 91%, 92%, 93%, 94%, 95%, 96%, 97%, 98%, or 99% identity thereto;
xi. the VH comprises or consists of the amino acid sequence set forth in SEQ ID NO: 37 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 41 or an amino acid sequence having at least 90%, 91%, 92%, 93%, 94%, 95%, 96%, 97%, 98%, or 99% identity thereto;
xii. the VH comprises or consists of the amino acid sequence set forth in SEQ ID NO: 27 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 32 or an amino acid sequence having at least 90%, 91%, 92%, 93%, 94%, 95%, 96%, 97%, 98%, or 99% identity thereto;
xiii. the VH comprises or consists of the amino acid sequence set forth in SEQ ID NO: 12 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 17 or an amino acid sequence having at least 90%, 91%, 92%, 93%, 94%, 95%, 96%, 97%, 98%, or 99% identity thereto;
xiv. the VH comprises or consists of the amino acid sequence set forth in SEQ ID NO: 44 or 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or 105 or an amino acid sequence having at least 90%, 91%, 92%, 93%, 94%, 95%, 96%, 97%, 98%, or 99% identity thereto; the VH comprises or consists of the amino acid sequence set forth in SEQ ID NO: 44 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 49 or an amino acid sequence having at least 90%, 91%, 92%, 93%, 94%, 95%, 96%, 97%, 98%, or 99% identity thereto;
xv. the VH comprises or consists of the amino acid sequence set forth in SEQ ID NO: 104 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 105 or an amino acid sequence having at least 90%, 91%, 92%, 93%, 94%, 95%, 96%, 97%, 98%, or 99% identity thereto; or
xvi. the VH comprises or consists of the amino acid sequence set forth in SEQ ID NO: 54 or an amino acid sequence having at least 90%, 91%, 92%, 93%, 94%, 95%, 96%, 97%, 98%, or 99% identity thereto, and the VL comprises or consists of the amino acid sequence set forth in SEQ ID NO: 58 or an amino acid sequence having at least 90%, 91%, 92%, 93%, 94%, 95%, 96%, 97%, 98%, or 99% identity thereto.

6. The antibody or the antigen-binding fragment thereof according to claim 1 or 2, comprising a heavy chain variable region VH and a light chain variable region VL, wherein
i. the VH comprises or consists of the amino acid sequence set forth in SEQ ID NO: 63, 69, 71, 73, 75, 77, or 79, and the VL comprises or consists of the amino acid sequence set forth in SEQ ID NO: 81, 86, or 88;
ii. the VH comprises or consists of the amino acid sequence set forth in SEQ ID NO: 63, and the VL comprises or consists of the amino acid sequence set forth in SEQ ID NO: 81;
iii. the VH comprises or consists of the amino acid sequence set forth in SEQ ID NO: 69, and the VL comprises or consists of the amino acid sequence set forth in SEQ ID NO: 86 or 88;
iv. the VH comprises or consists of the amino acid sequence set forth in SEQ ID NO: 71, and the VL comprises or consists of the amino acid sequence set forth in SEQ ID NO: 86 or 88;
v. the VH comprises or consists of the amino acid sequence set forth in SEQ ID NO: 73, and the VL comprises or consists of the amino acid sequence set forth in SEQ ID NO: 86 or 88;
vi. the VH comprises or consists of the amino acid sequence set forth in SEQ ID NO: 75, and the VL comprises or consists of the amino acid sequence set forth in SEQ ID NO: 86 or 88;
vii. the VH comprises or consists of the amino acid sequence set forth in SEQ ID NO: 77, and the VL comprises or consists of the amino acid sequence set forth in SEQ ID NO: 86 or 88;
viii. the VH comprises or consists of the amino acid sequence set forth in SEQ ID NO: 79, and the VL comprises or consists of the amino acid sequence set forth in SEQ ID NO: 86 or 88;
ix. the VH comprises or consists of the amino acid sequence set forth in SEQ ID NO: 2, and the VL comprises or consists of the amino acid sequence set forth in SEQ ID NO: 7;
x. the VH comprises or consists of the amino acid sequence set forth in SEQ ID NO: 22, and the VL comprises or consists of the amino acid sequence set forth in SEQ ID NO: 81;
xi. the VH comprises or consists of the amino acid sequence set forth in SEQ ID NO: 37, and the VL comprises or consists of the amino acid sequence set forth in SEQ ID NO: 41;
xii. the VH comprises or consists of the amino acid sequence set forth in SEQ ID NO: 27, and the VL comprises or consists of the amino acid sequence set forth in SEQ ID NO: 32;
xiii. the VH comprises or consists of the amino acid sequence set forth in SEQ ID NO: 12, and the VL comprises or consists of the amino acid sequence set forth in SEQ ID NO: 17;
xiv. the VH comprises or consists of the amino acid sequence set forth in SEQ ID NO: 44 or 104, and the VL comprises or consists of the amino acid sequence set forth in SEQ ID NO: 49 or 105; the VH comprises or consists of the amino acid sequence set forth in SEQ ID NO: 44, and the VL comprises or consists of the amino acid sequence set forth in SEQ ID NO: 49;
xv. the VH comprises or consists of the amino acid sequence set forth in SEQ ID NO: 104, and the VL comprises or consists of the amino acid sequence set forth in SEQ ID NO: 105; or
xvi. the VH comprises or consists of the amino acid sequence set forth in SEQ ID NO: 54, and the VL comprises or consists of the amino acid sequence set forth in SEQ ID NO: 58.

7. The antibody or the antigen-binding fragment thereof according to any one of claims 1-6, further comprising a heavy chain constant region HC, wherein, for example, the heavy chain constant region HC of the antibody is a heavy chain constant region of IgG1, IgG2, IgG3, or IgG4, preferably a heavy chain constant region of IgG1.

8. The antibody or the antigen-binding fragment thereof according to claim 7, wherein the heavy chain constant region
(i) comprises or consists of an amino acid sequence having at least 85%, 90%, 91%, 92%, 93%, 94%, 95%, 96%, 97%, 98%, or 99% identity to the amino acid sequence of SEQ ID NO: 91; or
(ii) comprises or consists of the amino acid sequence of SEQ ID NO: 91.

9. The antibody or the antigen-binding fragment thereof according to any one of claims 1-8, further comprising a light chain constant region, wherein, for example, the light chain constant region is a lambda or kappa light chain constant region.

10. The antibody or the antigen-binding fragment thereof according to claim 9, wherein the light chain constant region
(i) comprises or consists of an amino acid sequence having at least 85%, 90%, 91%, 92%, 93%, 94%, 95%, 96%, 97%, 98%, or 99% identity to an amino acid sequence selected from SEQ ID NO: 92; or
(ii) comprises or consists of an amino acid sequence selected from SEQ ID NO: 92.

11. The antibody or the antigen-binding fragment thereof according to any one of claims 1-10, wherein the antibody comprises a heavy chain; the heavy chain
(i) comprises or consists of an amino acid sequence having at least 90%, 91%, 92%, 93%, 94%, 95%, 96%, 97%, 98%, or 99% identity to the amino acid sequence of SEQ ID NO: 1, 11, 21, 26, 36, 43, 53, 62, 68, 70, 72, 74, 76, 78, or 106;
(ii) comprises or consists of the amino acid sequence of SEQ ID NO: 1, 11, 21, 26, 36, 43, 53, 62, 68, 70, 72, 74, 76, 78, or 106; or
(iii) is the heavy chain in (ii), with the lysine at the C-terminus of the heavy chain being substituted by alanine.

12. The antibody or the antigen-binding fragment thereof according to any one of claims 1-11, wherein the antibody comprises a light chain; the light chain
(i) comprises or consists of an amino acid sequence having at least 90%, 91%, 92%, 93%, 94%, 95%, 96%, 97%, 98%, or 99% identity to the amino acid sequence of SEQ ID NO: 6, 16, 31, 40, 48, 57, 80, 85, 87, or 107; or
(ii) comprises or consists of the amino acid sequence of SEQ ID NO: 6, 16, 31, 40, 48, 57, 80, 85, 87, or 107.

13. The antibody or the antigen-binding fragment thereof according to any one of claims 1-12, comprising a heavy chain and a light chain, wherein
i. the heavy chain comprises or consists of the amino acid sequence set forth in SEQ ID NO: 62, 68, 70, 72, 74, 76, or 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80, 85, or 87 or an amino acid sequence having at least 90%, 91%, 92%, 93%, 94%, 95%, 96%, 97%, 98%, or 99% identity thereto;
ii. the heavy chain comprises or consists of the amino acid sequence set forth in SEQ ID NO: 62 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
iii. the heavy chain comprises or consists of the amino acid sequence set forth in SEQ ID NO: 68 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
iv. the heavy chain comprises or consists of the amino acid sequence set forth in SEQ ID NO: 70 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
v. the heavy chain comprises or consists of the amino acid sequence set forth in SEQ ID NO: 72 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
vi. the heavy chain comprises or consists of the amino acid sequence set forth in SEQ ID NO: 74 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
vii. the heavy chain comprises or consists of the amino acid sequence set forth in SEQ ID NO: 76 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
viii. the heavy chain comprises or consists of the amino acid sequence set forth in SEQ ID NO: 78 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 85 or 87 or an amino acid sequence having at least 90%, 91%, 92%, 93%, 94%, 95%, 96%, 97%, 98%, or 99% identity thereto;
ix. the heavy chain comprises or consists of the amino acid sequence set forth in SEQ ID NO: 1 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 6 or an amino acid sequence having at least 90%, 91%, 92%, 93%, 94%, 95%, 96%, 97%, 98%, or 99% identity thereto;
x. the heavy chain comprises or consists of the amino acid sequence set forth in SEQ ID NO: 21 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 80 or an amino acid sequence having at least 90%, 91%, 92%, 93%, 94%, 95%, 96%, 97%, 98%, or 99% identity thereto;
xi. the heavy chain comprises or consists of the amino acid sequence set forth in SEQ ID NO: 36 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 40 or an amino acid sequence having at least 90%, 91%, 92%, 93%, 94%, 95%, 96%, 97%, 98%, or 99% identity thereto;
xii. the heavy chain comprises or consists of the amino acid sequence set forth in SEQ ID NO: 26 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 31 or an amino acid sequence having at least 90%, 91%, 92%, 93%, 94%, 95%, 96%, 97%, 98%, or 99% identity thereto;
xiii. the heavy chain comprises or consists of the amino acid sequence set forth in SEQ ID NO: 11 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 16 or an amino acid sequence having at least 90%, 91%, 92%, 93%, 94%, 95%, 96%, 97%, 98%, or 99% identity thereto;
xiv. the heavy chain comprises or consists of the amino acid sequence set forth in SEQ ID NO: 43 or 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or 107 or an amino acid sequence having at least 90%, 91%, 92%, 93%, 94%, 95%, 96%, 97%, 98%, or 99% identity thereto;
xv. the heavy chain comprises or consists of the amino acid sequence set forth in SEQ ID NO: 43 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 48 or an amino acid sequence having at least 90%, 91%, 92%, 93%, 94%, 95%, 96%, 97%, 98%, or 99% identity thereto;
xvi. the heavy chain comprises or consists of the amino acid sequence set forth in SEQ ID NO: 106 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 107 or an amino acid sequence having at least 90%, 91%, 92%, 93%, 94%, 95%, 96%, 97%, 98%, or 99% identity thereto;
xvii. the heavy chain comprises or consists of the amino acid sequence set forth in SEQ ID NO: 53 or an amino acid sequence having at least 90%, 91%, 92%, 93%, 94%, 95%, 96%, 97%, 98%, or 99% identity thereto, and the light chain comprises or consists of the amino acid sequence set forth in SEQ ID NO: 57 or an amino acid sequence having at least 90%, 91%, 92%, 93%, 94%, 95%, 96%, 97%, 98%, or 99% identity thereto; or
xviii. on the basis of the heavy chain and light chain in any one of i-xvii, the lysine at the C-terminus of the heavy chain is substituted by alanine.

14. The antibody or the antigen-binding fragment thereof according to any one of claims 1-13, wherein the antibody is a humanized antibody.

15. The antibody or the antigen-binding fragment thereof according to any one of claims 1-14, wherein the antibody is a monoclonal antibody.

16. The antibody or the antigen-binding fragment thereof according to any one of claims 1-15, wherein the antigen-binding fragment is an antibody fragment selected from: a Fab, a Fab', a Fab'-SH, an Fv, a single-chain antibody, a (Fab')₂, a single-domain antibody (e.g., VHH), a domain antibody (dAb), a heavy-chain antibody, and a linear antibody.

17. The antibody or the antigen-binding fragment thereof according to claim 16, wherein the single-chain antibody is an scFv.

18. A multispecific binding molecule, comprising the antibody or the antigen-binding fragment thereof according to any one of claims 1-17.

19. The multispecific binding molecule according to claim 18, wherein the multispecific binding molecule is a bispecific binding molecule.

20. The multispecific binding molecule according to claim 19, wherein the multispecific binding molecule is a bispecific binding molecule, e.g., a fusion protein, specifically binding to FGFR2 and VEGF, e.g., VEGFA, such as VEGF165.

21. The multispecific binding molecule according to claim 20, comprising a first target-binding region specifically binding to FGFR2 and a second target-binding region specifically binding to VEGFA, wherein the first target-binding region comprises the antibody or the antigen-binding fragment thereof according to any one of claims 1-17.

22. The multispecific binding molecule according to claim 21, wherein the second target-binding region comprises a VEGF receptor or a functional fragment thereof, or an antibody or an antigen-binding fragment thereof specifically binding to VEGFR2.

23. The multispecific binding molecule according to claim 22, wherein the VEGF receptor is VEGFR1, and/or the functional fragment comprises or consists of:
an extracellular domain of the VEGF receptor or a fragment thereof; for example, the fragment of the extracellular domain comprises or consists of extracellular domain 2.

24. The multispecific binding molecule according to claim 23, wherein
the extracellular domain of VEGFR1 comprises or consists of the following amino acid sequence:
i, the amino acid sequence set forth in SEQ ID NO: 102;
ii, an amino acid sequence having at least 90%, 91%, 92%, 93%, 94%, 95%, 96%, 97%, 98%, or 99% identity to the amino acid sequence set forth in SEQ ID NO: 102; or
iii, an amino acid sequence having at least 90%, 91%, 92%, 93%, 94%, 95%, 96%, 97%, 98%, or 99% identity to the amino acid sequence set forth in SEQ ID NO: 102 and comprising the amino acid sequence set forth in SEQ ID NO: 100;
or, extracellular domain 2 of VEGFR1 comprises or consists of the following amino acid sequence:
i, the amino acid sequence set forth in SEQ ID NO: 100;
ii, an amino acid sequence having at least 90%, 91%, 92%, 93%, 94%, 95%, 96%, 97%, 98%, or 99% identity to the amino acid sequence set forth in SEQ ID NO: 100; or
iii, an amino acid sequence having at least 90%, 91%, 92%, 93%, 94%, 95%, 96%, 97%, 98%, or 99% identity to the amino acid sequence set forth in SEQ ID NO: 100 and comprising the amino acid sequence set forth in SEQ ID NO: 100.

25. The multispecific binding molecule according to any one of claims 18-24, wherein the first target-binding region is a Fab fragment of the anti-FGFR2 antibody as defined in any one of claims 1-17.

26. The multispecific binding molecule according to claim 25, wherein the Fab comprises a CH1; the CH1 is a CH1 from IgG1, IgG2, IgG3, or IgG4, preferably a CH1 from IgG1.

27. The multispecific binding molecule according to claim 26, wherein the CH1
(i) comprises or consists of an amino acid sequence having at least 85%, 90%, 91%, 92%, 93%, 94%, 95%, 96%, 97%, 98%, or 99% identity to the amino acid sequence of SEQ ID NO: 96; or
(ii) comprises or consists of the amino acid sequence of SEQ ID NO: 96.

28. The multispecific binding molecule according to any one of claims 18-27, comprising an Fc region, e.g., an Fc dimer, wherein the two Fc regions constituting the Fc dimer are identical or different.

29. The multispecific binding molecule according to claim 28, wherein the Fc region is a CH1 from IgG1, IgG2, IgG3, or IgG4, preferably an Fc region from IgG1.

30. The multispecific binding molecule according to claim 29, wherein the Fc region comprises or consists of the amino acid sequence set forth in SEQ ID NO: 93 or an amino acid sequence having at least 90% identity, e.g., 95%, 96%, 97%, 99%, or higher identity, thereto.

31. The multispecific binding molecule according to claim 29, wherein the Fc region comprises a C-terminal lysine-to-alanine substitution.

32. The multispecific binding molecule according to claim 31, wherein the Fc region comprises or consists of the amino acid sequence set forth in SEQ ID NO: 97 or an amino acid sequence having at least 90% identity, e.g., 95%, 96%, 97%, 99%, or higher identity, thereto.

33. The multispecific binding molecule according to any one of claims 28-32, wherein the multispecific binding molecule is a bispecific binding molecule and comprises a first target-binding region, a second target-binding region, and an Fc region;
the first target-binding region is a Fab fragment of an anti-FGFR2 antibody, comprising a Fab heavy chain VH-CH1 and a Fab light chain VL-CL;
the second target-binding region comprises or consists of an extracellular domain of a VEGF receptor or a fragment thereof (e.g., comprising or consisting of ECD2);
the C-terminus of the CH1 of the Fab fragment heavy chain is fused to the N-terminus of the Fc region, and the N-terminus of the extracellular domain of the VEGF receptor is fused to the C-terminus of the Fc region, thereby constituting the heavy chain;
the Fab fragment light chain constitutes the light chain.

34. The multispecific binding molecule according to claim 33, wherein the bispecific binding molecule comprises two heavy chains and two light chains; the two heavy chains may be identical or different, or the two light chains may be identical or different.

35. The multispecific binding molecule according to claim 33 or 34, wherein the N-terminus of the extracellular domain of the VEGF receptor is fused to the C-terminus of the Fc region via a linker; for example, the linker comprises or consists of (GGGGS)n, wherein n = 1, 2, 3, or 4.

36. The multispecific binding molecule according to claim 35, wherein
the heavy chain comprises or consists of the amino acid sequence set forth in SEQ ID NO: 89, or an amino acid sequence having at least 85%, 90%, 91%, 92%, 93%, 94%, 95%, 96%, 97%, 98%, or 99% identity thereto;
and/or
the light chain comprises or consists of the amino acid sequence set forth in SEQ ID NO: 85, or an amino acid sequence having at least 85%, 90%, 91%, 92%, 93%, 94%, 95%, 96%, 97%, 98%, or 99% identity thereto.

37. The antibody or the antigen-binding fragment thereof according to any one of claims 1-17 or the multispecific binding molecule according to any one of claims 18-36, wherein the antibody or the antigen-binding fragment thereof or the multispecific binding molecule is ADCC-enhanced; for example, the antibody or the antigen-binding fragment thereof or the multispecific binding molecule has a modified glycosylation type, e.g., is hypofucosylated or afucosylated, or has an increased bisecting GlcNac structure.

38. A nucleic acid molecule, encoding any one of the chains of the antibody or the antigen-binding fragment thereof according to any one of claims 1-17 or any one of the chains of the multispecific binding molecule according to any one of claims 18-36, or consisting of the nucleic acid sequence.

39. An expression vector, comprising the nucleic acid molecule according to claim 37, wherein preferably, the expression vector is pCDNA, e.g., pCDNA3.1.

40. A host cell, comprising the nucleic acid molecule according to claim 37 or the expression vector according to claim 38, wherein preferably, the host cell is prokaryotic or eukaryotic, e.g., a 293 cell or a CHO cell.

41. The host cell according to claim 40, wherein the host cell is a host cell with an altered glycosylation mechanism, e.g., a cell with complete or partial knockout of the FUT8 gene or complete or partial disruption of the FUT8 gene or protein functionality, such as a CHO cell with knockout of the FUT8 gene.

42. A method for preparing the antibody or the antigen-binding fragment thereof according to any one of claims 1-17 and 37 or the multispecific binding molecule according to any one of claims 18-37, comprising: culturing a host cell comprising the nucleic acid molecule according to claim 38 or the expression vector according to claim 39 or the host cell according to claim 40 or 41 in a condition suitable for expressing the chains of the antibody, and optionally recovering the antibody from the host cell (or the host cell culture).

43. An antibody or an antigen-binding fragment thereof or a multispecific binding molecule prepared by the method according to claim 42.

44. An immunoconjugate, comprising: the antibody or the antigen-binding fragment thereof according to any one of claims 1-17 and 37 or the multispecific binding molecule according to any one of claims 18-37.

45. A pharmaceutical composition or a medicament or a formulation, comprising the antibody or the antigen-binding fragment thereof according to any one of claims 1-17 and 37 or the multispecific binding molecule according to any one of claims 18-37, or the immunoconjugate according to claim 44, and optionally a pharmaceutical supplementary material.

46. A pharmaceutical combination product, comprising: the antibody or the antigen-binding fragment thereof according to any one of claims 1-17 and 37 or the multispecific binding molecule according to any one of claims 18-37, or the immunoconjugate according to claim 44, and one or more additional therapeutic agents (e.g., a chemotherapeutic agent, a cytokine, a cytotoxic agent, an additional antibody, a small molecule drug, or an immunomodulatory agent).

47. A method for preventing or treating a cancer in a subject, comprising: administering to the subject an effective amount of the antibody or the antigen-binding fragment thereof according to any one of claims 1-17 and 37 or the multispecific binding molecule according to any one of claims 18-37, or the immunoconjugate according to claim 44, or the pharmaceutical composition or the formulation according to claim 45, or the pharmaceutical combination product according to claim 46.

48. The method according to claim 47, wherein the cancer is an FGFR2-positive cancer; for example, the cancer is **characterized by** having a protein level and/or nucleic acid level of FGFR2 or having an elevated protein level and/or nucleic acid level of FGFR2 in a tumor cell of the cancer (e.g., compared with the protein level and/or nucleic acid level of FGFR2 in a cell of the same tissue of a healthy individual, or compared with the protein level and/or nucleic acid level of FGFR2 in a healthy cell of the same tissue or a cell of an adjacent healthy tissue of the patient).

49. The method according to claim 47 or 48, wherein the cancer is a solid tumor or a hematological tumor, for example, gastric cancer, lung cancer (such as non-small cell lung cancer), esophageal cancer, bile duct cancer (such as unresectable or metastatic bile duct cancer), breast cancer, pancreatic cancer, or colorectal cancer.

50. The method according to any one of claims 47-49, further comprising administering in combination with an additional therapy such as a therapeutic modality (e.g., surgical therapy or radiotherapy) and/or an additional therapeutic agent (e.g., a chemotherapeutic agent, a cytokine, a cytotoxic agent, an additional antibody, a small molecule drug, or an immunomodulatory agent (e.g., an immune checkpoint molecule inhibitor or agonist)).

51. A method for detecting the presence of FGFR2 in a biological sample, comprising
(i) contacting a biological sample with the antibody or the antigen-binding fragment thereof according to any one of claims 1-17 and 37 or the multispecific binding molecule according to any one of claims 18-37 in a condition allowing the binding to FGFR2, and
(ii) detecting whether a complex is formed by the antibody or the multispecific binding molecule and FGFR2,
wherein formation of the complex indicates the presence of FGFR2.
